(19) Europäisches Patentamt
European Patent Office
Office européen des brevets

(11) **EP 1 844 768 A1**

(12) **EUROPEAN PATENT APPLICATION**
published in accordance with Art. 158(3) EPC


(43) Date of publication:
**17.10.2007 Bulletin 2007/42**

(21) Application number: **06711789.5**

(22) Date of filing: **17.01.2006**

(51) Int Cl.:
*A61K 31/18* (2006.01) *A61K 31/255* (2006.01)
*A61K 31/47* (2006.01) *A61K 31/4709* (2006.01)
*A61K 31/04* (2006.01) *A61P 9/06* (2006.01)
*A61P 9/10* (2006.01) *A61P 9/12* (2006.01)

(86) International application number:
**PCT/JP2006/300509**

(87) International publication number:
**WO 2006/077821 (27.07.2006 Gazette 2006/30)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.01.2005 JP 2005011187**



(71) Applicant: **Dainippon Sumitomo Pharma Co., Ltd.**
**Osaka 541-8524 (JP)**

(72) Inventor: **KATAYAMA, Seiji**
**oki-cho, Suita-shi, Osaka, 5640053 (JP)**

(74) Representative: **Duckworth, Timothy John**
**J.A. Kemp & Co.,**
**14 South Square,**
**Gray's Inn**
**London WC1R 5JJ (GB)**


(54) **AROMATIC SULFONE COMPOUND AS ALDOSTERONE RECEPTOR MODULATOR**


(57) The present invention provides a compound represented by the following formula (I):

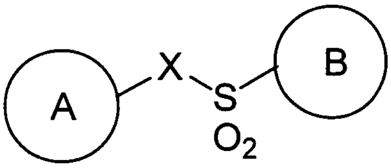

(1)

[wherein, A represents a group of the following formula (A-1):

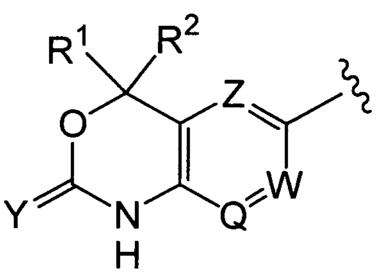

(A-1)

etc., $R^1$ and $R^2$ each independently represent a hydrogen atom etc., Z represents $CR^3$ etc., W represents $CR^4$ etc., Q represents $CR^5$ etc., $R^3$, $R^4$ and $R^5$ each independently represent a hydrogen atom etc., Y represents an oxygen atom or sulfur atom, X represents an oxygen atom etc. and B represents an optionally substituted aryl group or optionally substituted heteroaryl group], the prodrug thereof or the pharmaceutically acceptable salt thereof for preventing or treating various diseases such as hypertesion, cerebral stroke, cardiac failure, etc.


Fig. 1

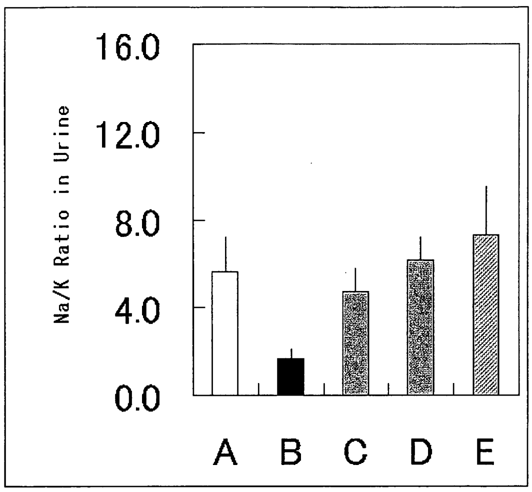


EP 1 844 768 A1

## Description

### Technical Field

The present invention relates to medicaments comprising aromatic sulfone compounds acting on aldosterone receptor MR. More specifically, the present invention relates to non-steroidal compounds and compositions which are modulators (i.e. antagonists, agonists and partial agonists) having high binding affinities to MR.

### Background Art

Nuclear receptors are transcriptional regulators having, as being ligands, biologically active low molecular-weight substances typically including steroid hormones and comprise gene superfamily. As steroid hormone receptors, mineralcorticoid receptor (MR), glucocorticoid receptor (GR), androgen receptor (AR), progesterone receptor (PR) and estrogen receptor (ER) are known, which play critical roles on physiological regulations through regulations of gene expressions.
Aldosterone is a biological ligand of MR, therefore MR is referred to as an aldosterone receptor. While aldosterone regulates electrolyte in kidneys through MR, overactivation of MR relates to various diseases such as hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism and inflammations. Accordingly, compounds binding to MR and regulating its functions exhibit various physiological effects associated with the above-mentioned diseases; for example, compounds (antagonists and partial agonists) competing with aldosterone on the MR and suppressing overactivation of MR are useful for agents preventing or treating the above-mentioned diseases.
The steroid compounds such as spironolactone and eplerenone have been commercialized as medicaments for the aldosterone receptor MR antagonists. Patent Literature 1 reports indole derivatives as non-steroidal MR/GR modulators. 1,4-Dihydro-2H-3,1-benzoxazin-2-one derivatives are disclosed in Patent Literature 2 as HIV reverse transcriptase inhibitors and also disclosed in Patent Literature 3 as PR or AR modulators. They, however, are different in their usages and do not have an aromatic sulfone side chain which is a characteristic feature of the compound of the present invention. Patent Literature 4 discloses 1,2- dihydroquinoline derivatives and 1,2,3,4-tetrahydroquinoline derivatives as steroid receptor regulators, however, which do not have an aromatic sulfone side chain structurally characterizing the compound of the present invention.
Patent Literature 1: International Publication Pamphlet WO2004/067529,
Patent Literature 2: International Publication Pamphlet WO98/14436,
Patent Literature 3: International Publication Pamphlet WO01/16108, and
Patent Literature 4: International Publication Pamphlet WO96/19458.

### Disclosure of the Invention

### Problems to Be Solved by the Invention

The present invention intends to provide a non-steroidal medicament being a modulator (i.e. an antagonist, agonist and partial agonist) which has high binding affinities to aldosterone receptor MR and exhibit preventive or treatment effects for various diseases such as hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism and inflammations.

### Means to Solve the Problems

The inventors of the present invention, after diligent studies, have found that the following compound has a high binding affinity and an activity of antagonist, agonist or partial agonist to aldosterone receptor MR, and achieved the present invention.
That is, the present invention includes the following aspects:

[1] An agent for preventing or treating hypertesion, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations, comprising a compound represented by the formula (1):

[Chemical Formula 1]

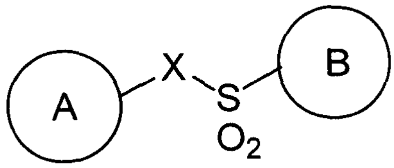

(1)

[wherein A represents any of groups represented by the following formula (A-1), (A-2), (A-3), (A-4) or (A-5);

[Chemical Formula 2]

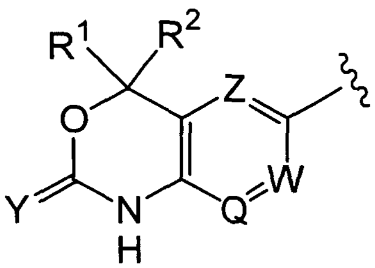

(A-1)

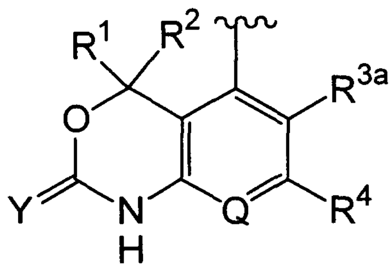

(A-2)

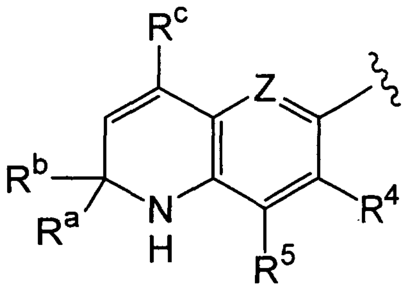

(A-3)

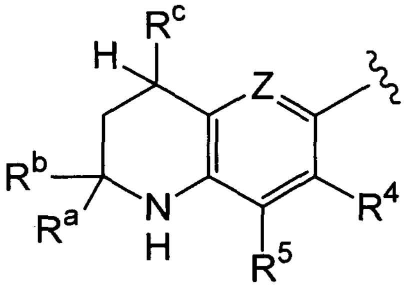

(A-4)

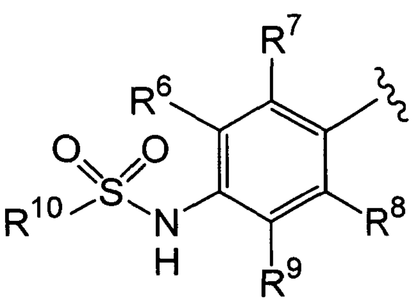

(A-5)

(wherein $R^1$ and $R^2$ each independently represent a hydrogen atom, optionally substituted alkyl group, optionally substituted alkenyl group, optionally substituted alkynyl group, optionally substituted cycloalkyl group, optionally substituted

aryl group or optionally substituted heteroaryl group, or $R^1$ and $R^2$ represent an optionally substituted cycloalkane ring or optionally substituted saturated heterocyclic ring by being combined together with each other's adjacent carbon atom;

Z represents a nitrogen atom or $CR^3$, W represents a nitrogen atom or $CR^4$, and Q represents a nitrogen atom or $CR^5$;
$R^3$, $R^{3a}$, $R^4$, $R^5$, $R^6$, $R^7$, $R^8$ and $R^9$ each independently represent a hydrogen atom, halogen atom, optionally substituted alkyl group, optionally substituted cycloalkyl group, optionally substituted alkenyl group, optionally substituted alkynyl group, optionally substituted aryl group, optionally substituted heteroaryl group, cyano group, nitro group, hydroxyl group, optionally substituted amino group, optionally substituted alkoxy group, optionally substituted alkanoyl group, optionally substituted alkoxycarbonyl group, carboxy group, optionally substituted carbamoyl group, optionally substituted alkylthio group, optionally substituted alkylsulfinyl group, optionally substituted sulfamoyl group or optionally substituted alkylsulfonyl group;
$R^{10}$ represents an optionally substituted alkyl group;
$R^a$, $R^b$ and $R^c$, each independently represent a hydrogen atom or optionally substituted alkyl group; and
Y represents an oxygen atom or sulfur atom);
X represents an oxygen atom, $NR^{11}$ or $CR^{12}R^{13}$
(wherein $R^{11}$ represents a hydrogen atom, optionally substituted alkyl group, optionally substituted alkanoyl group, optionally substituted aroyl group, optionally substituted alkoxycarbonyl group, optionally substituted alkylsulfonyl group, optionally substituted arylsulfonyl group, optionally substituted heteroarylsulfonyl group or the group represented by the formula -C(O)-C(O)-$OR^{11a}$ (wherein $R^{11a}$ represents a hydrogen atom or optionally substituted alkyl group); and
$R^{12}$ and $R^{13}$ each independently represent a hydrogen atom, optionally substituted alkyl group or optionally substituted cycloalkyl group, or $R^{12}$ and $R^{13}$ represent an optionally substituted cycloalkane ring by being combined together with each other's adjacent carbon atom); and
B represents an optionally substituted aryl group or optionally substituted heteroaryl group.],
a prodrug thereof or a pharmaceutically acceptable salt thereof. [0008]

[2] A compound represented by the formula (1a);

[Chemical Formula 3]

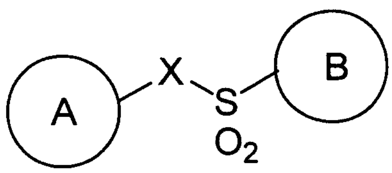

(1a)

[wherein A represents any of groups represented by the following formula (A-1), (A-2), (A-3), (A-4) or (A-5);
[Chemical Formula 4]

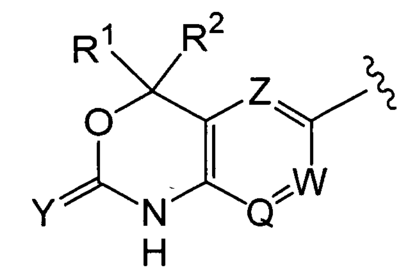

(A-1)

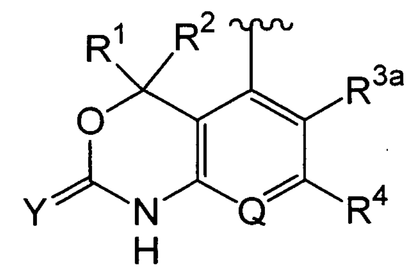

(A-2)

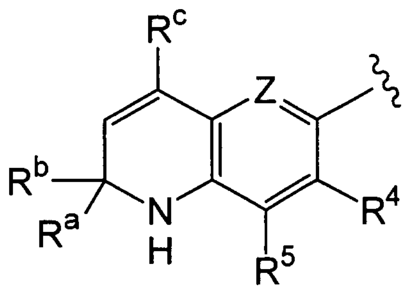

(A-3)

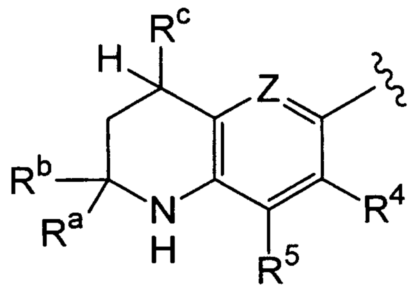

(A-4)

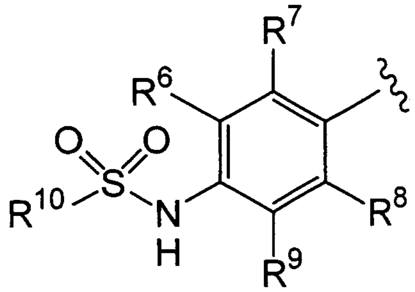

(A-5)

(wherein $R^1$ and $R^2$ each independently represent a hydrogen atom, optionally substituted alkyl group, optionally substituted alkenyl group, optionally substituted alkynyl group, optionally substituted cycloalkyl group, optionally substituted aryl group or optionally substituted heteroaryl group, or $R^1$ and $R^2$ represent an optionally substituted cycloalkane ring or optionally substituted saturated heterocyclic ring by being combined together with each other's adjacent carbon atom;

Z represents a nitrogen atom or $CR^3$, W represents a nitrogen atom or $CR^4$, and Q represents a nitrogen atom or $CR^5$;
$R^3$, $R^{3a}$, $R^4$, $R^5$, $R^6$, $R^7$, $R^8$ and $R^9$ each independently represent a hydrogen atom, halogen atom, optionally substituted alkyl group, optionally substituted cycloalkyl group, optionally substituted alkenyl group, optionally substituted alkynyl group, optionally substituted aryl group, optionally substituted heteroaryl group, cyano group, nitro group, hydroxyl group, optionally substituted amino group, optionally substituted alkoxy group, optionally substituted alkanoyl group, optionally substituted alkoxycarbonyl group, carboxy group, optionally substituted carbamoyl group, optionally substituted alkylthio group, optionally substituted alkylsulfinyl group, optionally substituted sulfamoyl group or optionally substituted alkylsulfonyl group;
$R^{10}$ represents an optionally substituted alkyl group;
$R^a$, $R^b$ and $R^c$ each independently represent a hydrogen atom or optionally substituted alkyl group; and
Y represents an oxygen atom or sulfur atom);
X represents an oxygen atom, $NR^{11}$ or $CR^{12}R^{13}$
(wherein $R^{11}$ represents a hydrogen atom, optionally substituted alkyl group, optionally substituted alkanoyl group, optionally substituted aroyl group, optionally substituted alkoxycarbonyl group, optionally substituted alkylsulfonyl

group, optionally substituted arylsulfonyl group, optionally substituted heteroarylsulfonyl group or the group represented by the formula -C(O)-C(O)-$OR^{11a}$ (wherein $R^{11a}$ represents a hydrogen atom or optionally substituted alkyl group); and

$R^{12}$ and $R^{13}$ each independently represent a hydrogen atom, optionally substituted alkyl group or optionally substituted cycloalkyl group, or $R^{12}$ and $R^{13}$ represent an optionally substituted cycloalkane ring by being combined together with each other's adjacent carbon atom); and

B represents an optionally substituted aryl group or optionally substituted heteroaryl group;

wherein, when the A is the compound represented by the formula (A-1), Z is $CR^3$, W is $CR^4$ and Q is $CR^5$, $R^1$ represents an optionally substituted alkenyl group, optionally substituted alkynyl group, optionally substituted cycloalkyl group, optionally substituted aryl group or optionally substituted heteroaryl group.],
the prodrug thereof or the pharmaceutically acceptable salt thereof.

[3] The compound, the prodrug thereof or the pharmaceutically acceptable salt thereof described in [2], wherein X is an oxygen atom, $NR^{11}$ or $CR^{12}R^{13}$ (wherein $R^{11}$ represents a hydrogen atom, optionally substituted alkyl group or optionally substituted alkanoyl group; and $R^{12}$ and $R^{13}$ each independently represent a hydrogen atom or optionally substituted alkyl group).

[4] The compound, the prodrug thereof or the pharmaceutically acceptable salt thereof described in [3], wherein B is an optionally substituted phenyl, optionally substituted naphthyl, optionally substituted pyridyl, optionally substituted furanyl, optionally substituted pyrrolyl or optionally substituted thienyl.

[5] The compound, the prodrug thereof or the pharmaceutically acceptable salt thereof described in [4], wherein A is the group represented by the formula (A-6);

[Chemical Formula 5]

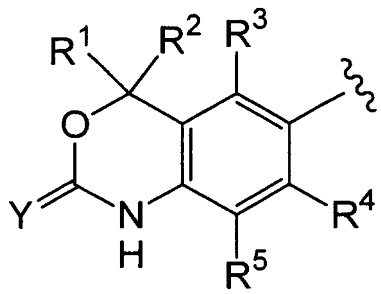

(A-6)

wherein $R^1$ is an optionally substituted alkenyl group, optionally substituted alkynyl group, optionally substituted cycloalkyl group, optionally substituted aryl group or optionally substituted heteroaryl group.

[6] The compound, the prodrug thereof or the pharmaceutically acceptable salt thereof described in [5], wherein $R^3$, $R^4$ and $R^5$ are each independently a hydrogen atom, halogen atom, optionally substituted alkyl group, hydroxyl group or optionally substituted alkoxy group.

[7] The compound, the prodrug thereof or the pharmaceutically acceptable salt thereof described in [4], wherein A is the group represented by the formula (A-7);

[Chemical Formula 6]

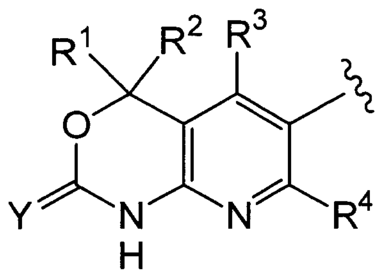

(A-7)

wherein $R^1$ and $R^2$ are each independently a hydrogen atom or optionally substituted alkyl group.

[8] The compound, the prodrug thereof or the pharmaceutically acceptable salt thereof described in [7], wherein $R^3$ and $R^4$ are each independently a hydrogen atom, halogen atom, optionally substituted alkyl group, hydroxyl group or optionally substituted alkoxy group.

[9] The compound, the prodrug thereof or the pharmaceutically acceptable salt thereof described in [4], wherein the A is the group represented by the formula (A-3) or (A-4), and $R^4$ and $R^5$ are each independently a hydrogen atom, halogen atom, optionally substituted alkyl group, hydroxyl group or optionally substituted alkoxy group.
[10] The compound, the prodrug thereof or the pharmaceutically acceptable salt thereof described in [2], wherein the A is the group represented by the formula (A-5).
[11] A pharmaceutical composition comprising the compound, the prodrug thereof or the pharmaceutically acceptable salt thereof described in any of [2] to [10], as its active ingredient.
[12] An agent for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations, the agent comprising the compound, the prodrug thereof or the pharmaceutically acceptable salt thereof described in any of [2] to [10], as its active ingredient.
[13] A method for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations, which comprises administering the compound, the prodrug thereof or the pharmaceutically acceptable salt thereof described in any of [2] to [10], in an effective dose to a patient requiring treatment.
[14] A use of the compound, the prodrug thereof or the pharmaceutically acceptable salt thereof described in any of [2] to [10], for manufacturing an agent for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations.

Hereinafter, the compound represented by the formula (1), the prodrug thereof or the pharmaceutically acceptable salt thereof is generically denoted as "Invention Compound" depending on requirements.

Effects of the Invention

The invention has proved that Invention Compound has a high binding affinity and an activity of antagonist, agonist or partial agonist to aldosterone receptor MR. Thus, the present invention enables provision of an agent for preventing or treating cardiovascular diseases including hypertension, cerebral stroke, arrhythmia, cardiac failure, cardiac

hypertrophy, arteriosclerosis, blood vessel restenosis, kidney fibrosis, myocardial infarction, diabetes complications, renal diseases, edema, primary aldosteronism and inflammations.

Brief Description of the Drawing

[Fig. 1]
In Fig. 1, the symbol A represents the aldosterone-nonadministered group. The symbol B represents the aldosterone+solvent (0.5% methylcellulose)-administered group. The symbol C represents the aldosterone+Compound 24 (10mg/kg body weight) orally administered group. The symbol D represents the aldosterone+Compound 24 (30mg/kg body weight)-orally administered group. The symbol E represents the aldosterone+eplerenone (3mg/kg body weight)-orally administered group.

Best Mode for Carrying Out the Invention

The present invention will be explained more specifically as follows.
The explanations regarding the respective groups in the present invention, unless otherwise specifically defined, are applied to the cases that the groups are included as a part of other groups.
The number of substituents in the present description, without particular limitation as long as the substituents are substitutable, is one or plural.
The "halogen atom" includes a fluorine atom, chlorine atom, bromine atom and iodine atom.
The "alkyl group" includes linear or branched alkyl groups having 1 to 10 carbon atoms, for example, such as methyl, ethyl, propyl, isopropyl, butyl, isobutyl, sec-butyl, tert-butyl, pentyl, 1-methylbutyl, 2-methylbutyl, 3-methylbutyl, 1-ethylpropyl, hexyl, heptyl, octyl, nonyl and decyl. The preferred alkyl group includes linear or branched alkyl groups having 1 to 6 carbon atoms.
The "alkenyl group" includes linear or branched alkenyl groups having 2 to 6 carbon atoms which have at least one double bond, for example, such as vinyl, 1-propenyl, 2-propenyl, 1-methyl-1-propenyl, 1-butenyl, 2-butenyl, 3-butenyl and 1-methyl-1-butenyl. The preferred alkenyl group includes linear or branched alkenyl groups having 3 to 6 carbon atoms.
The "alkynyl group" includes linear or branched alkynyl groups having 2 to 6 carbon atoms which have at least one triple bond, for example, such as ethynyl, 1-propynyl, 2-propynyl, 1-methyl-2-propynyl, 1-butynyl, 2-butynyl, 3-butynyl and 1-methyl-2-butynyl. The preferred alkynyl group includes linear or branched alkynyl groups having 3 to 6 carbon atoms.
The "cycloalkyl group" includes saturated or unsaturated cycloalkyl groups having 3 to 8 carbon atoms, for example, such as cyclopropyl, cyclobutyl, cyclopentyl, cyclohexyl, cycloheptyl, cyclooctyl, cyclobutenyl, cyclopentenyl, cyclohexenyl, cycloheptenyl and cyclooctenyl. The preferred cycloalkyl group includes saturated or unsaturated cycloalkyl groups having 3 to 6 carbon atoms.
The "alkoxy group" includes linear or branched alkoxy groups having 1 to 10 carbon atoms, for example, such as methoxy, ethoxy, propoxy, butoxy, isopropoxy, isobutoxy, sec-butoxy, tert-butoxy, pentoxy, hexoxy, heptoxy, octoxy, nonyloxy and decyloxy. The preferred alkoxy group includes linear or branched alkoxy groups having 1 to 6 carbon atoms.
The "alkanoyl group" includes linear or branched alkanoyl groups having 1 to 10 carbon atoms, for example, such as formyl, acetyl, propionyl, butyryl, isobutyryl, valeryl, isovaleryl, pivaloyl, hexanoyl, heptanoyl, octanoyl, nonanoyl and decanoyl. The preferred alkanoyl group includes linear or branched alkanoyl groups having 1 to 6 carbon atoms.
The "alkoxycarbonyl group" includes linear or branched alkoxycarbonyl groups having 2 to 11 carbon atoms, for example, such as methoxycarbonyl, ethoxycarbonyl, propoxycarbonyl, butoxycarbonyl, isopropoxycarbonyl, isobutoxycarbonyl, sec-butoxycarbonyl, tert-butoxycarbonyl, pentoxycarbonyl, hexoxycarbonyl, heptoxycarbonyl, octoxycarbonyl, nonyloxycarbonyl and decyloxycarbonyl. The preferred alkoxycarbonyl group includes alkoxycarbonyl groups having a linear or branched alkoxy group with 1 to 6 carbon atoms.
The "alkylthio group" includes alkylthio groups having 1 to 10 carbon atoms, for example, such as methylthio, ethylthio, propylthio, butylthio, isopropylthio, isobutylthio, sec-butylthio, tert-butylthio, pentylthio, hexylthio, heptylthio, octylthio, nonylthio and decylthio. The preferred alkylthio group includes alkylthio groups having a linear or branched alkyl group with 1 to 6 carbon atoms.
The "alkylsulfinyl group" includes alkylsulfinyl groups having 1 to 10 carbon atoms, for example, such as methylsulfinyl, ethylsulfinyl, propylsulfinyl, butylsulfinyl, isopropylsulfinyl, isobutylsulfinyl, sec-butylsulfinyl, tert-butylsulfinyl, pentylsulfinyl, hexylsulfinyl, heptylsulfinyl, octylsulfinyl, nonylsulfinyl and decylsulfinyl. The preferred alkylsulfinyl group includes alkylsulfinyl groups having a linear or branched alkyl group with 1 to 6 carbon atoms.
The "alkylsulfonyl group" includes alkylsulfonyl groups having 1 to 10 carbon atoms, for example, such as methylsulfonyl, ethylsulfonyl, propylsulfonyl, butylsulfonyl, isopropylsulfonyl, isobutylsulfonyl, sec-butylsulfonyl, tert-butylsulfonyl, pentylsulfonyl, hexylsulfonyl, heptylsulfonyl, octylsulfonyl, nonylsulfonyl and decylsulfonyl. The preferred alkylsulfonyl group includes alkylsulfonyl groups having a linear or branched alkyl group with 1 to 6 carbon atoms.

The substituent in the "substituted alkyl group", " substituted alkenyl group", "substituted alkynyl group", "substituted alkoxy group", "substituted cycloalkyl group", " substituted alkanoyl group", "substituted alkoxycarbonyl group", "substituted alkylthio group", "substituted alkylsulfinyl group" and "substituted alkylsulfonyl group" includes, for example, halogen atom, hydroxyl group, nitro, cyano, alkoxy group, cycloalkyl group, amino group, alkylamino group, dialkylamino group, alkanoylamino group, alkoxycarbonylamino group, alkylsulfonyl group and arylsulfonyl group.

The substituent in the substituted alkyl group and substituted alkoxy group, besides those mentioned above, includes an optionally substituted aryl group and optionally substituted heteroaryl group.

The substituent in the substituted cycloalkyl group, besides those mentioned above, includes an alkyl group.

The preferred substituent in the "substituted alkyl group", "substituted alkenyl group", "substituted alkynyl group", "substituted alkoxy group", "substituted cycloalkyl group", "substituted alkanoyl group", "substituted alkoxycarbonyl group", "substituted alkylthio group", "substituted alkylsulfinyl group" and "substituted alkylsulfonyl group" includes, for example, a halogen atom, hydroxyl group, amino group, alkylamino group and dialkylamino group.

The "aryl group" includes aryl groups having 10 or less carbon atoms, for example, such as phenyl and naphthyl. The "heteroaryl group" includes, for example, a monocyclic 5- or 6-membered aromatic heterocyclic group and bicyclic 9- or 10-membered aromatic heterocyclic group which include 1 to 4 hetero atoms selected from the group consisting of a nitrogen atom, sulfur atom and oxygen atom, specifically including pyridyl (the nitrogen atom may be oxidized.), thienyl, furyl, pyrrolyl, pyrazolyl, imidazolyl, pyrazyl, pyrimidyl, pyridazyl, oxazolyl, thiazolyl, oxadiazolyl, triazolyl, tetrazolyl, quinolyl, benzothienyl, benzofuryl, indolyl, quinazolinyl, benzothiazolyl, benzoxazolyl, benzimidazolyl, naphthyridinyl and the like. The preferred heteroaryl group includes a monocyclic 5- or 6-membered aromatic heterocyclic group which includes 1 to 3 hetero atoms selected from the group consisting of a nitrogen atom, sulfur atom and oxygen atom. More preferably, a thienyl and pyridyl are included.

The "aroyl group" includes, for example, an aroyl group with 11 or less carbon atoms such as benzoyl and naphthoyl.

The aryl portion in the "arylsulfonyl group" represents the same meaning as mentioned above.

The substituent in the "substituted aryl group" and "substituted heteroaryl group" includes, for example, a halogen atom, hydroxyl group, nitro, cyano, an alkyl group (this alkyl group may be substituted with, for example, halogen atom, hydroxyl group or amino group), an alkoxy group (this alkoxy group may be substituted with, for example, a halogen atom(s)), an alkoxycarbonyl group, carboxy group, amino group (this amino group may be substituted with, for example, one or two alkyl groups, alkanoyl groups or alkoxycarbonyl groups), carbamoyl group, an aryl group, an aryloxy group, an alkylsulfonyl group and an arylsulfonyl group.

Moreover, the substituent in the substituted aryl group includes an alkylenedioxy group such as methylenedioxy and ethylenedioxy.

The substituent in the substituted aryl group and substituted heteroaryl group which are the substituents of the substituted alkyl group and substituted alkoxy group, and the substituent in the "substituted arylsulfonyl group" and "substituted aroyl group" include the same substituents in the above-mentioned "substituted aryl group" and "substituted heteroaryl group".

The preferred substituent in the substituted aryl group and substituted heteroaryl group which are the substituents of the "substituted alkyl group" and "substituted alkoxy group" of $R^1$ and $R^2$, and the preferred substituent in the "substituted aryl group", "substituted heteroaryl group", "substituted arylsulfonyl group" and "substituted aroyl group" include, for example, a halogen atom, hydroxyl group, nitro, cyano, an alkyl group (this alkyl group may be substituted with, for example, a halogen atom(s), hydroxyl group(s) or amino group(s)), an alkoxy group (this alkoxy group may be substituted with, for example, a halogen atom(s)), an alkoxycarbonyl group, carboxy group, amino group, an alkylamino group, a dialkylamino group, carbamoyl group and an alkylenedioxy group.

The "cycloalkane ring" includes saturated or unsaturated cycloalkanes having 3 to 8 carbon atoms of which 2 hydrogen atoms bonding to the same carbon atom are changed to bonding links, for example, such as cyclopropane, cyclobutane, cyclopentane, cyclohexane, cycloheptane, cyclooctane, cyclobutene, cyclopentene, cyclohexene, cycloheptene and cyclooctene.

The "saturated heterocyclic ring" includes, for example, a monocyclic 5-to 8-membered saturated heterocyclic ring which includes 1 to 4 hetero atoms selected from the group consisting of nitrogen atom, sulfur atom and oxygen atom and of which 2 hydrogen atoms bonding to the same carbon atom are changed to bonding links, specifically including pyrrolidine, piperidine, piperazine, morpholine, thiomorpholine and perhydroazepine.

The substituent in the "substituted amino group" includes, for example, an alkyl group, an alkyl group optionally substituted with an aryl group(s), an aryl group, an alkanoyl group and an arylcarbonyl group.

The substituent in the "substituted carbamoyl group" and "substituted sulfamoyl group" includes, for example, an alkyl group, an alkyl group optionally substituted with an aryl group(s) and an aryl group.

Hereinafter, methods for producing the compound represented by the formula (1) in the present invention will be explained with referring Examples, but the present invention should not be construed to be limited thereto.

The compound represented by the formula (1) can be produced from known compounds by combining known synthetic methods. For example, the following methods allow the synthesis.

Among the compounds represented by the formula (1), the compound represented by the formula (110) or its salt, for example, can be produced through the following method.

Production Method 1 (Sulfonylation)

[Chemical Formula 7]

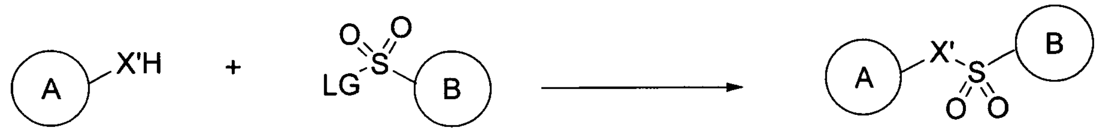

(101) (102) (110)

(X' = O, $NR^{11}$) (X' = O, $NR^{11}$)

(wherein X' represents an oxygen atom or $NR^{11}$, A, B and $R^{11}$ represent the same meanings mentioned above, and LG represents a leaving group (for example, halogen atoms such as a chlorine atom, bromine atom and iodine atom)).

Subjecting the compound (101) or its salt to a reaction with the compound (102) or its salt allows the production of the compound (110). The reaction can be conducted, in the presence of a base if required, besides in the presence of a catalyst as the case may be, in an appropriate inert solvent at a temperature ranging from about -20°C to the boiling point of the solvent for 10 minutes to 48 hours. When the disulfonamidation is conducted under the case of $X=NR^{11}$ and $R^{11}=H$, the resultant can be converted to a monosulfonamide product through hydrolysis with an appropriate base (for example, tetrabutylammonium fluoride etc.).

The base includes, for example, organic bases such as triethylamine, pyridine and 2,4,6-collidine, and inorganic bases such as potassium carbonate, sodium hydroxide and sodium hydride.

The catalyst includes, for example, 4-dimethylaminopyridine.

The inert solvent includes, for example, acetonitrile; halogenated hydrocarbon solvents such as chloroform and dichloromethane; aromatic hydrocarbon solvents such as benzene and toluene; ether solvents such as diethyl ether, tetrahydrofuran and 1,4-dioxane ; polar aprotic solvents such as N,N-dimethylformamide, N-methylpyrrolidone and dimethyl sulfoxide ; and a mixture thereof.

Among the compounds represented by the formula (1), the compound represented by the formula (112) or its salt, for example, can be produced through the following method.

Production Method 2 (Alkylation)

[Chemical Formula 8]

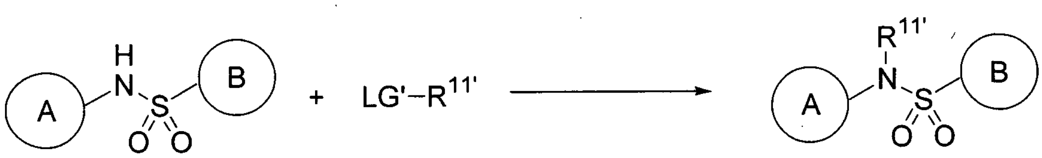

(111) (103) (112)

(wherein A and B represent the same meanings mentioned above, $R^{11'}$ represents the groups among the abovementioned $R^{11}$ except the hydrogen atom, and LG' represents a leaving group (for example, halogen atoms such as a chlorine atom, bromine atom and iodine atom, or sulfonyloxy groups such as tosyloxy, mesyloxy and trifluoromethanesulfonyloxy)).

For example, by subjecting the compound (111) having X=NH which can be synthesized in the above-mentioned step (Production Method 1) or its salt to a reaction with the compound (103) or its salt, the compound (112) (X=$NR^{11}$) can be produced. The reaction can be conducted, in the presence of a base if required, besides in the presence of a phase-transfer catalyst as the case may be, in an appropriate inert solvent at a temperature ranging from about -20°C to the boiling point of the solvent for 10 minutes to 48 hours.

The base includes, for example, organic bases such as triethylamine and pyridine, inorganic bases such as potassium carbonate, sodium hydroxide and sodium hydride, and metal alkoxides such as sodium methoxide and potassium tert-butoxide.

The phase-transfer catalyst includes, for example, tetrabutylammonium iodide and tetrabutylammonium hydrogensulfate.

The inert solvent includes, for example, acetonitrile; halogenated hydrocarbon solvents such as chloroform and dichloromethane; aromatic hydrocarbon solvents such as benzene and toluene; ether solvents such as diethyl ether, tetrahydrofuran and 1,4-dioxane; alcoholic solvents such as methanol, ethanol and 2-propanol; polar aprotic solvents such as N,N-dimethylformamide, N-methylpyrrolidone and dimethyl sulfoxide; and a mixture thereof.

Among the compounds represented by the formula (1), the compound represented by the formula (210) or formula (220) or the salts thereof, for example, can be produced through the following method.

Production Method 3

[Chemical Formula 9]

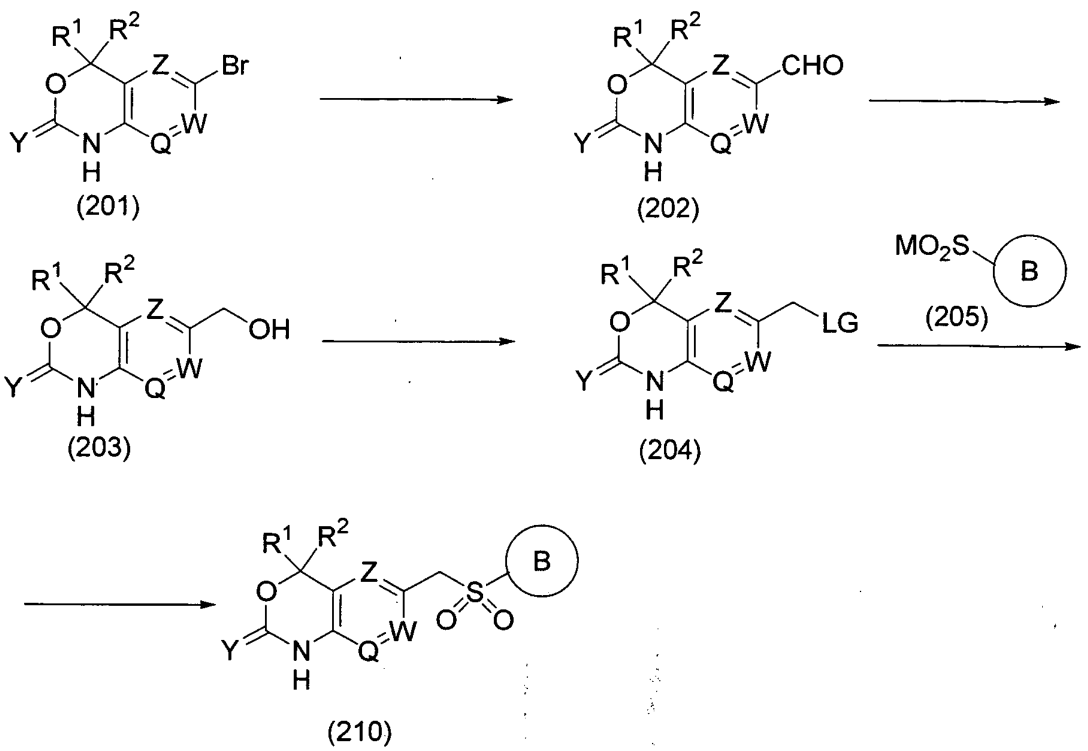

(wherein B, $R^1$, $R^2$, Q, W, Y, Z and LG represent the same meanings mentioned above, and M represents an alkali metal (for example, sodium and potassium)).

Step 1 (Formylation)

Subjecting the compound (201) or its salt to a formylation allows the production of the intermediate (202). The reaction can be conducted by subjecting to a halogen-metal exchange reaction with an organometallic reagent, followed by treatment with a formyl-donating agent, or by subjecting to a reaction, in the presence of a chelating agent as the case may be, in an appropriate inert solvent at a temperature ranging from about -100°C to the boiling point of the solvent for 10 minutes to 48 hours.
The organometallic reagent includes, for example, organolithium reagents such as n-butyllithium, sec-butyllithium and tert-butyllithium.
The formyl-donating agent includes, for example, N,N-dimethylformamide, N-formylpiperidine, N-formylmorpholine, N-methoxy-N-methylformamide, N-methyl-N-phenylformamide, formamide and ethyl formate.
The chelating agent includes, for example, N,N,N',N'-tetramethylethylenediamine.
The inert solvent includes, for example, aromatic hydrocarbon solvents such as benzene and toluene; ether solvents such as diethyl ether and tetrahydrofuran; and a mixture thereof.

Step 2 (Reduction)

Reducing the compound (202) or its salt to an alcohol allows the production of the intermediate (203). The reaction can be conducted, in the presence of a reducing agent, in an appropriate inert solvent at a temperature ranging from about -20°C to the boiling point of the solvent for 10 minutes to 48 hours.
The reducing agent includes, for example, sodium borohydride and lithium borohydride.
The inert solvent includes, for example, alcoholic solvents such as methanol, ethanol and 2-propanol; ether solvents such as diethyl ether, tetrahydrofuran and 1,4-dioxane; and a mixture thereof.

Step 3 (Introduction of Leaving Group)

By converting the alcohol group of the compound (203) or its salt to a leaving group, the intermediate (204) can be produced. The reaction can be conducted, in the presence of a halogenating agent, in an appropriate inert solvent at a temperature ranging from about -20°C to the boiling point of the solvent for 10 minutes to 48 hours.
The halogenating agent includes, for example, thionyl chloride and a combination of N-bromosuccinimide and triphenylphosphine.
The inert solvent includes, for example, acetonitrile; halogenated hydrocarbon solvents such as chloroform and dichloromethane; aromatic hydrocarbon solvents such as benzene and toluene; ether solvents such as diethyl ether, tetrahydrofuran and 1,4-dioxane; polar aprotic solvents such as N,N- dimethylformamide, N-methylpyrrolidone and dimethyl sulfoxide; and a mixture thereof.

Step 4 (Sulfonylation)

Subjecting the compound (204) or its salt to a reaction with the compound (205) or its salt allows the production of the compound (210) or its salt. The reaction can be conducted, in the presence of a base if required, besides in the presence of a phase-transfer catalyst as the case may be, in an appropriate inert solvent at a temperature ranging from about -20°C to the boiling point of the solvent for 10 minutes to 48 hours.
The base, phase-transfer catalyst and inert solvent include those mentioned above (Production Method 2).

Steps 5 to 7 (Introduction of Protective Group - Introduction of Substituent - Removal of Protective Group)

[Chemical Formula 10]

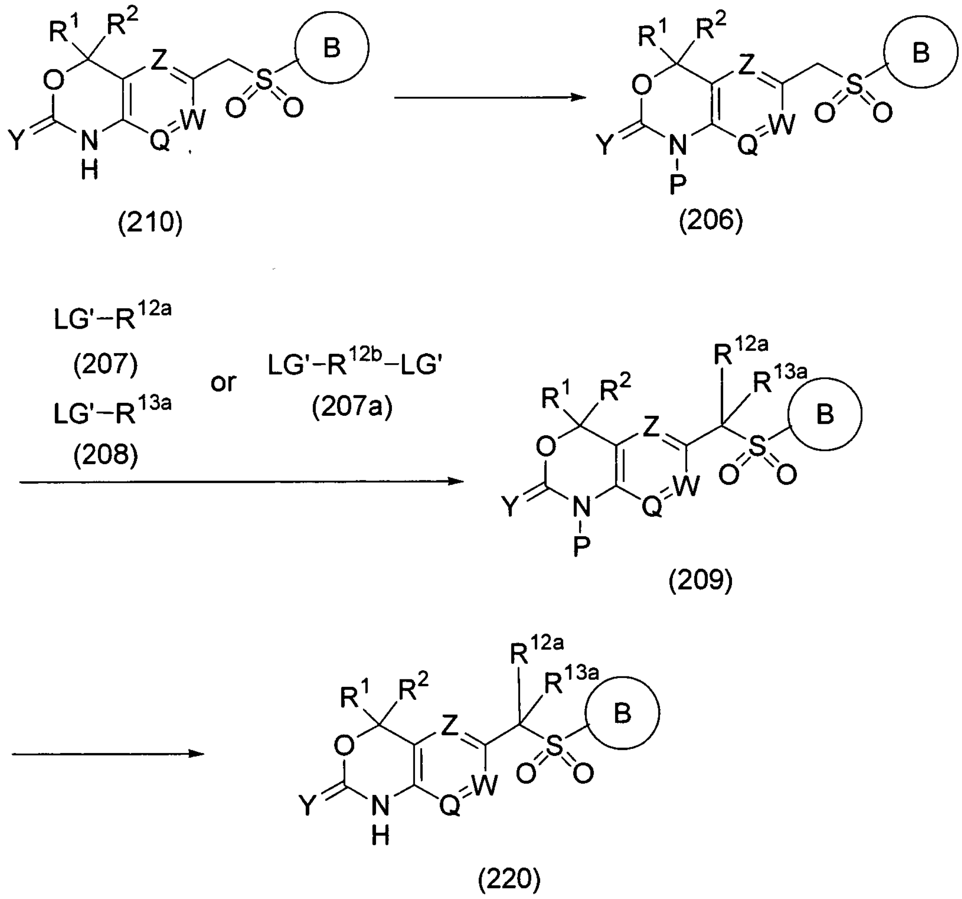


(210) (206) (209) (220)

(wherein B, $R^1$, $R^2$, Q, W, Y, Z, LG' and M represent the same meanings mentioned above; $R^{12a}$ and $R^{13a}$ respectively represent the same meanings as in the above-mentioned $R^{12}$ and $R^{13}$, but exclude the case that both of them are a hydrogen atom; and P represents a protective group (for example, alkyloxycarbonyl groups such as tert-butoxycarbonyl, and substituted alkyl groups such as trimethylsilylethoxymethyl, benzyl and methoxyphenylmethyl)).

The compound (210) or its salt are converted into the intermediate (206) through introduction of a protective group on the NH at 1-position thereof, followed by sequentially or singly reacting with the compound (207) or its salt and the compound (208) or its salt in this order to synthesize the intermediate (209) and then by removing the protective group from the intermediate (209), thus the compound (220) can be produced.

When either $R^{12a}$ or $R^{13a}$ is a hydrogen atom, any one of the compound (207) or compound (208) is used.

As alternative, by using the compound (207a) or its salt in place of the compounds (207) and (208), a compound which is an optionally substituted cycloalkane ring formed by combining $R^{12a}$ and $R^{13a}$ together with each other's adjacent carbon atom may be synthesized.

The reaction can be conducted, in the presence of a base, besides in the presence of a chelating agent as the case may be, in an appropriate inert solvent at a temperature ranging from about -100°C to the boiling point of the solvent for 10 minutes to 48 hours.

The base includes, for example, organolithium reagents such as lithium diisopropylamide, and inorganic bases such as potassium carbonate and cesium carbonate.

The chelating agent includes, for example, those mentioned above (Production Method 3 - Step 1).

The inert solvent includes, for example, acetonitrile; aromatic hydrocarbon solvents such as benzene and toluene; ether solvents such as diethyl ether, tetrahydrofuran and 1,4-dioxane; polar aprotic solvents such as N,N-dimethylformamide, N- methylpyrrolidone and dimethyl sulfoxide; and a mixture thereof.

As the protective group, conventional protective groups disclosed in literatures (for example, Protective Groups in Organic Synthesis, T. W. Greene, John Wiley & Sons Inc., (1981)) can be used; more specifically, the protective group for the NH at 1-position includes tert-butoxycarbonyl, benzyloxycarbonyl, trimethylsilylethoxymethyl, benzyl, 4-methoxyphenylmethyl, and the like.

The introduction and removal of the protective group can be conducted with methods routinely used in organic chemical synthesis (for example, refer to the above-cited Protective Groups in Organic Synthesis) or other methods corresponding thereto.
The Y is preferably an oxygen atom while conducting the reactions of each step, and can be converted to a sulfur atom at the final stage or an appropriate stage. The reaction can be conducted, in the presence of a thionating agent, in an appropriate inert solvent at a temperature ranging from 0°C to the boiling point of the solvent for 10 minutes to 120 hours.
The thionating agent includes Lawesson's reagent, phosphorus pentasulfide and the like.
The inert solvent includes, for example, acetonitrile; halogenated hydrocarbon solvents such as chloroform and dichloromethane; aromatic hydrocarbon solvents such as monochlorobenzene, xylene and toluene; ether solvents such as tetrahydrofuran, 1,2-dimethoxyethane and 1,4-dioxane; and a mixture thereof.

Among the compounds represented by the formula (1), the compound represented by the formula (310) or its salt, for example, can be produced through the following method.

Production Method 4

[Chemical Formula 11]

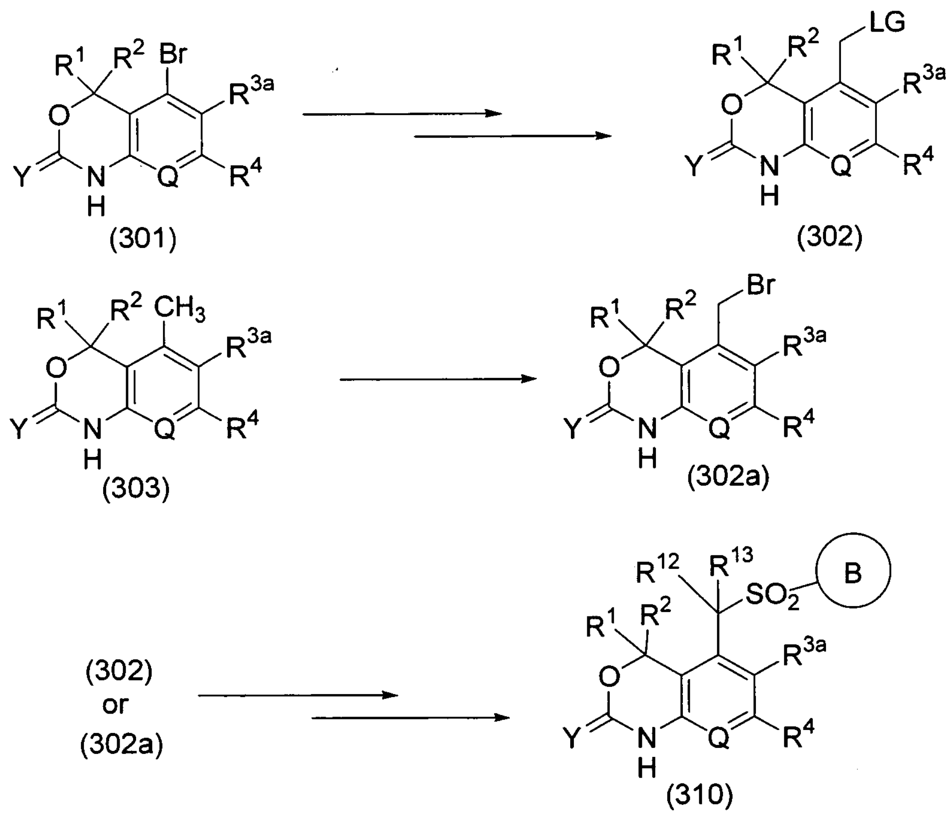

(301) (302) (303) (302a) (302) or (302a) (310)

(wherein B, Q, $R^1$, $R^2$, $R^{3a}$, $R^4$, $R^{12}$, $R^{13}$, Y and LG represent the same meanings mentioned above).
By subjecting the compound (301) or its salt to the same steps as in Production Method 3, via the intermediate (302) the compound (310) can be produced.
As alternative, brominating a compound having a methyl group at its 5-position such as the compound (303) also allows the production of the intermediate (302a). The reaction can be conducted, in the presence of a radical initiator and brominating agent, in an appropriate inert solvent at a temperature ranging from -20°C to the boiling point of the solvent for 10 minutes to 48 hours.
The radical initiator includes, for example, α,α'- azobisisobutylnitrile and benzoyl peroxide.
The brominating agent includes, for example, N-bromosuccinimide and bromine.
The inert solvent includes, for example, acetonitrile; halogenated hydrocarbon solvents such as carbon tetrachloride, chloroform and dichloromethane; aromatic hydrocarbon solvents such as monochlorobenzene and benzene; ester solvents such as ethyl acetate; and a mixture thereof.

Among the compounds represented by the formula (1), the compound represented by the formula (410) or its salt, for example, can be produced through the following method.

Production Method 5

[Chemical Formula 12]

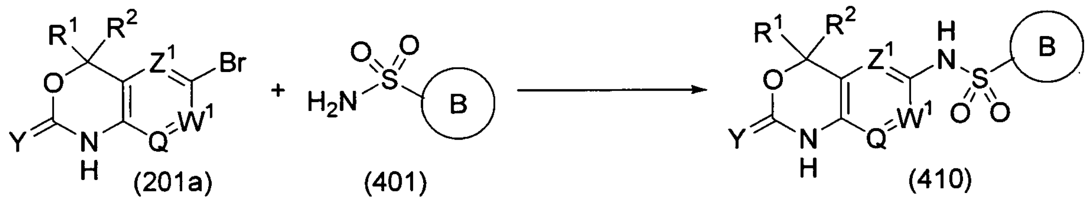

(201a)

(401)

(410)

($Z^1$ or $W^1$ = N)

($Z^1$ or $W^1$ = N)

(wherein B, $R^1$, $R^2$, Q, and Y represent the same meanings mentioned above; and $Z^1$ and $W^1$ represent the above-mentioned Z and W but at least one of $Z^1$ and $W^1$ is a nitrogen atom).
By subjecting the compound (201a) or its salt to reaction with the compound (401) or its salt, the compound (410) ($Z^1$ or $W^1$ = N) can be produced. The reaction can be conducted, in the presence of a base if required, besides in the presence of a metal catalyst and ligand as the case may be, in an appropriate inert solvent at a temperature ranging from about 0°C to the boiling point of the solvent for 10 minutes to 48 hours.
The base includes, for example, organic bases such as triethylamine, pyridine and 2,4,6-collidine; and inorganic bases such as potassium carbonate, cesium carbonate, cesium acetate, sodium hydroxide and sodium hydride.
The metal catalyst includes, for example, copper(I) iodide, copper(II) acetate, palladium(II) acetate and tris(dibenzylideneacetone)dipalladium(0).
The ligand includes, for example, 9,9-dimethyl-4,5-bis (diphenylphosphino)xanthene (Xantphos) and 2-dicyclohexylphosphino-2'-(N,N-dimethylamino)biphenyl.
The inert solvent includes, for example, acetonitrile; ether solvents such as tetrahydrofuran and 1,4-dioxane; polar aprotic solvents such as N,N-dimethylformamide, N-methylpyrrolidone and dimethyl sulfoxide; and a mixture thereof.

Among the compounds represented by the formula (1), the compound represented by the formula (420) or its salt, for example, can be produced through the following method.

Production Method 6

[Chemical Formula 13]

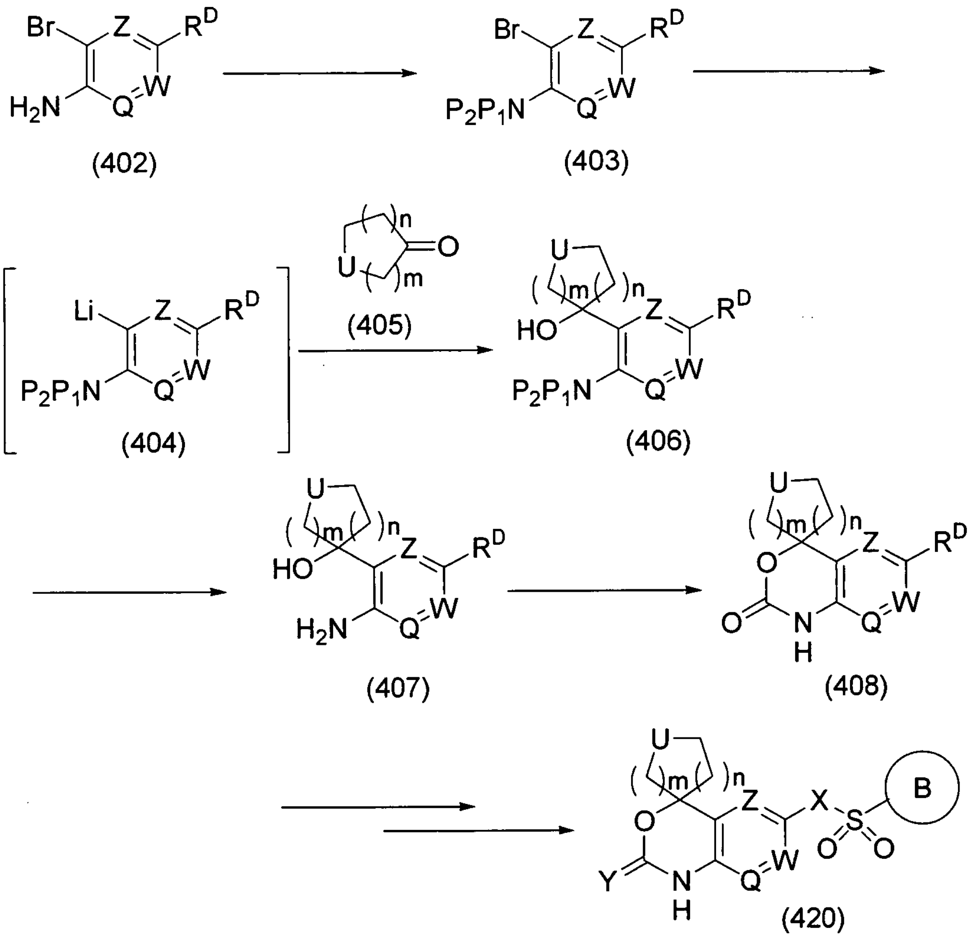
(402) (403) (404) (405) (406) (407) (408) (420)

(wherein B, Q, W, X, Y and Z represent the same meanings mentioned above, $R^D$ represents a hydrogen atom, an appropriately protected oxygen atom or an appropriately protected nitrogen atom, U represents a single bond, oxygen atom, sulfur atom, optionally substituted methylene or optionally substituted amine; m and n each independently represent an integer of 1 to 4 wherein m+n $\leqq$ 5; $P^1$ and $P^2$ each independently represent a protective group (for example, acyl groups such as pivaloyl, alkyloxycarbonyl groups such as tert-butoxycarbonyl or substituted alkyl groups such as dibenzyl) but either of them may represent a hydrogen atom depending on situations.)

Steps 1 to 4 (Introduction of Protective Group - Lithiation - Alkylation - Removal of Protective Group)

The compound (402) or its salt is converted into the intermediate (403) by introducing a protective group on their amino group, followed by reaction with a lithium base to convert into the intermediate (404), and further followed by reaction with the cyclic ketone compound (405) to allow the synthesis of the compound (406). By removing the protective group from this resulting compound, the compound (407) can be produced.
The reaction can be conducted, in the presence of the lithium base, besides in the presence of a chelating agent as the case may be, in an appropriate inert solvent at a temperature ranging from about -100°C to the boiling point of the solvent used for 10 minutes to 48 hours.
The lithium base includes, for example, organolithium reagents such as n-butyllithium, sec-butyllithium, tert-butyllithium and lithiumdiisopropylamide, and lithium metal.
The chelating agent and inert solvent include, for example, those mentioned above (Production Method 3 - Step 1).
As the protective group, conventional protective groups disclosed in literatures (for example, the above-cited Protective Groups in Organic Synthesis) can be used; more specifically, including tert-butoxycarbonyl, dibenzyl, pivaloyl and the like.
The introduction and removal of the protective group can be conducted with methods routinely used in organic chemical

synthesis (for example, refer to the above-cited Protective Groups in Organic Synthesis) or other methods corresponding thereto.

Step 5 (Cyclization)

By subjecting the compound (407) or its salt to reaction with an appropriate carbonyl-donating agent, the cyclic intermediate (408) can be produced. The reaction can be conducted, in the presence of the carbonyl-donating agent, in an appropriate inert solvent at a temperature ranging from about -20°C to the boiling point of the solvent for 10 minutes to 48 hours.
The carbonyl-donating agent includes, for example, 1,1'-carbonylbis-1H-imidazole (CDI), triphosgene and phosgene.
The inert solvent includes, for example, halogenated hydrocarbon solvents such as chloroform and dichloromethane; aromatic hydrocarbon solvents such as toluene; ether solvents such as diethyl ether, tetrahydrofuran and 1,4-dioxane; and a mixture thereof.
The compound (408), when its $R^D$ is hydrogen, is prepared to a compound corresponding to the intermediate (201) of the above-mentioned Production Method 3 through the bromination with routinely used general methods, and then allowed the production of the compound (420) according the method described in Production Method 3. Otherwise, the compound (408), when its $R^D$ is hydrogen, is subjected, through routinely used general methods, to an amination via nitration followed by reduction or, when its $R^D$ is a protected oxygen atom or protected nitrogen atom, is subjected to a removal of protection group through routinely used general methods; consequently respectively prepared to a compound corresponding to the intermediate (101) of the above-mentioned Production Method 1, and then allowed the production of the compound (420) according the method described in the Production Method 1.
Among the compounds represented by the formula (1), the compound represented by the formula (510) or its salt, for example, can be produced through the following method.

Production Method 7

[Chemical Formula 14]

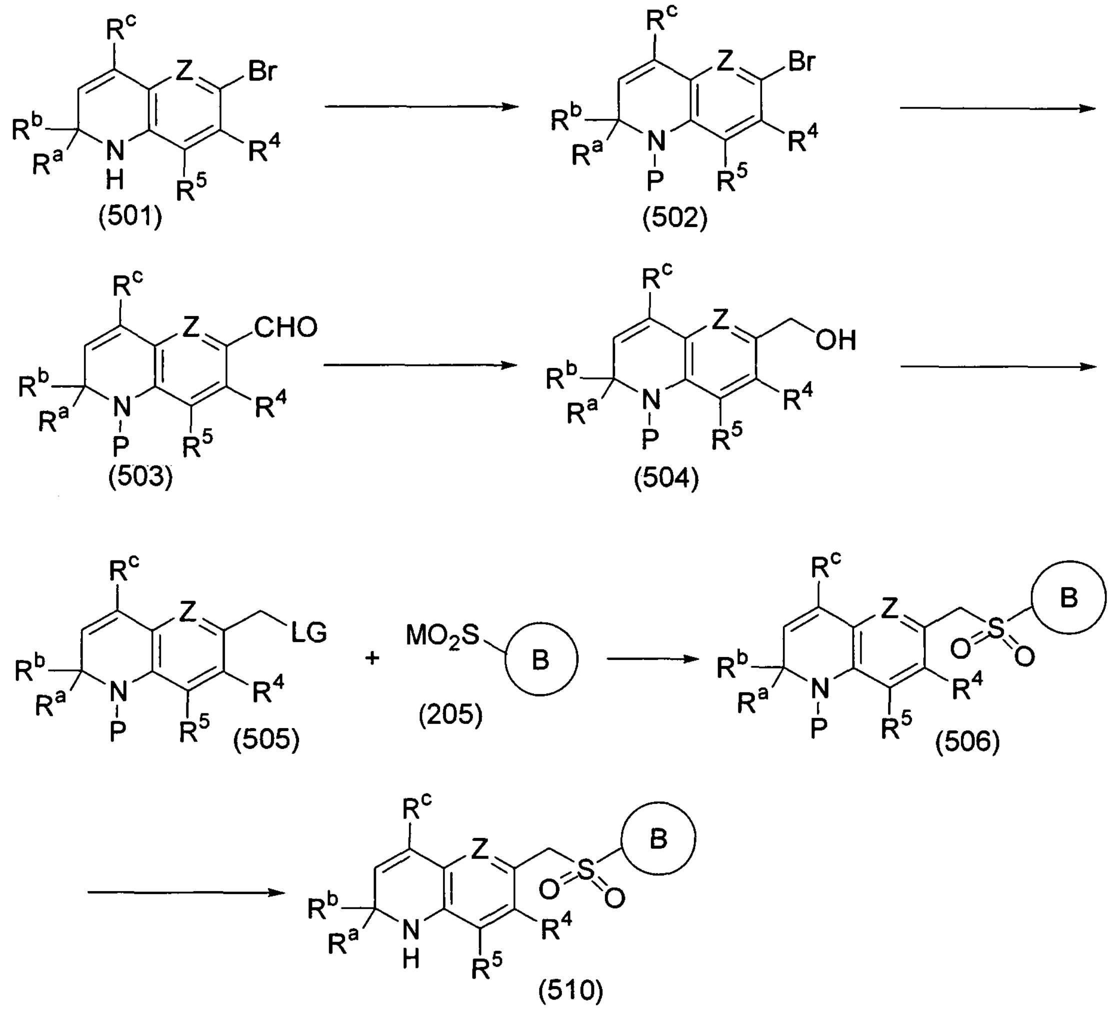

(501) (502) (503) (504) (505) (205) (506) (510)

(wherein B, $R^a$, $R^b$, $R^c$, $R^4$, $R^5$, Z, LG, M, and P represent the same meanings mentioned above.)

The compound (501) or its salt are converted into the intermediate (502) through introduction of a protective group on the NH at 1-position, followed by subjecting to formylation, reduction, introduction of leaving group and sulfonylation steps, as done in the steps 1 to 4 of Production Method 3, to produce the intermediate (506); thereafter, this resultant intermediate is subjected to removal of the protective group, thus the compound (510) can be produced.

As the protective group, conventional protective groups mentioned above (Production Method 3 - Steps 5 to 7) can be used; more specifically, the protective group for the NH at 1-position includes tert-butoxycarbonyl, and the like.

The introduction and removal of the protective group can be conducted with methods routinely used in organic chemical synthesis (for example, refer to the above-cited Protective Groups in Organic Synthesis) or other methods corresponding thereto.

Among the compounds represented by the formula (1), the compound represented by the formula (520) or its salt, for example, can be produced through the following method.

Production Method 8

[Chemical Formula 15]

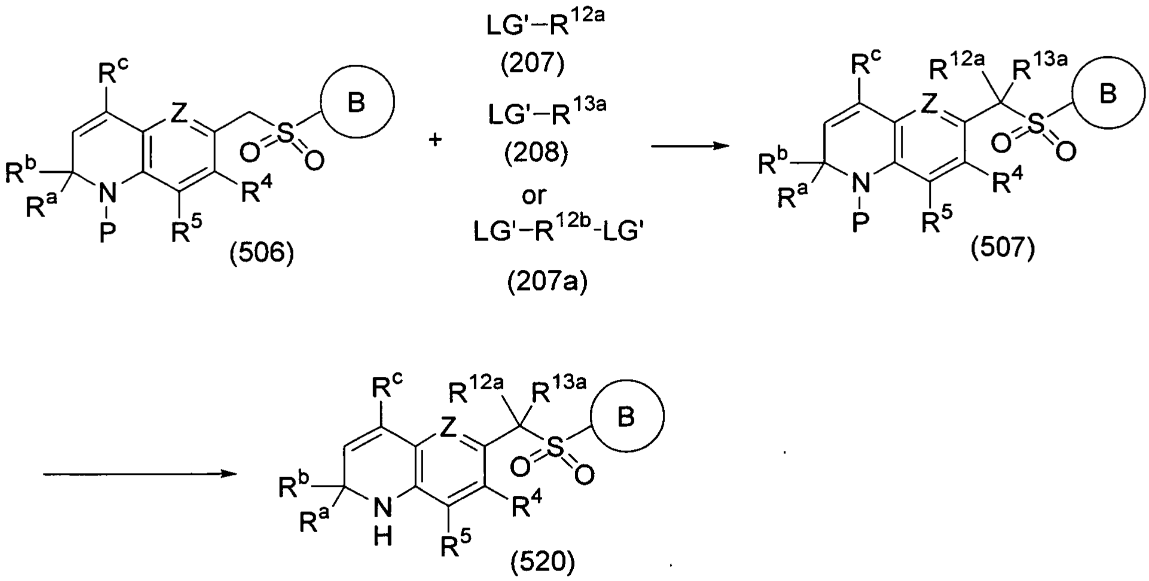


(506) (507) (520)

(wherein B, $R^a$, $R^b$, $R^c$, $R^4$, $R^5$, $R^{12a}$, $R^{13a}$, Z, LG' and P represent the same meanings mentioned above).
By subjecting the compound (506) mentioned above (Production Method 7) to steps of introduction of $R^{12a}$ and $R^{13a}$ and removal of protective group, as done in the Steps 6 to 7 of Production Method 3, the compound (520) can be produced.
As the protective group, conventional protective groups mentioned above (Production Method 3 - Steps 5 to 7) can be used; more specifically, the protective group for the NH at 1-position includes tert-butoxycarbonyl, and the like.
The introduction and removal of the protective group can be conducted with methods routinely used in organic chemical synthesis (for example, refer to the above-cited Protective Groups in Organic Synthesis) or other methods corresponding thereto.

Among the compounds represented by the formula (1), the compound represented by the formula (610) or its salt, for example, can be produced through the following method.

Production Method 9

[Chemical Formula 16]

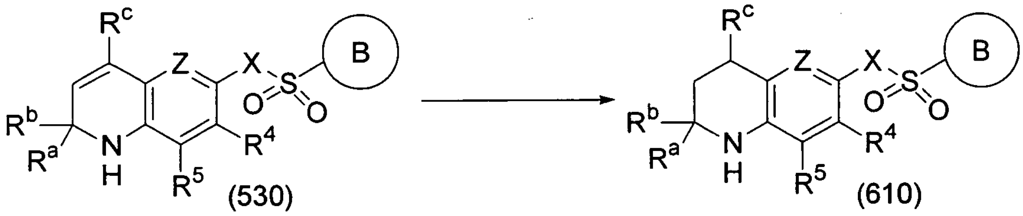

(530) (610)

(wherein B, $R^a$, $R^b$, $R^c$, $R^4$, $R^5$ and X represent the same meanings mentioned above.)
By hydrogenating the compound (530) or its salt which can be produced in the above-mentioned Production Method 1, Production Method 2 and Production Methods 7 and 8, the compound (610) can be produced. As alternative, by hydrogenating the intermediates at any of their appropriate stages in the above-mentioned Production Methods 7 to 8 being capable of producing the compound (530), followed by subjecting to similar steps, the compound (610) can be produced.
The hydrogenation reaction can be conducted under a hydrogen atmosphere of 1 to 5 atmospheres, or be conducted, depending on situations, using ammonium formate in place of the hydrogen, in the presence of a metal catalyst, besides in the presence of an acid as the case may be, in an appropriate inert solvent at a temperature ranging from about 0°C

to the boiling point of the solvent for 10 minutes to 48 hours.
The metal catalyst includes, for example, palladium-carbon, palladium hydroxide-carbon, Raney nickel and platinum oxide.
The acid includes, for example, mineral acids such as hydrochloric acid or organic acids such as formic acid and acetic acid.
The inert solvent includes, for example, alcoholic solvents such as methanol, ethanol and 2-propanol; ether solvents such as tetrahydrofuran; ester solvents such as ethyl acetate; and a mixture thereof.
Among the compounds represented by the formula (1), the compound represented by the formula (620) or its salt, for example, can be also produced through the following method.

Production Method 10

[Chemical Formula 17]

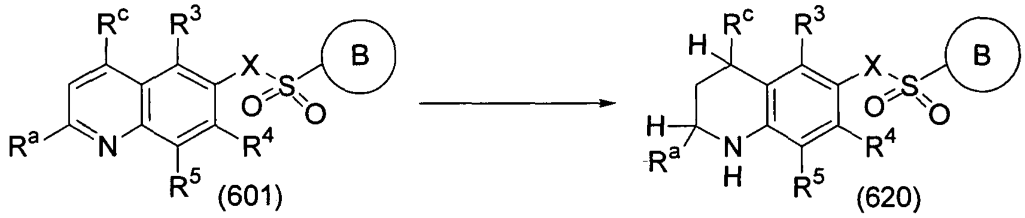

(wherein B, $R^a$, $R^c$, $R^3$, $R^4$, $R^5$ and X represent the same meanings mentioned above).
The compound (620) can be produced through reduction of the quinoline A ring (nitrogen-containing ring) portion of the compound (601) or its salt. As alternative, by applying quinoline derivatives' form to the starting materials or intermediates of the above-mentioned Production Methods 7 to 8 being capable of producing the compound (530), and then subjecting to a similar reduction at an appropriate stage, followed by subjecting to similar steps, the compound (620) can be produced.
The reduction reaction can be conducted in an alcoholic solvent such as methanol and ethanol by using sodium borohydride and nickel chloride at a temperature ranging from 0°C to the boiling point of the solvent for 10 minutes to 48 hours; otherwise, being conducted under a hydrogen atmosphere of 1 to 5 atmospheres or with the use of ammonium formate in place of the hydrogen depending on situations, in the presence of a metal catalyst, besides in the presence of an acid as the case may be, in an appropriate inert solvent at a temperature ranging from 0°C to the boiling point of the solvent for 10 minutes to 48 hours.
The metal catalyst, acid and inert solvent includes those described above (Production Method 9).
Among the compounds represented by the formula (1), the compound represented by the formula (710) or its salt, for example, can be produced through the following method.

Production Method 11

[Chemical Formula 18]

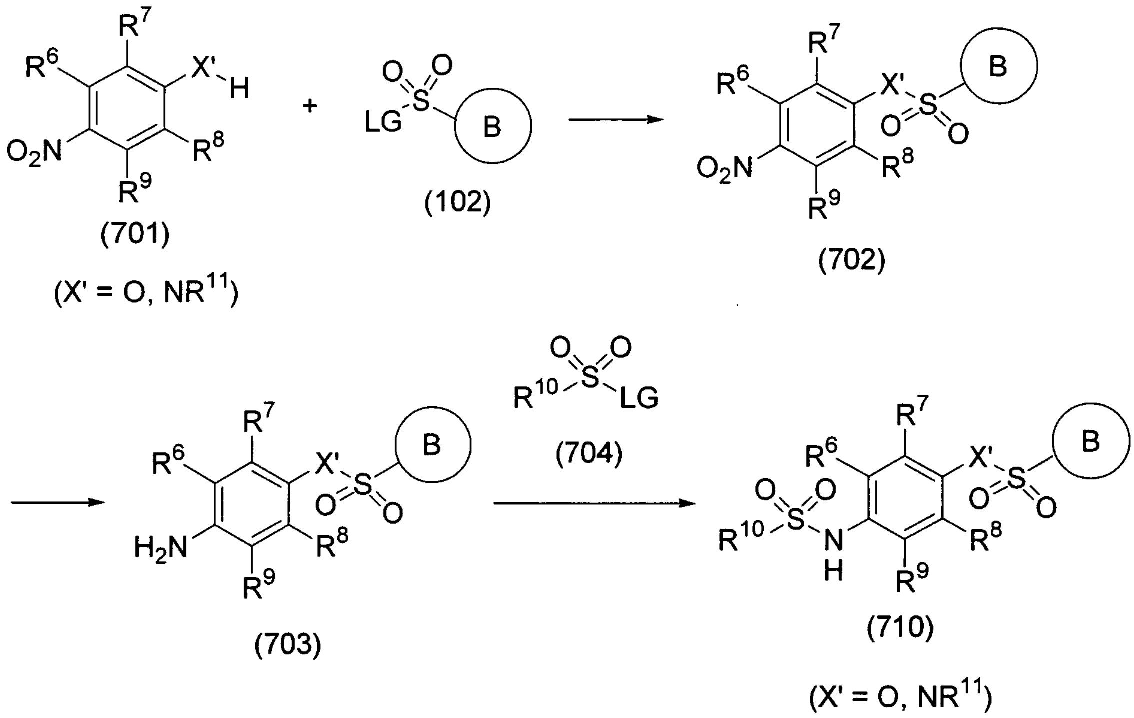

(701) (X' = O, NR$^{11}$) (102) (702) (703) (704) (710) (X' = O, NR$^{11}$)

(wherein B, $R^6$, $R^7$, $R^8$, $R^9$, $R^{10}$ $R^{11}$, X' and LG represent the same meanings mentioned above).

Step 1 (Sulfonylation)

By subjecting the compound (701) or its salt to a reaction with the compound (102) or its salt through the same method as mentioned above (Production Method 1), the intermediate (702) can be produced.

Step 2 (Reduction)

By reducing the compound (702) or its salt to an aniline derivative, the intermediate (703) can be produced. The reaction can be conducted through the same hydrogenation as mentioned above (Production Method 9) or conducted, in the presence of a metal reducing agent, in an appropriate inert solvent at a temperature ranging from about 0°C to the boiling point of the solvent for 10 minutes to 48 hours.
The metal reducing agent includes, for example, tin(II) chloride, reduced iron and titanium(III) trichloride.
The inert solvent includes, for example, water, diluted hydrochloric acid, acetic acid, acetone, acetonitrile, alcoholic solvents such as methanol, ethanol and 2-propanol; ether solvents such as tetrahydrofuran and 1,2-dimethoxyethane; ester solvents such as ethyl acetate; polar aprotic solvents such as N,N-dimethylformamide; and a mixture thereof.

Step 3 (Sulfonamidation)

By subjecting the compound (703) or its salt to a reaction with the compound (704) or its salt through the same method as mentioned above (Production Method 1), the compound (710) (X' = O, NR$^{11}$) can be produced.
Among the compounds represented by the formula (1), the compound represented by the formula (720) or its salt, for example, can be produced through the following method.

Production Method 12

[Chemical Formula 19]

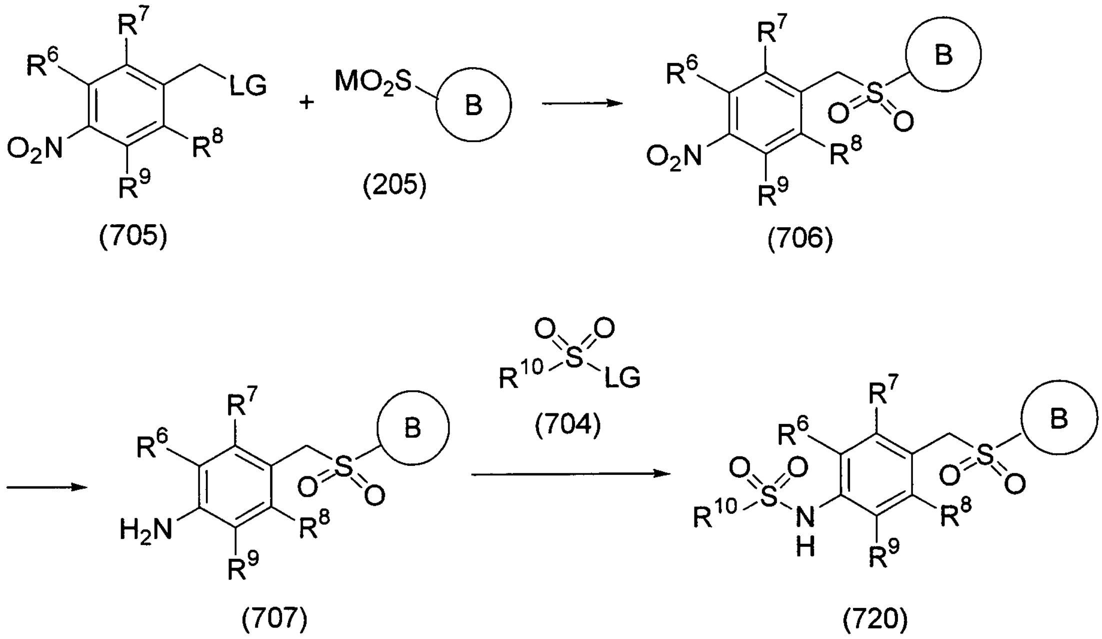

(705) (205) (706) (704) (707) (720)

(wherein B, $R^6$, $R^7$, $R^8$, $R^9$, $R^{10}$, LG and M represent the same meanings mentioned above).

Step 1 (Sulfonylation)

By subjecting the compound (705) or its salt to a reaction with the compound (205) or its salt through the same method as mentioned above (Production Method 3 - Step 4), the intermediate (706) can be produced.

Step 2 (Reduction)

By reducing the compound (706) or its salt to an aniline derivative through the same method as mentioned above (Production Method 11 - Step 2), the intermediate (707) can be produced.

Step 3 (Sulfonamidation)

By subjecting the compound (707) or its salt to a reaction with the compound (704) or its salt through the same method as mentioned above (Production Method 1), the compound (720) can be produced.

Production Method of Intermediate

Among the compound (101), the compounds of which A represents the formula (A-1), formula (A-2), formula (A-6) or formula (A-7), that is, the compounds (201), (301), (303) and (201a), can be prepared according to the methods disclosed in the literatures (International Publication Pamphlet WO98/14436, International Publication Pamphlet WO00/66570, International Publication Pamphlet WO00/66592 and International Publication Pamphlet WO01/16108) or other methods corresponding thereto.
Among the compound (101), the compounds of which A represents the formula (A-3) or formula (A-4) and the compound (501) can be prepared according to the methods disclosed in the literatures (International Publication Pamphlet WO96/19458, Tetrahedron Lett., (2002), 43, 3907-3910) and International Publication Pamphlet WO03/004028) or to other methods corresponding thereto.
The compound (601) can be prepared with the usage of commercial quinoline derivatives, or by optionally converting a functional group of the derivatives; and the compound (701) and compound (705) can be also prepared with the usage of commercial nitrobenzene derivatives, or by optionally converting a functional group of the derivatives. The functional group conversion can be conducted with conventional methods routinely used (for example, refer to Comprehensive Organic Transformations, R.C. Larock, (1989)).

The compound (102) can be prepared with the usage of commercial aryl- or heteroaryl-sufonyl halide derivatives, or through an optional functional group conversion from aryl- or heteroaryl-sulfonic acid derivatives or aryl- or heteroaryl-thiol derivatives. The functional group conversion can be conducted with conventional methods routinely used. As alternative, the compound (102) can be prepared by the reaction of an aryl- or heteroaryl-derivative and chlorosulfuric acid. The reaction can be conducted through directly mixing both reactants, besides in an appropriate inert solvent as the case may be, at a temperature ranging from about -20°C to 140°C for 10 minutes to 168 hours.

The inert solvent includes, for example, halogenated hydrocarbon solvents such as chloroform; trifluoroacetic acid, thionyl chloride, and a mixture thereof.

The compound (103) can be prepared with the usage of commercial alkyl halide derivatives, alkanoyl halide derivatives, aroyl halide derivatives, alkoxycarbonyl halide derivatives or arylsulfonyl halide derivatives, or through an optional functional group conversion from alkyl alcoholic derivatives, alkyl- or aryl-carboxylic acid derivatives or alkyl- or aryl-sulfonic acid derivatives. The functional group conversion can be conducted with conventional methods routinely used.

The compound (205) can be prepared with the usage of commercial aryl- or heteroaryl-sulfinic acid derivatives, or their salts, or through an optional functional group conversion from aryl- or heteroaryl-sulfonyl halide derivatives, aryl- or heteroaryl-sulfonic acid derivatives or aryl- or heteroaryl-thiol derivatives. The functional group conversion can be conducted with conventional methods routinely used.

The compound (207), (207a) and (208) can be prepared with the usage of commercial alkyl halide derivatives, or through an optional functional group conversion from alkyl alcohol derivatives. The functional group conversion can be conducted with conventional methods routinely used.

The compound (401) can be prepared with the usage of commercial aryl- or heteroaryl-sulfonamide derivatives, or through condensing an aryl or heteroaryl-sulfonyl halide derivative and ammonia. The reaction can be conducted with conventional methods routinely used.

The compound (402) can be prepared with the usage of commercial 2-bromoaniline derivatives or corresponding nitrogen-containing derivative, or by brominating aniline or an aminopyridine derivative. The reaction can be conducted with conventional methods routinely used.

The compound (704) can be prepared with the usage of commercial alkylsulfonyl halide derivatives, or through an optional functional group conversion from alkylsulfonic acid derivatives or alkylthiol derivatives. The functional group conversion can be conducted with conventional methods routinely used.

Furthermore, the compound represented by the above-mentioned formula (1) may be converted to another compound represented by the formula (1) through optionally converting its functional group. The functional group conversion can be conducted with conventional methods routinely used (for example, refer to the above-cited Comprehensive Organic Transformations).

Moreover, especially a halogen group or the trifluoromethanesulfonyloxy group derived from hydroxyl group, and the like are convertible for an alkenyl group, alkynyl group, aryl group, heteroaryl group, cyano group, amino group, etc. with an addition reaction which uses metal catalysts such as palladium.

In the present description, protective groups, condensing agents and the like may be expressed with their abbreviations according to IUPAC-IUB (Nomenclature Committee of the International Union of Biochemistry), which are commonly used in this art.

Salts suitable to starting compounds and intended compounds, and acceptable as medicaments are commonly used nontoxic salts, which include acid addition salts such as organic acid salts (for example, acetate, trifluoroacetate, maleate, fumarate, citrate, tartrate, methanesulfonate, benzenesulfonate, formate and toluenesulfonate) and inorganic acid salts (for example, hydrochloride, hydrobromide, hydroiodide, sulfate, nitrate and phosphate); salts with amino acids (for example, arginine, asparagic acid and glutamic acid); metal salts such as alkali metal salts (for example, sodium salt and potassium salt) and alkaline earth metal salts (for example, calcium salt and magnesium salt); ammonium salt; and organic base salts (for example, trimethylamine salt, triethylamine salt, pyridine salt, picoline salt, dicyclohexylamine salt and N,N'-dibenzylethylenediamine salt); and the like, and which can be optionally selected by the person skilled in the art.

If, in the above-mentioned production methods, any of functional groups excluding the group positioned at the intended reaction site are changed under the explained reaction conditions or are unsuitable for conducting the explained methods, the site outside the intended reaction site may be protected and then de-protected after the reaction to obtain intended compounds. As the protective group, conventional protective groups disclosed in literatures (for example, the above-cited Protective Groups in Organic Synthesis) can be used; more specifically, being exemplifying with, as protective groups for amine, ethoxycarbonyl, tert-butoxycarbonyl, acetyl and benzyl, and as protective groups for hydroxyl group, trialkylsilyl groups such as trimethylsilyl, acetyl and benzyl.

The introduction and removal of protective groups can be conducted with methods routinely used in organic chemical synthesis (for example, refer to the above-cited Protective Groups in Organic Synthesis) or other methods corresponding thereto.

Intermediates and intended compounds in each of the above-mentioned production methods can be isolated and purified

by being subjected to methods routinely used in synthetic organic chemistry, for example, the methods such as neutralization, filtration, extraction, washing, drying, concentration, recrystallization and various chromatographies. Moreover, the intermediates can be served to following reactions without being particularly purified.

Although some of the compounds (1) of the present invention may have their tautomers, the present invention encompasses, including such compounds, all the possible isomers and mixtures thereof.

To acquire pharmaceutically acceptable salts of the compound (1) of the present invention, if the compound (1) is obtainable in the form of the pharmaceutically acceptable salts, it can be acquired by purifying as it is; while, if obtainable in a free form, it can be acquired by dissolving or suspending in an appropriate organic solvent, followed by addition of an acid or base to form its salt through a conventional method. Moreover, although the compound (1) and its pharmaceutically acceptable salt may exist in the form of an addition product with water or various solvents, these addition products are also encompassed by the present invention. Although the compound (1) of the present invention may have one or more stereoisomers based on an asymmetric carbon atom, all of these isomers and the mixtures thereof are encompassed in the scope of the invention.

Furthermore, prodrugs of the compound (1) of the present invention are also encompassed in the scope of the present invention. In the present invention, a prodrug means a derivative which is decomposed through acid hydrolysis or enzymically in the living body to give the compound represented by the above-mentioned formula (1). For example, when the compound represented by the above-mentioned formula (1) has a hydroxyl group, amino group or carboxy group, a prodrug can be manufactured by modifying the group in accordance with a routine method.

For example, if the compound has a carboxy group, included are the compounds in which the carboxy group is turned into an alkoxycarbonyl group, into an alkylthiocarbonyl group or into an alkylaminocarbonyl group.

Moreover, for example, if the compound has an amino group, included are the compounds in which the amino group is substituted with an alkanoyl group to turn into an alkanoylamino group, or substituted with an alkoxycarbonyl group to turn into an alkoxycarbonylamino group, into an alkanoyloxymethylamino group or into a hydroxylamine.

Moreover, for example, if the compound has a hydroxyl group, included are the compounds in which the hydroxyl group is substituted with the above-mentioned alkanoyl group to turn into an alkanoyloxy group, into a phosphate or into an alkanoyloxymethyloxy group.

An alkyl portion of the group being used for making a prodrug includes the above-mentioned alkyl group, the alkyl group, for example, may be substituted with an alkoxy group and the like. Preferred examples include the followings.

In the case exemplified with the compound in which a carboxyl group turn into an alkoxycarbonyl group: included are alkoxycarbonyl such as methoxycarbonyl and ethoxycarbonyl, or alkoxycarbonyl substituted with alkoxy group such as methoxymethoxycarbonyl, ethoxymethoxycarbonyl, 2-methoxyethoxycarbonyl, 2-methoxyethoxymethoxycarbonyl and pivaloyloxymethoxycarbonyl.

Invention Compound has high binding affinities to aldosterone receptor MR and pharmacologic actions as an aldosterone receptor modulator such as antagonist activities or partial agonist activities; therefore, being useful for treating or preventing cardiovascular diseases such as hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism and inflammations.

For the medical purpose, the compound of the present invention can be used in the form of medical formulations as mixtures with pharmaceutically acceptable carriers such as organic or inorganic solid or liquid excipients which are suitable to oral or parenteral administering or external uses including topical, enteral, intravascular, intramuscular, inhalational, nasal, intraarticular, intrathecal, transbronchial or ophthalmic routes. The medical formulations include solids, semi-solids or liquids such as capsules, tablets, pellets, sugar-coated tablets, powders, granules, suppositories, ointments, creams, lotions, inhalants, injections, cataplasms, gels, tapes, eye drops, liquid medicines, syrups, aerosols, suspensions and emulsions. These formulations can be manufactured through routine methods. Depending on requirements, these formulations can be added with preparation auxiliaries, stabilizers, wetting agents or emulsifiers, buffering agents, and other conventional additives.

Dosages of Invention Compound vary depending on patients' ages and states, while, as an average dose of the compound (1) per one time, about 0.1mg, 1mg, 10mg, 50mg, 100mg, 250mg, 500mg, and 1,000mg are effective for cardiovascular diseases such as hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism and inflammations. When administering to humans, the dose capable to be administered is generally 0.1mg per day/individual to about 1,000mg per day/individual, preferably 1 mg per day/individual to about 100mg per day/individual.

The present invention will be explained more detail with referring Examples and Test Examples, but the invention is never limited thereto. In the present description, the following abbreviations may be used for simply describing.

Me: Methyl
Et: Ethyl

Pr : Propyl
i-Pr: Isopropyl
t-Bu: tert-Butyl
Ph: Phenyl
Py: Pyridyl
Bn: Benzyl
Boc: tert-Butoxycarbonyl

Examples

Reference example 1

Methyl 2-amino-5-methoxybenzoate

To an ethyl acetate (14mL) solution of methyl 5-methoxy-2-nitrobenzoate (1.60g, 7.58 mmol), 10% palladium-carbon (containing 50% water) (320mg) was added and stirred under a hydrogen atmosphere of 0.25MPa at 20-25°C for 3 hours. After the reaction, palladium-carbon was filtered off and the solvent was distilled off under reduced pressure. The obtained residue was purified by silica gel chromatography (hexane/ethyl acetate = 10/1 - 5/1) to give methyl 2-amino-5-methoxybenzoate (1.29g, 94%) $^1$H-NMR ($CDCl_3$) δ :3.76(3H,s), 3.88(3H,s), 5.37(2H,br), 6.63(1H,d,J=8.8Hz), 6.95(1H,dd,J=8.8, 3.1Hz), 7.35(1H,d,J=3.1Hz)

Reference example 2

2-(2-Amino-5-methoxyphenyl)propan-2-ol

To a tetrahydrofuran (4.0mL) solution of methyl 2-amino-5-methoxybenzoate (362mg, 2.00 mmol), 0.93M methylmagnesium bromide-tetrahydrofuran solution (8.60mL, 8.00 mmol) was added at 0°C, and then, the mixture was warmed to 20-25°C and stirred for 2.5 hours. After the reaction, aqueous ammonium chloride was added thereto, and the mixture was extracted with ethyl acetate. The organic layer was washed with aqueous ammonium chloride and saturated aqueous sodium chloride, subsequently, and dried over anhydrous magnesium sulfate. After filtration, the solvent was distilled off under reduced pressure. The obtained residue was purified by silica gel column chromatography (hexane/ethyl acetate = 3/1 - 2/1) to give 2-(2-amino-5-methoxyphenyl)propan-2-ol (336mg, 93%).
$^1$H-NMR ($CDCl_3$) δ :1.65(6H,s), 3.75(3H,s), 6.62(1H,d,J=8.5Hz), 6.67(1H,dd, J=8.5, 2.7Hz), 6.76(1H,d,J=2.7Hz)

Reference example 3

6-Methoxy-4,4-dimethyl-1,4-dihydro-2H-3,1-benzoxazin-2-one

To a 1,4-dioxane (9.5mL) solution of 2-(2-amino-5-methoxyphenyl) propan-2-ol (338mg, 1.86 mmol), triphosgene (194mg, 0.651 mmol) was added at 20-25°C, and then, the mixture was refluxed under heating for one hour. After the reaction, the solvent was distilled off under reduced pressure. The obtained residue was subjected to recrystallization from a mixture of chloroform/ethyl acetate/hexane to give 6-methoxy-4,4-dimethyl-1,4-dihydro-2H-3,1-benzoxazin-2-one (302mg, 78%).
$^1$H-NMR ($CDCl_3$) δ :1.71(6H,s), 3.79(3H,s), 6.70(1H,s), 6.78(2H,m), 8.68(1H,br.s)

Reference example 4

6-Hydroxy-4,4-dimethyl- 1,4-dihydro-2H-3, 1-benzoxazin-2-one

To a dichloromethane (8.0mL) solution of 6-methoxy-4,4-dimethyl-1,4-dlhydro-2H-3,1-benzoxazin-2-one (104mg, 0.500 mmol), 1M boron tribromide-dichloromethane solution (1.00mL, 1.00 mmol) was added at -78°C, and then, the mixture was warmed to 20-25°C and stirred for 4 hours. After the reaction, water was added to the mixture at 0°C and followed by extraction with ethyl acetate. The organic layer was washed with saturated aqueous sodium chloride and dried over anhydrous magnesium sulfate. After filtration, the solvent was distilled off under reduced pressure. The obtained residue was purified by silica gel column chromatography (chloroform/ethyl acetate = 3/1, further added 3% methanol and eluted) to give 6-hydroxy-4,4-dimethyl-1,4-dihydro-2H-3,1-benzoxazin-2-one (93mg, 96%).
$^1$H-NMR (DMSO-$d_6$) δ :1.53(6H,s), 6.62-6.70(3H,m), 9.20(1H,s), 9.89(1H,s)

Reference example 5

(2-Amino-5-methoxyphenyl)methanol

To a tetrahydrofuran (8.0mL) suspension of lithium aluminum hydride (228mg, 6.00 mmol), a tetrahydrofuran (8.0mL) solution of the compound prepared by Reference example 1 was added dropwise at 0°C over 25 minutes, and then, the mixture was warmed to 20-25°C and further stirred for 2 hours. After the reaction, water, 2N aqueous sodium hydroxide solution and water were subsequently added dropwise, and ethyl acetate and anhydrous magnesium sulfate were added and stirred. After filtration, the solvent was distilled off under reduced pressure. The obtained residue was purified by silica gel column chromatography (hexane/ethyl acetate = 1/1) to give (2-amino-5-methoxyphenyl)methanol (235mg, 77%).
LC-MS (M+1): 154.1

Reference example 6

2-(2-Amino-5-bromophenyl)propan-2-ol

To a diethyl ether (100mL) solution of methyl 3-bromoanthranilate (9.20g, 40.0 mmol), 3.0M methylmagnesium bromide-tetrahydrofuran solution (55mL, 165 mmol) was added dropwise under ice-cooling and stirred at 0°C for 3.5 hours. The reaction solution was poured into an aqueous ammonium chloride solution at 0°C and extracted with ethyl acetate. The organic layer was dried over anhydrous magnesium sulfate. The solvent was distilled off under reduced pressure, and the obtained residue was purified by silica gel column chromatography (hexane/ethyl acetate = 4/1) to give 2-(2-amino-5-bromophenyl)propan-2-ol (5.72g, 62%).
$^1$H-NMR ($CDCl_3$) $\delta$ :1.65(6H,s), 6.52(1H,d,J=8.4Hz), 7.14(1H,dd,J=8.4, 2.2Hz), 7.20(1H,d,J=2.4Hz)

Reference example 7

6-Bromo-4,4-dimethyl-1,4-dihydro-2H-3,1-benzoxazin-2-one

6-Bromo-4,4-dimethyl-1,4-dihydro-2H-3,1-benzoxazin-2-one was produced by the same method as Reference example 3 from 2-(2-amino-5-bromophenyl)propan-2-ol.
$^1$H-NMR ($CDCl_3$) $\delta$ :1.71(6H,s), 6.73(1H,d,J=8.4Hz), 7.36(1H,dd,J=8.4, 2.1Hz), 8.61(1H,s)

Reference example 8

4,4-Dimethyl-2-oxo-1,4-dihydro-2H-3,1-benzoxazin-6-carbaldehyde

To a tetrahydrofuran (5mL) solution of 6-bromo-4,4-dimethyl-1,4-dihydro-2H-3,1-benzoxazin-2-one (173mg, 0.676 mmol), 1.58M n-butyllithium-hexane solution (1.4mL, 2.2 mmol) was added dropwise at -70°C and stirred for 30 minutes. After that, N,N-dimethylformamide (0.20mL, 2.6 mmol) was added dropwise and the mixture was warmed to 20-25°C and stirred for 2 hours. The reaction liquid was poured into water and extracted with ethyl acetate. The organic layer was dried over anhydrous magnesium sulfate. The solvent was distilled off under reduced pressure and the obtained residue was purified by silica gel column chromatography (hexane/ethyl acetate = 2/1 - 1/1) to give 4,4-dimethyl-2-oxo-1,4-dihydro-2H-3,1-benzoxazin-6-carbaldehyde (86.3mg, 62%).
$^1$H-NMR ($CDCl_3$) $\delta$ :1.78(6H,s), 6.98(1H,d,J=8.0Hz), 7.72(1H,s), 7.79(1H,dd, J=8.4, 2.0Hz), 9.91 (1H,s)

Reference example 9

6-(Hydroxymethyl)-4,4-dimethyl-1,4-dihydro-2H-3,1-benzoxazin-2-one

To a mixed solution of tetrahydrofuran (0.50mL) and methanol (2.0mL) of 4,4-dimethyl-2-oxo-1,4-dihydro-2H-3,1-benzoxazin-6-carbaldehyde (84.0mg, 0.409 mmol), sodium borohydride (30.6mg, 0.81 mmol) was added under ice-cooling and stirred at 20-25°C for 1.5 hours. The solvent was distilled off under reduced pressure and saturated aqueous ammonium chloride was added to the obtained residue. The mixture was extracted with ethyl acetate, and the organic layer was dried over anhydrous magnesium sulfate. The solvent was distilled off under reduced pressure and the obtained residue was purified by thin layer chromatography (hexane/ethyl acetate = 1/2) to give 6-(hydroxymethyl)-4,4-dimethyl-1,4-dihydro-2H-3,1-benzoxazin-2-one (65.1mg, 77%).
$^1$H-NMR ($CDCl_3$) $\delta$ :1.73(6H,s), 4.67(1H,d,J=5.5Hz), 6.79(1H,d,J=8.1Hz), 7.18(1H,s), 7.21-7.25(1H,m) 7.92 (1H,s)

Reference example 10

Methyl 2-methyl-6-nitrobenzoate

A mixture of 2-methyl-6-nitrobenzoic acid (3.62g, 20.0 mmol) and thionyl chloride (15mL) was stirred at 80°C for 3.5 hours. An excess of the thionyl chloride was distilled off under reduced pressure and the obtained residue was dissolved with methylene chloride (5mL). The solution was added dropwise to a methanol (10mL) solution of triethylamine (3.0mL) at 0°C and stirred at 20-25°C for 3 hours. The solvent was distilled off under reduced pressure. A saturated aqueous sodium bicarbonate solution was added to the obtained residue and the mixture was extracted with ethyl acetate. The organic layer was dried over anhydrous magnesium sulfate. The solvent was distilled off under reduced pressure to give methyl 2-methyl-6-nitrobenzoate (3.82g, 98%).
$^1$H-NMR ($CDCl_3$) δ :2.42(3H,s), 3.97(3H,s), 7.50(1H,t,J=7.9Hz), 7.55(1H,d, J=6.4Hz), 7.99(2H,d,J=8.0Hz)

Reference example 11

Methyl 2-amino-6-methylbenzoate

A mixture of methyl 2-methyl-6-nitrobenzoate (3.80g, 19.5 mmol) and 10% palladium-carbon (containing 50% water) (1.03g) was stirred under a hydrogen atmosphere in methanol (20mL) at 20-25°C for 1.5 hours. After filtering off palladium-carbon, the solvent was distilled off under reduced pressure to give methyl 2-amino-6-methylbenzoate (3.13g, 97%).
$^1$H-NMR ($CDCl_3$) δ :2.43(3H,s), 3.88(3H,s), 2.37(3H,s), 6.52(1H,s), 6.54(1H,s), 7.08(1H,t,J=7.9Hz)

Reference example 12

2-(2-Amino-6-methylphenyl)propan-2-ol

2-(2-Amino-6-methylphenyl)propan-2-ol was produced by the same method as Reference example 6 from 2-(2-amino-6-methylphenyl)propan-2-ol.
$^1$H-NMR ($CDCl_3$) δ :1.73(6H,s), 2.43(3H,s), 6.49-6.54(2H,m), 6.90(1H,t, J=7.7Hz)

Reference example 13

4,4,5-Trimethyl-1,4-dihydro-2H-3,1-benzoxazin-2-one

4,4,5-Trimethyl-1,4-dihydro-2H-3,1-benzoxazin-2-one was produced by the same method as Reference example 3 from 2-(2-amino-6-methylphenyl)propan-2-ol.
$^1$H-NMR ($CDCl_3$) δ :1.80(6H,s), 2.41(3H,s), 6.67(1H,d,J=7.7Hz), 6.85(1H,d, J=7.1Hz), 7.10(1H,d,J=7.7Hz)

Reference example 14

Benzyl 5-(dibenzylamino)-2-nitrobenzoate

To a toluene (5.0mL) suspension of 5-amino-2-nitrobenzoic acid (182mg, 1.00 mmol), diisopropylethylamine (697 μL, 4.00 mmol), benzyl bromide (416 μL, 3.50 mmol) and tetrabutylammonium iodide (111mg, 0.30 mmol) were added and refluxed under heating with stirring for 6 hours. Water was poured into the reaction liquid, which was followed by extraction with ethyl acetate. The separated organic layer was washed with saturated aqueous sodium chloride and dried over anhydrous sodium sulfate. The solvent was distilled off under reduced pressure, and the obtained residue was purified by silica gel column chromatography (hexane/ethyl acetate = 20/1 - 6/1) to give benzyl 5-(dibenzylamino)-2-nitrobenzoate (318mg, 70%).
$^1$H-NMR ($CDCl_3$) δ :4.73(4H,s), 5.31(2H,s), 6.69(1H,dd,J=9.2, 2.9Hz), 6.75(1H, d,J=2.9Hz), 7.15-7.18(4H,m), 7.28-7.39 (11H,m), 7.97(1H,d,J=9.2Hz)

Reference example 15

Benzyl 2-amino-5-(dibenzylamino)benzoate

To an ethanol (7.0mL) solution of benzyl 5-(dibenzylamino)-2-nitrobenzoate (318mg, 0.703 mmol), tin (II) chloride dihydrate (793mg, 3.51 mmol) was added and refluxed under heating with stirring for 3.5 hours. After allowing to be cooled, the reaction liquid was diluted with ethyl acetate. Aqueous sodium bicarbonate was added thereto, and the mixture was filtered and extracted with ethyl acetate. The organic layer was washed with saturated aqueous sodium chloride and dried over anhydrous magnesium sulfate. The solvent was distilled off under reduced pressure to give benzyl 2-amino-5-(dibenzylamino)benzoate (290mg, 97%).
$^1$H-NMR ($CDCl_3$) $\delta$ :4.46(4H,s), 5.23(2H,s), 6.55(1H,d,J=9.0), 6.89(1H,dd,J=9.0, 3.1Hz), 7.19-7.37(16H,m)

Reference example 16

2-[2-Amino-5-(dibenzylamino)phenyl]propan-2-ol

2-[2-Amino-5-(dibenzylamino)phenyl]propan-2-ol was produced by the same method as Reference example 2 from benzyl 2-amino-5-(dibenzylamino)benzoate.
$^1$H-NMR ($CDCl_3$) $\delta$ :1.47(6H,s), 3.59(2H,br.s), 4.49(4H,s), 6.51-6.58(3H,m), 7.19-7.36(10H,m)

Reference example 17

6-(Dibenzylamino)-4,4-dimethyl-1,4-dihydro-2H-3,1-benzoxazin-2-one

6-(Dibenzylamino)-4,4-dimethyl-1,4-dihydro-2H-3,1-benzoxazin-2-one was produced by the same method as Reference example 3 from 2-[2-amino-5-(dibenzylamino)phenyl]propan-2-ol.
$^1$H-NMR ($CDCl_3$) $\delta$ :1.55(6H,s), 4.60(4H,s), 6.46(1H,d,J=2.4Hz), 6.58(1H,d, J=8.6Hz), 6.62(1H,dd,J=8.6, 2.4Hz), 7.23-7.39(10H,m)

Reference example 18

6-Amino-4,4-dimethyl-1,4-dihydro-2H-3,1-benzoxazin-2-one

To a mixed solution of methanol (6.0mL) - ethyl acetate (6.0mL) of 6-(dibenzylamino)-4,4-dimethyl-1,4-dihydro-2H-3,1-benzoxazin-2-one (181mg, 0.486 mmol), 10% palladium hydroxide-carbon (containing 50% water) (60mg) was added and stirred under a hydrogen atmosphere of 0.2MPa at 20-25°C for 2.5 hours. After the reaction, the catalyst was filtered off and the solvent was distilled off under reduced pressure to give 6-amino-4,4-dimethyl-1,4-dihydro-2H-3,1-benzoxazin-2-one (99mg, 100%).
$^1$H-NMR ($CDCl_3$) $\delta$ :1.67(6H,s), 3.25(2H,br.s), 6.48(1H,d,J=2.4Hz), 6.57(1H, dd,J=8.3, 2.4Hz), 6.70(1H,d,J=8.3Hz), 9.31 (1H,br.s)

Reference example 19

2-(1-Ethoxyvinyl)-3-nitropyridine

To a toluene (122 mL) solution of 2-chloro-3-nitropyridine (3.87g, 24.4 mmol) and tributyl-1-ethoxyvinyltin (9.70g, 26.9 mmol), triphenylphosphine (384mg, 1.46 mmol) and tetrakis(triphenylphosphine)palladium (564mg, 0.488 mmol) were added and refluxed under a nitrogen atmosphere under heating for 4 hours. After allowing to be cooled, the solvent was distilled off under reduced pressure. The obtained residue was purified by silica gel column chromatography (hexane/ethyl acetate = 12/1 - 10/1) to give 2-(1-ethoxyvinyl)-3-nitropyridine (4.58g, 97%).
$^1$H-NMR, ($CDCl_3$) $\delta$ :1.31 (3H,t,J=7.0Hz), 3.91(2H,q,J=7.0Hz), 4.55(1H,d, J=2.6Hz), 5.10(1H,d,J=2.6Hz), 7.38-7.43(1H, m), 7.96-8.00(1H,m), 8.71-8.73 (1H,m)

Reference example 20

1-(3-Nitropyridin-2-yl)ethanone

To an acetic acid (46mL) solution of 2-(1-ethoxyvinyl)-3-nitropyridine (4.58g, 23.6 mmol), 3N hydrochloric acid (34mL) was added and stirred at 20-25°C for 9 hours. The reaction liquid was neutralized with an aqueous sodium hydroxide solution, made weak basic (pH 8-9) with an aqueous sodium bicarbonate solution and extracted with ethyl acetate. The separated organic layer was washed with saturated aqueous sodium chloride and dried over anhydrous sodium sulfate. The solvent was distilled off under reduced pressure and the obtained residue was purified by silica gel column chromatography (hexane/ethyl acetate = 2/1) to give 1-(3-nitropyridin-2-yl)ethanone (3.08g, 79%).

$^1$H-NMR ($CDCl_3$) δ :2.73 (3H,s), 7.60(1H,dd,J=8.3, 4.8Hz), 8.23(1H,dd,J=8.3, 1.5Hz), 8.83(1H,dd,J=4.8, 1.5Hz)

Reference example 21

1-(3-Aminopyridin-2-yl)ethanone

To an ethanol (40mL) solution of 1-(3-nitropyridin-2-yl)ethanone (3.08g, 18.6 mmol), 5% palladium-barium sulfate (1g) was added and stirred under a hydrogen atmosphere of 0.3MPa at 20-25°C for 5 hours. After the reaction, the reaction liquid was subjected to Celite filtration and the filtrate was distilled under reduced pressure. The concentrated residue was purified by silica gel column chromatography (hexane/ethyl acetate = 2/1) to give 1-(3-aminopyridin-2-yl) ethanone (2.40g, 95%).
$^1$H-NMR ($CDCl_3$) δ :2.71 (3H,s), 6.14(2H,br.s), 6.97(1H,dd,J=8.4, 1.5Hz), 7.20 (1H,dd,J=8. 4.1Hz), 8.01(1H,dd,J=4.1, 1.5Hz)

Reference example 22

2-(3-Aminopyridin-2-yl)propan-2-ol

To a 1.2M methyllithium-diethyl ether solution (56mL, 67 mmol), a diethyl ether (60mL) solution of 1-(3-aminopyridin-2-yl)ethanone (2.27g, 16.7 mmol) was added dropwise over 20 minutes under ice-cooling and further stirred at 20-25°C for 2 hours. Water was poured into the reaction liquid under ice-cooling, which was followed by extraction with ethyl acetate and chloroform. The separated organic layer was dried over anhydrous sodium sulfate. The solvent was distilled off under reduced pressure and the obtained residue was purified by silica gel column chromatography (hexane/ethyl acetate = 5/2 - 1/1) to give 2-(3-aminopyridin-2-yl)propan-2-ol (2.52g, 99%).
$^1$H-NMR ($CDCl_3$) δ :1.66 (6H,s), 4.40(2H,br.s), 6.92(1H,dd,J=7.9, 1.5Hz), 7.00 (1H,dd,J=7.9, 4.6Hz), 7.90(1H,dd,J=4.6, 1.5Hz)

Reference example 23

2-(3-Amino-6-bromopyridin-2-yl)propan-2-ol

To an acetonitrile (20mL) solution of 2-(3-aminopyridin-2-yl) propan-2-ol (152mg, 1.00 mmol) cooled to -35°C in a dry ice-acetonitrile bath, N-bromosuccinimide (NBS) (178mg, 1.00 mmol) was added and stirred with allowing to be gradually warmed from -35 to 0°C for one hour. Water (2mL) was added thereto, and then the solvent was distilled off under reduced pressure. The obtained residue was purified by silica gel column chromatography (chloroform/ethyl acetate = 30/1 - 20/1) to give 2-(3-amino-6-bromopyridin-2-yl)propan-2-ol (213mg, 92%).
$^1$H-NMR ($CDCl_3$) δ :1.64 (6H,s), 2.55(1H,br.s), 4.58(2H,br.s), 6.78(1H,d, J=8.3Hz), 7.11(1H,dd,J=8.3Hz)

Reference example 24

6-Bromo-4,4-dimethyl-1,4-dihydro-2H-pyrido[3,2-d] [1,3]oxazin-2-one

6-Bromo-4,4-dimethyl-1,4-dihydro-2H-pyrido[3,2-d][1,3]oxazin-2-one was produced by the same method as Reference example 3 from 2-(3-amino-6-bromopyridin-2-yl)propan-2-ol.
$^1$H-NMR ($CDCl_3$) δ :1.76(6H,s), 7.03(1H,d,J=8.3Hz), 7.35(1H,d,J=8.3Hz), 9.00 (1H,br.s)

Reference example 25

Ethyl 3-aminoisonicotinate

To an ethanol (20mL) solution of 3-aminolsonicotinoic acid (1.4g, 10 mmol), conc. sulfuric acid (2.94g, 30 mmol) was added and refluxed under heating for 20 hours. The reaction liquid was distilled under reduced pressure. Water was added to the obtained residue, which was made its pH to 8-9 with a 2N sodium hydroxide solution under ice-cooling. The precipitated solid was filtered and dried to give ethyl 3-aminoisonicotinate (0.70g, 42%). Further, the filtrate was extracted with ethyl acetate, and the organic layer was washed with water and saturated aqueous sodium chloride, subsequently, and dried over anhydrous magnesium sulfate. The solvent was distilled under reduced pressure to give ethyl 3-aminoisonicotinoate (0.30g, 18%)
$^1$H-NMR (DMSO-$d_6$) δ :1.30(3H,t,J=7.1Hz), 4.28(2H,q,J=7.1Hz), 6.66 (2H,br.s), 7.45(1H,d,J=5.2Hz), 7.73(1H,d,J=5.2Hz), 8.23(1H,s)

Reference example 26

2-(3-Aminopyridin-4-yl)propan-2-ol

2-(3-Aminopyridin-4-yl)propan-2-ol was produced by the same method as Reference example 2 from ethyl 3-aminoisonicotinoate.
$^1$H-NMR (DMSO-$d_6$) δ :1.46(6H,s), 5.38(1H,s), 5.46(2H,br.s), 6.92(1H,d, J=5.1Hz), 7.68(1H,d,J=5.1Hz), 7.90(1H,s)

Reference example 27

2-(5-Amino-2-bromopyridin-4-yl)propan-2-ol

2-(5-Amino-2-bromopyridin-4-yl)propan-2-ol was produced by the same method as Reference example 23 from 2-(3-aminopyridin-4-yl)propan-2-ol.
$^1$H-NMR ($CDCl_3$) δ :1.64(6H,s), 2.05(1H,s), 4.71(2H,br.s), 7.10(1H,s), 7.75(1H,s)

Reference example 28

6-Bromo-4,4-dimethyl-1,4-dihydro-2H-pyrido[3,4-d] [1,3]oxazin-2-one

6-Bromo-4,4-dimethyl-1,4-dihydro-2H-pyrido[3,4-d][1,3]oxazin-2-one was produced by the same method as Reference example 3 from 2-(5-amino-2-bromopyridin-4-yl)propan-2-ol.
$^1$H-NMR ($CDCl_3$) δ :1.74(6H,s), 7.25(1H,s), 8.03(1H,s), 9.27(1H,br.s)

Reference example 29

Allyl 2-aminonicotinoate

To an acetone (84mL) suspension of 2-aminonicotinoic acid (1.40g, 10.1 mmol), potassium carbonate (3.25g, 23.5 mmol) and allyl bromide (1.10mL, 12.7 mmol) were added and refluxed under heating with stirring for 10 hours. An aqueous sodium bicarbonate was poured into the reaction solution, followed by extraction with ethyl acetate. The organic layer was washed with saturated aqueous sodium chloride and dried over anhydrous magnesium sulfate. The solvent was distilled off under reduced pressure to give allyl 2-aminonicotinoate (1.62g, 90%).
$^1$H-NMR ($CDCl_3$) δ :4.79(2H,dt,J=5.7, 1.4Hz), 5.30(1H,ddd,J=10.4, 2.8, 1.4Hz), 5.40(1H,ddd,J=17.2, 2.8, 1.4Hz), 6.02 (1H,ddt, J=17.2, 10.4, 5.7Hz), 6.63 (1H,dd,J=7.8, 4.8Hz), 8.17(1H,dd, J=7.8, 1.9Hz), 8.23(1H,dd,J=4.8, 1.9Hz)

Reference example 30

2-(2-Aminopyridin-3-yl)propan-2-ol

2-(2-Aminopyridin-3-yl)propan-2-ol was produced by the same method as Reference example 2 from allyl 2-aminonicotinoate.
$^1$H-NMR ($CDCl_3$) δ :1.64(6H,s), 5.50(2H,br.s), 6.57(1H,dd,J=7.7, 5.0Hz), 7.32 (1H,dd,J=7.7, 1.7Hz), 7.90(1H,dd,J=5.0, 1.7Hz)

Reference example 31

4,4-Dimethyl-1,4-dihydro-2H-pyrido [2,3-d] [1, 3]oxazin-2-one

To a tetrahydrofuran (50mL) solution of 2-(2-aminopyridin-3-yl) propan-2-ol (1.03g, 6.77 mmol), 1,1'-carbonylbis-1H-imidazole (CDI) (2.33g, 14.4 mmol) was added and refluxed under heating with stirring for 4 hours. After allowing to be cooled, the solvent was distilled off under reduced pressure and the obtained residue was purified by silica gel column chromatography (hexane/ethyl acetate = 1/1 - 1/2) to give 4,4-dimethyl-1,4-dihydro-2H-pyrido[2,3-d][1,3]oxazin-2-one (1.16g, 96%).
$^1$H-NMR ($CDCl_3$) δ :1.73(6H,s), 7.06(1H,dd,J=7.5, 5.0Hz), 7.48(1H,dd,J=7.5, 1.5Hz), 8.38(1H,dd,J=5.0, 1.5Hz), 10.33 (1H,br.s)

Reference example 32

6-Bromo-4,4-dimethyl-1,4-dihydro-2H-pyrido[2,3-d][1,3]oxazin-2-one

To an acetonitrile (50mL) solution of 4,4-dimethyl-1,4-dihydro-2H-pyrido[2,3-d][1,3]oxazin-2-one (1.08g, 6.08 mmol), N-bromosuccinimide (NBS) (1.11g, 6.21 mmol) was added and refluxed under heating with stirring for 4.5 hours. An aqueous sodium bicarbonate solution was poured into the reaction solution, which was followed by extraction with ethyl acetate. The separated organic layer was washed with saturated aqueous sodium chloride and dried over anhydrous magnesium sulfate. The solvent was distilled off under reduced pressure, and the obtained crude product was purified by silica gel column chromatography (hexane/ethyl acetate = 7/1 - 3/1) and washed with acetonitrile to give 6-bromo-4,4-dimethyl-1,4-dihydro-2H-pyrido[2,3-d][1,3] oxazin-2-one (747mg, 48%).
$^1$H-NMR ($CDCl_3$) $\delta$ :1.73(6H,s), 7.57(1H,d,J=2.2Hz), 7.97(1H,br.s), 8.30(1H, d,J=2.2Hz)

Reference example 33

4,4-Dimethyl-2-oxo-1,4-dihydro-2H-pyrido[2,3-d][1,3] oxazine-6-carbaldehyde

4,4-Dimethyl-2-oxo-1,4-dihydro-2H-pyrido[2,3-d] [1,3]oxazine-6-carbaldehyde was produced by the same method as Reference example 8 from 6-bromo-4,4-dimethyl-1,4-dihydro-2H-pyrido[2,3-d][1,3]oxazine-2-one.
$^1$H-NMR ($CDCl_3$) $\delta$ :1.78(6H,s), 8.00(1H,d,J=1.9Hz), 8.18(1H,br.s), 8.83(1H,d, J=1.9Hz), 10.02(1H,s)

Reference example 34

6-(Hydroxymethyl)-4,4-dimethyl-1,4-dihydro-2H-pyrido[2,3-d][1,3]oxazine-2-one

6-(Hydroxymethyl)-4,4-dimethyl-1,4-dihydro-2H-pyrido[2,3-d][1,3] oxazine-2-one was produced by the same method as Reference example 9 from 4,4-dimethyl-2-oxo-1,4-dihydro-2H-pyrido[2,3-d][1,3]oxazine-6-carbaldehyde.
$^1$H-NMR ($CDCl_3$) $\delta$ :1.74(6H,s), 1.80(1H,t,J=5.6Hz), 4.71(1H,d,J=5.6Hz), 7.54 (1H,d,J=1.8Hz), 8.23(1H,d,J=1.8Hz), 8.38 (1H,br.s)

Reference example 35

4,4-Dimethyl-6-nitro-1,4-dihydro-2H-pyrido[2,3-d][1,3]oxazine-2-one

To a conc. sulfuric acid (1.80mL, 33.7 mmol) solution of the compound (320mg, 1.80 mmol) prepared in Reference example 31, fuming nitric acid (2.00mL, 42.9 mmol) was added at 0°C and warmed to and kept at 65°C with stirring for 15 hours. After allowing to be cooled, the reaction liquid was diluted with a large excess of ice at 0°C, which was followed by neutralization with an aqueous sodium hydroxide solution and extraction with ethyl acetate. The separated organic layer was washed with saturated aqueous sodium chloride and dried over anhydrous magnesium sulfate. The solvent was distilled off under reduced pressure, and the obtained crude product was purified by washing with a suspensible solvent (hexane/chloroform = 1/1) to give 4,4-dimethyl-6-nitro-1,4-dihydro-2H-pyrido[2,3-d][1,3]oxazine-2-one (342mg, 85%).
$^1$H-NMR ($CDCl_3$) $\delta$ :1.81(6H,s), 8.28(1H,d,J=2.5Hz), 9.01(1H,br.s), 9.18(1H, d,J=2.5Hz)

Reference example 36

6-Amino-4,4-dimethyl-1,4-dihydro-2H-pyrido[2,3-d] [1,3]oxazine-2-one

To a mixed solution of methanol (5mL)-ethyl acetate (50mL) of 4,4-dimethyl-6-nitro-1,4-dihydro-2H-pyrido[2,3-d][1,3]oxazine-2-one (386mg, 1.73 mmol), 10% palladium-carbon (containing 50% water) (100mg) was added and stirred under a hydrogen atmosphere of 0.25MPa at 20-25°C for one hour. After the catalyst was filtered off, the filtrate was distilled off under reduced pressure to give a residue. The obtained crude product was purified by silica gel column chromatography (hexane/ethyl acetate = 1/5 to ethyl acetate only) to give 6-amino-4,4-dimethyl-1,4-dihydro-2H-pyrido [2,3-d][1,3]oxazin-2-one (280mg, 84%).
$^1$H-NMR ($CDCl_3$) $\delta$ :1.69(6H,s), 3.60(2H,br.s), 6.85(1H,d,J=2.4Hz), 7.83(1H, d,J=2.4Hz), 9.35(1H,br.s)

Reference example 37

(2-Amino-5-nitrophenyl)(phenyl)methanol

To a 2-propanol (60mL) solution of 2-amino-5-nitrobenzophenone (3.00g, 12.4 mmol), sodium borohydride (0.70g, 19 mmol) was added at 20-25°C and refluxed under heating for 30 minutes. An aqueous ammonium chloride solution was poured into the reaction liquid under ice-cooling, and the solvent was distilled off under reduced pressure. An aqueous sodium bicarbonate solution was added to the residue, which was followed by extraction with ethyl acetate. The organic layer was washed with a saturated aqueous sodium chloride solution, and then dried over anhydrous magnesium sulfate. The solvent was distilled off under reduced pressure to give (2-amino-5-nitrophenyl)(phenyl)methanol (3.44g, 100%).
$^1$H-NMR ($CDCl_3$) δ :5.86(1H,s), 6.57-6.60(1H,m), 7.29-7.41(5H,m), 7.99-8.02 (2H,m)

Reference example 38

6-Nitro-4-phenyl-1,4-dihydro-2H-3,1-benzoxazine-2-one

6-Nitro-4-phenyl-1,4-dihydro-2H-3,1-benzoxazine-2-one was produced by the same method as Reference example 3 from (2-amino-5-nitrophenyl)(phenyl)methanol.
$^1$H-NMR (DMSO-$d_6$) δ :6.72(1H,s), 7.14(1H,d,J=9.0Hz), 7.35-7.49(5H,m), 7.84(1H,d,J=2.6Hz), 8.23(1H,dd,J=8.8, 2.6Hz), 11.06(1H,br.s)

Reference example 39

6-Amino-4-phenyl-1,4-dihydro-2H-3,1-benzoxazine-2-one

6-Amino-4-phenyl-1,4-dihydro-2H-3,1-benzoxazine-2-one was produced by the same method as Reference example 15 from 6-nitro-4-phenyl-1,4-dihydro-2H-3,1-benzoxazine-2-one.
$^1$H-NMR (DMSO-$d_6$) δ :4.86(2H,br.s), 6.09(1H,d,J=2.4Hz), 6.32(1H,s), 6.47(1H, dd,J=8.4, 2.4Hz), 6.64(1H,d,J=8.3Hz), 7.30-7.45(5H,m), 9.87(1H,br.s)

Reference example 40

1-(2-Aminophenyl)prop-2-en-1-ol

To a 1M vinylmagnesium bromide-tetrahydrofuran (14mL, 14 mmol) solution, a tetrahydrofuran (10mL) solution of 2-aminobenzaldehyde (484mg, 4.00 mmol) was added dropwise at 20-25°C, and then stirred at 20-25°C for one hour. After the reaction, water was added thereto and the reaction mixture was extracted with ethyl acetate. The organic layer was washed with saturated aqueous sodium chloride, and then dried over anhydrous sodium sulfate. After filtration, the solvent was distilled off under reduced pressure. The obtained residue was purified by silica gel column chromatography (hexane/ethyl acetate = 10/1 - 2/1) to give 1-(2-aminophenyl)prop-2-en-1-ol (351mg, 59%)
$^1$H-NMR ($CDCl_3$) δ :5.20(1H,d,J=5.2Hz), 5.26(1H,dt,J=10.3, 1.3Hz), 5.36(1H, dt,J=17.1, 1.4Hz), 6.17(1H,ddd,J=17.1, 10.3, 5.2Hz), 6.66(1H,d,J=7.9Hz), 6.73 (1H,t-like,J=7.5Hz), 7.08-7.13(2H,m)

Reference example 41

4-Vinyl-1,4-dihydro-2H-3,1-benzoxazin-2-one

4-Vinyl-1,4-dlhydro-2H-3,1-benzoxazin-2-one was produced by the same method as Reference example 3 from 1-(2-aminophenyl) prop-2-en-1-ol.
$^1$H-NMR ($CDCl_3$) δ :5.36(1H,d,J=17.1Hz), 5.42(1H,d,J=10.5Hz), 5.81 (1H,d, J=6.4Hz), 6.03(1H,ddd,J=17.1, 10.5, 6.4Hz), 6.85(1H,d,J=7.7Hz), 7.05-7.12 (2H,m), 7.26-7.31(1H,m), 8.19(1H,br.s)

Reference example 42

6-Nitro-4-vinyl-1,4-dihydro-2H-3,1-benzoxazin-2-one

To an acetic anhydride (14 mL)-acetic acid (7mL) mixed solution of 4-vinyl-1,4-dihydro-2H-3,1-benzoxazin-2-one (380mg, 2.17 mmol), fuming nitric acid (0.152mL, 3.26 mmol) was added at 0°C and warmed to 50°C and kept at the temperature for 9 hours with stirring. After allowing to be cooled, water was added to the reaction liquid to give a dilution, which was extracted with ethyl acetate. The separated organic layer was washed with saturated aqueous sodium chloride, and then dried over anhydrous sodium sulfate. The solvent was distilled off under reduced pressure to give a crude product. The residue was purified by silica gel column chromatography (toluene/ethyl acetate = 20/1 - 5/1) to give 6-nitro-4-vinyl-1,4-dihydro-2H-3,1-benzoxazin-2-one (226mg, 47%).
$^1$H-NMR ($CDCl_3$) δ :5.48(1H,d,J=17.4Hz), 5.56(1H,d,J=10.3Hz), 5.90 (1H,d,J=6.4Hz), 6.09(1H,ddd,J=17.4, 10.3, 6.4Hz), 6.98(1H,d,J=8.8Hz), 8.05 (1H,d,J=2.4Hz), 8.23(1H,dd,J=8.8, 2.4Hz), 8.68(1H,br.m)

Reference example 43

6-Amino-4-vinyl-1,4-dihydro-2H-3,1-benzoxazin-2-one

6-Amino-4-vinyl-1,4-dihydro-2H-3,1-benzoxazin-2-one was produced by the same method as Reference example 15 from 6-nitro-4-vinyl-1,4-dihydro-2H-3,1-benzoxazin-2-one.
$^1$H-NMR (DMSO-$d_6$) δ :5.22(2H,br.m), 5.27-5.32(2H,m), 5.72(1H,d,J=6.4Hz), 5.96(1H,m), 6.35(1H,d,J=2.2Hz), 6.48(1H, dd,J=8.4, 2.2Hz), 6.59(1H,d,J=8.4Hz), 9.79(1H,s)

Reference example 44

2-(2-Aminophenyl)but-3-yn-2-ol

To a 0.5M ethynylmagnesium bromide-tetrahydrofuran solution (25.0mL, 12.5 mmol), a tetrahydrofuran (13mL) solution of 2'-aminoacetophenone (486mg, 3.60 mmol) was added dropwise at 0°C and stirred at 20-25°C for 2.5 hours. After the reaction, water was added and followed by extraction with ethyl acetate. The organic layer was washed with saturated aqueous sodium chloride, and then dried over anhydrous sodium sulfate. After filtration, the solvent was distilled off under reduced pressure. The obtained residue was purified by silica gel column chromatography (hexane/ethyl acetate = 10/1 - 5/1) to give 2-(2-aminophenyl)but-3-yn-2-ol (521mg, 90%).
$^1$H-NMR ($CDCl_3$) δ :1.92(3H,s), 2.71(1H,s), 3.48(1H,br.s), 4.44(2H,br.s), 6.71(1H,dd,J=7.9, 1.3Hz), 6.78(1H,dt,J=1.3, 7.5Hz), 7.13(1H,dt,J=1.6, 7.9Hz), 7.48(1H,dd,J=7.9, 1.6Hz)

Reference example 45

4-Ethynyl-4-methyl-1,4-dihydro-2H-3,1-benzoxazin-2-one

4-Ethynyl-4-methyl-1,4-dihydro-2H-3,1-benzoxazin-2-one was produced by the same method as Reference example 3 from 2-(2-aminophenyl)but-3-yn-2-ol.
$^1$H-NMR ($CDCl_3$) δ :2.02(3H,s), 2.74(1H,s), 6.88(1H,d,J=7.7Hz), 7.12(1H, t-like,J=7.7Hz), 7.30(1H,dd,J=7.7, 1.3Hz), 7.35(1H,d, J=7.7Hz), 8.55(1H,br.s)

Reference example 46

4-Ethynyl-4-methyl-6-nitro-1,4-dihydro-2H-3,1-benzoxazin-2-one

4-Ethynyl-4-methyl-6-nitro-1,4-dihydro-2H-3,1-benzoxazin-2-one was produced by the same method as Reference example 42 from 4-ethynyl-4-methyl-1,4-dihydro-2H-3,1-benzoxazin-2-one.
$^1$H-NMR ($CDCl_3$) δ :2.09(3H,s), 2.85(1H,s), 7.03(1H,d,J=8.6Hz), 8.25(1H, dd,J=8.6, 2.0Hz), 8.29(1H,d,J=2.0Hz), 9.23 (1H,br.s)

Reference example 47

6-Amino-4-ethynyl-4-methyl-1,4-dihydro-2H-3,1-benzoxazin-2-one

6-Amino-4-ethynyl-4-methyl-1,4-dihydro-2H-3,1-benzoxazin-2-one was produced by the same method as Reference example 15 from 4-ethynyl-4-methyl-6-nitro-1,4-dihydro-2H-3,1-benzoxazin-2-one.
$^1$H-NMR (DMSO-$d_6$) δ :1.79(3H,s), 3.87(1H,s), 4.98(2H,br.s), 6.48-6.62(3H,m), 10.01(1H,br.s)

Reference example 48

6-Amino-4-methyl-4-vinyl-1,4-dihydro-2H-3,1-benzoxazin-2-one

6-Amino-4-methyl-4-vinyl-1,4-dihydro-2H-3,1-benzoxazin-2-one was produced by the same methods as Reference example 44, Reference example 3, Reference example 42, and then Reference example 15 from 2'-aminoacetophenone.
$^1$H-NMR (DMSO-$d_6$) δ :1.61(3H,s), 4.87(2H,br.s), 5.01(1H,d,J=17.3Hz), 5.15 (1H,d,J=10.6Hz), 5.95(1H,dd,J=17.0, 10.6Hz), 6.43(1H,d,J=2.2Hz), 6.46(1H,dd, J=8.4, 2.2Hz), 6.57(1H,d,J=8.4Hz), 9.79(1H,s)

Reference example 49

4-Nitrophenyl 4-methylbenzenesulfonate

To an acetone (10mL) solution of p-nitrophenol (696mg, 5.00 mmol), potassium carbonate (1.73g, 12.5 mmol) and p-toluenesulfonyl chloride (1.05g, 5.50 mmol) were added and then stirred at 20-25°C for 2.5 hours. After the reaction, 1N hydrochloric acid was added, and the reaction mixture was neutralized and extracted with ethyl acetate. The organic layer was washed with an aqueous sodium bicarbonate solution and then saturated aqueous sodium chloride, and dried over anhydrous magnesium sulfate. After filtration, the solvent was distilled off under reduced pressure to give 4-nitrophenyl 4-methylbenzenesulfonate (1.47g, 100%).
LC-MS (M+1): 294.1

Reference example 50

4-Aminophenyl 4-methylbenzenesulfonate

4-Aminophenyl 4-methylbenzenesulfonate was produced by the same method as Reference example 36 from 4-nitrophenyl 4-methylbenzenesulfonate.
LC-MS (M+1): 264.2

Reference example 51

6-Bromo-2,2,4-trimethyl-1,2-dihydroquinoline

To an acetone (480mL) solution of 4-bromoaniline (13.8g, 80.0 mmol), scandium trifluoromethanesulfonate (3.94g, 8.00 mmol) was added, stirred at 20-25°C for 10 hours, and refluxed under heating for 60 hours. The reaction liquid was concentrated under reduced pressure. The crude product was purified by silica gel column chromatography (hexane/ethyl acetate = 30/1 - 25/1) to give 6-bromo-2,2,4-trimethyl-1,2-dihydroquinoline (6.61g, 33%).
$^1$H-NMR ($CDCl_3$) δ :1.26(6H,s), 1.95(3H,d,J=1.5Hz), 3.69(1H,br.s), 5.33(1H, d,J=1.1Hz), 6.31(1H,d,J=8.4Hz), 7.04(1H, dd,J=8.4, 2.2Hz), 7.12(1H,d, J=2.2Hz)

Reference example 52

tert-Butyl 6-bromo-2,2,4-trimethylquinoline-1(2H)-carboxylate

A tetrahydrofuran (40mL) solution of 6-bromo-2,2,4-trimethyl-1,2-dihydroquinoline (4.04g, 16.0 mmol) was cooled to -78°C, and 1.56M n-butyllithium-hexane solution (10.9mL, 17.0 mmol) was added thereto and kept -78°C for 30 minutes. After that, the reaction mixture was allowed to be warmed to 0°C and a tetrahydrofuran (5mL) solution of di-tert-butyl dicarbonate ($Boc_2O$) (3.92g, 18.0 mmol) was added dropwise. The reaction solution was warmed to 20-25°C and stirred for 3 hours. An aqueous ammonium chloride solution was added to the reaction solution, which was followed by extraction with ethyl acetate. The separated organic layer was washed with saturated aqueous sodium chloride, and dried over anhydrous magnesium sulfate. The crude product obtained by concentration under reduced pressure was purified by silica gel column chromatography (hexane/diethyl ether = 30/1 - 10/1) to give tert-butyl 6-bromo-2,2,4-trimethylquinoline-1(2H)-carboxylate (5.29g, 94%).
$^1$H-NMR ($CDCl_3$) δ : 1.48(6H,s), 1.51(9H,s), 1.98(3H,d,J=1.5Hz), 5.47(1H, d,J=1.3Hz), 7.05(1H,d,J=7.5Hz), 7.19(1H,d, J=2.4Hz), 7.23(1H,dd,J=7.5, 2.4Hz)

Reference example 53

tert-Butyl 6-formyl-2,2,4-trimethylquinoline-1(2H)-carboxylate

A tetrahydrofuran (15mL) solution of tert-butyl 6-bromo-2,2,4-trimethylquinoline-1(2H)-carboxylate (2.46g, 7.00 mmol) was cooled to -78°C, and 1.4M tert-butyllithium-heptane solution (9.50mL, 13.3 mmol) was added dropwise thereto and stirred at -78°C for 2 hours. N,N-Dimethylformamide (1.36mL, 17.5 mmol) was added dropwise thereto and stirred at the same temperature for one hour, and then warmed to 0°C and stirred for 30 minutes. A saturated aqueous ammonium chloride solution was poured into the reaction liquid and followed by extraction with a mixture of ethyl acetate-toluene. The separated organic layer was washed with water and then saturated aqueous sodium chloride, subsequently, dried over anhydrous sodium sulfate and concentrated under reduced pressure. The obtained crude product was purified by silica gel column chromatography (hexane/ethyl acetate = 20/1 - 8/1) to give tert-butyl 6-formyl-2,2,4-trimethylquinoline-1(2H)-carboxylate (1.95g, 92%).
$^1$H-NMR ($CDCl_3$) δ :1.53(6H,s), 1.55(9H,d,J=0.6Hz), 2.06(3H,d,J=0.9Hz), 5.49(1H,d,J=1.3Hz), 7.24(1H,d,J=8.6Hz), 7.62(1H,dd, J=8.5, 1.7Hz), 7.66(1H, d,J=1.7Hz), 9.87(1H,s)

Reference example 54

tert-Butyl 6-(hydroxymethyl)-2,2,4-trimethylquinoline- 1(2H)-carboxylate

A methanol (8mL) solution of tert-butyl 6-formyl-2,2,4-trimethylquinoline-1(2H)-carboxylate (846mg, 2.81 mmol) was cooled by ice and sodium borohydride (213mg, 5.63 mmol) was added thereto. The reaction mixture was stirred at 0°C for one hour. A saturated aqueous ammonium chloride solution was poured into the reaction liquid and followed by extraction with ethyl acetate. The separated organic layer was washed with saturated aqueous sodium chloride, dried over anhydrous sodium sulfate and concentrated under reduced pressure. The obtained crude product was purified by silica gel column chromatography (hexane/ethyl acetate = 5/1 - 3/1) to give tert-butyl 6-(hydroxymethyl)-2,2,4-trimethylquinoline-1(2H)-carboxylate (736mg, 86%).
$^1$H-NMR ($CDCl_3$) δ :1.49(6H,s), 1.51(9H,s), 1.63(1H,t,J=5.9Hz), 2.02(3H, d,J=1.5Hz), 4.63(2H,d,J=5.7Hz), 5.45(1H,d, J=1.3Hz), 7.11(1H,dd,J=8.3, 1.9Hz), 7.16(1H,d,J=1.9Hz), 7.17(1H,d, J=8.3Hz)

Reference example 55

tert-Butyl 2,2,4-trimethyl-6-{[(4-methylphenyl)sulfonyl]methyl}quinoline-1(2H)-carboxylate

A tetrahydrofuran (10mL) solution of tert-butyl 6-(hydroxymethyl)-2,2,4-trimethylquinoline-1(2H)-carboxylate (626mg, 2.06 mmol) was cooled to -20 to -15°C and triphenylphosphine (812mg, 3.09 mmol) was added thereto. Then, N-bromosuccinimide (NBS) (550mg, 3.09 mmol) was added portionwise over 15 minutes, and further stirred at -10 to 0°C for 30 minutes. A mixture of sodium p-toluenesulfinate (735mg, 4.13 mmol) and tetra- n-butylammonium iodide (76.2mg, 0.206 mmol) was added portionwise over 15 minutes and kept at 50°C with stirring for 4 hours. After cooling, a 3% aqueous sodium thiosulfate solution was poured into the reaction liquid and followed by extraction with ethyl acetate. The separated organic layer was washed with saturated aqueous sodium chloride, dried over anhydrous magnesium sulfate and concentrated under reduced pressure. The obtained crude product was purified by silica gel column chromatography (hexane/ethyl acetate = 10/1 - 5/1) to give tert-butyl 2,2,4-trimethyl-6-{[(4-methylphenyl) sulfonyl]methyl} quinoline-1(2H)-carboxylate (758mg, 83%).
$^1$H-NMR ($CDCl_3$) δ :1.47(6H,s), 1.50(9H,s), 1.85(3H,d,J=1.3Hz), 2.40(3H,s), 4.23(2H,s), 5.42(1H,d,J=1.3Hz), 6.78-6.81 (2H,m), 7.03(1H,d,J=9.0Hz), 7.23(2H, d,J=8.2Hz), 7.52(2H,d,J=8.2Hz)

Reference example 56

tert-Butyl 2,2,4-trimethyl-6-{1-[(4-methylphenyl)sulfonyl]ethyl}quinoline-1(2H)-carboxylate

A tetrahydrofuran (2.0mL) solution of tert-butyl 2,2,4-trimethyl-6-{[(4-methylphenyl)sulfonyl]methyl}quinoline-1 (2H)-carboxylate (112mg, 0.254 mmol) and N,N,N',N'-tetramethylethylenediamine (TMEDA) (38.3 μL, 0.254 mmol) was cooled to -78°C, 2.44M n-butyllithium-hexane solution (0.114mL, 0.279 mmol) was added dropwise, and stirred at the same temperature (-78°C) for one hour. Methyl iodide (17.4 μL, 0.279 mmol) was added dropwise and stirred at the same temperature for 35 minutes. Then, the reaction mixture was warmed to 20-25°C, and stirred for 40 minutes. A saturated aqueous ammonium chloride solution was poured into the reaction liquid and followed by extraction with ethyl acetate. The separated organic layer was washed with saturated aqueous sodium chloride, dried over anhydrous sodium

sulfate and concentrated under reduced pressure. The obtained crude product was purified by silica gel column chromatography (hexane/t-butyl methyl ether = 6/1) to give a part of tert-butyl 2,2,4-trimethyl-6-{1-[(4-methylphenyl)sulfonyl] ethyl}quinoline-1(2H)-carboxylate as a purified product. $^1$H-NMR ($CDCl_3$) $\delta$ :1.45(6H,s), 1.48(9H,s), 1.72(3H,d,J=7.2Hz), 1.82(3H,d,
J=0.92Hz), 2.36(3H,s), 4.14(1H,q,J=7.2Hz), 5.39(1H,d,J=1.3Hz), 6.77(1H,d, J=2.2Hz), 6.84(1H,dd,J=8,6, 2.2Hz), 7.01 (1H,d,J=8.6Hz), 7.16(2H,d,J=8.3Hz), 7.40(2H,d,J=8.3Hz)

Reference example 57

tert-Butyl (2,2,4-trimethyl-1,2-dihydroquinolin-6-yl)carbamate

A mixture of 4-(tert-butoxycarbonylamino)aniline (1.00g, 4.80 mmol), acetone (3.53mL, 48.0 mmol) and scandium trifluoromethanesulfonate (236mg, 0.48 mmol) was stirred in toluene (10mL) at 80°C for 18 hours. The reaction liquid was concentrated under reduced pressure, and the obtained crude product was purified by silica gel column chromatography (hexane/ethyl acetate = 4/1 - 1/1) to give tert-butyl (2,2,4- trimethyl-1,2-dihydroquinolin-6-yl) carbamate (555mg, 40%).
$^1$H-NMR ($CDCl_3$) $\delta$ :1.22(6H,s), 1.47(9H,s), 1.94(3H,s), 5.31(1H,s), 6.23(1H,br.s), 6.37(1H,d,J=8.4Hz), 6.92-7.01(2H,m)

Reference example 58

2,2,4-Trimethyl-1,2-dihydroquinoline-6-amine

To tert-butyl (2,2,4-trimethyl-1,2-dihydroquinolin-6-yl) carbamate (550mg, 1.01 mmol), acetic acid (5mL) and 4N hydrochloric acid-dioxane (2mL) were added and stirred at 20-25°C for one hour. The reaction liquid was poured into aqueous sodium bicarbonate and extracted with ethyl acetate. The organic layer was washed with water and saturated aqueous sodium chloride subsequently, and dried over anhydrous magnesium sulfate. The solvent was distilled off under reduced pressure to give 2,2,4-trimethyl-1,2-dihydroquinoline-6-amine (335mg, 93%).
$^1$H-NMR ($CDCl_3$) $\delta$ :1.24(6H,s), 1.95(3H,s), 5.35(1H,s), 6.35(1H,d,J=7.9Hz), 6.42-6.46(2H,m), 6.53(1H,d,J=2.6Hz)

Reference example 59

N-(2-Chloro-4-nitrophenyl)-4-methylbenzenesulfonamide

To a pyridine (30mL) solution of 2-chloro-4-nitroaniline (1.00g, 5.79 mmol), p-toluenesulfonyl chloride (2.44g, 12.6 mmol) was added and stirred at 20-25°C for 8 hours. The reaction liquid was poured into water and extracted with ethyl acetate. The organic layer was washed with diluted hydrochloric acid and saturated aqueous sodium chloride subsequently, and dried over anhydrous magnesium sulfate. The solvent was distilled off under reduced pressure. To the obtained residue, 1.0M tetrabutylammonium fluoride (TBAF)-tetrahydrofuran solution (11.6ml, 11.6 mmol) was added and stirred at 20-25°C for 2 hours. The reaction liquid was poured into an aqueous sodium bicarbonate and extracted with ethyl acetate. The organic layer was washed with aqueous sodium bicarbonate, water and saturated aqueous sodium chloride subsequently, and dried over anhydrous magnesium sulfate. The solvent was distilled off under reduced pressure, and the obtained residue was purified by silica gel column chromatography (ethyl acetate/hexane = 3/7) to give N-(2-chloro-4-nitrophenyl)-4-methylbenzenesulfonamide (0.90g, 48%).
LC-MS (M+1): 327.2

Reference example 60

N-(4-Amino-2-chlorophenyl)-4-methylbenzenesulfonamide

N-(4-Amino-2-chlorophenyl)-4-methylbenzenesulfonamide was produced by the same method as Reference example 15 from N-(2-chloro-4-nitrophenyl)-4-methylbenzenesulfonamide.
LC-MS (M+1): 297.2

Reference example 61

4-Methyl-N-(2-methyl-2-nitrophenyl)benzenesulfonamide

4-Methyl-N-(2-methyl-2-nitrophenyl)benzenesulfonamide was produced by the same method as Reference

example 59 from 2-methyl-4-nitroaniline.
LC-MS (M+1): 307.3

Reference example 62

N-(4-Amino-2-methylphenyl)-4-methylbenzenesulfonamide

N-(4-Amino-2-methylphenyl)-4-methylbenzenesulfonamide was produced by the same method as Reference example 15 from 4-methyl-N-(2-methyl-2-nitrophenyl)benzenesulfonamide.
LC-MS (M+1): 277.3

Reference example 63

N,4-Dimethyl-N-(2-methyl-4-nitrophenyl)benzenesulfonamide

A mixture of the compound (100mg, 0.33 mmol) prepared in Reference example 61, iodomethane (33 μL, 0.53 mmol) and potassium carbonate (115mg, 0.83 mmol) was stirred in acetone (2.5mL) at 40°C for 9 hours. A precipitate was filtered off and the solvent was distilled off under reduced pressure. The obtained residue was purified by silica gel column chromatography (hexane/ethyl acetate = 7/3) to give N,4-dimethyl-N-(2-methyl-4-nitrophenyl) benzenesulfonamide (105mg, 100%).
LC-MS (M+1): 321.0

Reference example 64

N-(4-Amino-2-methylphenyl)-N,4-dimethylbenzenesulfonamide

N-(4-Amino-2-methylphenyl)-N,4-dimethylbenzenesulfonamide was produced by the same method as Reference example 15 from N,4-dimethyl-N-(2-methyl-4-nitrophenyl)benzenesulfonamide.
LC-MS (M+1): 290.8

Reference example 65

4-Methyl-N-(5-nitropyridin-2-yl)benzenesulfonamide

To a pyridine (50mL) solution of 2-amino-5-nitropyridine (1.00g, 7.19 mmol), p-toluenesulfonyl chloride (1.44g, 7.55 mmol) was added and stirred at 80°C for 2 hours. The reaction liquid was poured into water and extracted with ethyl acetate. The organic layer was washed with diluted hydrochloric acid and saturated aqueous sodium chloride subsequently, and dried over anhydrous magnesium sulfate. The solvent was distilled off under reduced pressure, and the obtained residue was purified by silica gel column chromatography (ethyl acetate/hexane = 3/7) to give 4-methyl-N-(5-nitropyridin-2-yl)benzenesulfonamide (226mg, 10.7%).
LC-MS (M+1): 294.1

Reference example 66

N-(5-Aminopyridin-2-yl)-4-methylbenzenesulfonamide

To a methanol (10mL) solution of 4-methyl-N-(5-nitropyridin-2-yl)benzenesulfonamide (226mg, 0.771 mmol), ammonium formate (48g, 7.71 mmol) and 10% palladium-carbon (containing 50% water) (14mg) were added and stirred at 80°C for 4 hours. The reaction liquid was made to 20-25°C and filtered with Celite. The solvent was distilled off under reduced pressure and the obtained residue was extracted with ethyl acetate. The organic layer was washed with water and saturated aqueous sodium chloride subsequently, and dried over anhydrous magnesium sulfate. The solvent was distilled off under a reduced pressure, and the obtained residue was purified by silica gel column chromatography (ethyl acetate/hexane = 3/7) to give N-(5-aminopyridin-2-yl)-4-methylbenzenesulfonamide (86mg, 42%).
LC-MS (M+1): 263.9

Reference example 67

2,2,4-Trimethyl-1,2,3,4-tetrahydroquinoline-6-amine

A mixture of the compound (1.00g, 3.82mmol) prepared in Reference example 58 and 10% palladium-carbon (containing 50% water) (260mg) was stirred in methanol (5mL) under a hydrogen atmosphere at 20-25°C for one hour. Palladium-carbon was filtered off and the solvent was distilled off under reduced pressure to give 2,2,4-trimethyl-1,2,3,4-tetrahydroquinoline-6-amine dihydrochloride (1.01g, 100%).
LC-MS (M+1, for free form): 191.2

Reference example 68

2-Methylquinolin-6-yl 4-methylbenzenesulfonate

To a tetrahydrofuran (5mL) solution of 6-hydroxy-2-methylquinoline (100mg, 0.629 mmol), triethylamine (263 μL, 1.88 mmol) and p-toluenesulfonyl chloride (132mg, 0.69 mmol) were added and stirred at 20-25°C for 18 hours. The reaction liquid was poured into water and extracted with ethyl acetate. The organic layer was washed with water and saturated aqueous sodium chloride subsequently, and dried over anhydrous magnesium sulfate. The solvent was distilled off under reduced pressure, and the obtained residue was solidified and washed by diethyl ether to give 2-methylquinolin-6-yl 4-methylbenzenesulfonate (53mg, 27%).
$^1$H-NMR ($CDCl_3$) δ : 2.24(3H,s), 2.73(3H,s), 7.20(1H,dd,J=2.6, 9.2Hz), 7.26-7.32 (3H,m), 7.50(1H,m), 7.69-7.73(2H,m), 7.90(1H,d,J=9.2), 7.98(1H,d,J=8.4Hz)

Reference example 69

4-Amino-3-bromophenyl 4-methylbenzenesulfonate

To a tetrahydrofuran (10mL) solution of the compound (528mg, 2.00 mmol) prepared in Reference example 50, N-bromosuccinimide (NBS) (396mg, 2.20 mmol) was added at 0°C and stirred at 20-25°C for 30 minutes. An aqueous 5% sodium thiosulfate solution was added thereto, which was followed by extraction with ethyl acetate. The organic layer was washed with an aqueous 5% potassium carbonate solution and a saturated aqueous sodium chloride solution subsequently, and dried over anhydrous magnesium sulfate. The solvent was distilled off under reduced pressure, and the obtained residue was purified by silica gel column chromatography (hexane/ethyl acetate = 6/1) to give 4-amino-3-bromophenyl 4-methylbenzenesulfonate (603mg, 88%).
LC-MS (M+1, M+3): 342.0, 344.0

Reference example 70

4-Amino-3-(3-methylbut-2-en-1-yl)phenyl 4-methylbenzenesulfonate

To an N,N-dimethylformamide (2.5mL) solution of 4-amino-3-bromophenyl 4-methylbenzenesulfonate (171mg, 0.500 mmol) and tributyl (3-methyl-2-butenyl)tin (186 μL, 0.550 mmol), tetrakis(triphenylphosphine) palladium (29mg, 0.025 mmol) was added and stirred under a nitrogen atmosphere at 150°C for 4 hours. After cooling, 1% aqueous ammonia was added thereto, which was followed by extraction with a mixture of ethyl acetate and toluene. The organic layer was washed with water and saturated aqueous sodium chloride subsequently, and dried over anhydrous sodium sulfate. The solvent was distilled off under reduced pressure. The obtained residue was purified by silica gel column chromatography (hexane/ethyl acetate = 5/1 - 4/1) to give 4-amino-3-(3-methylbut-2-en-1-yl)phenyl 4-methylbenzenesulfonate (59mg, 36%).
$^1$H-NMR ($CDCl_3$) δ :1.65(3H,s), 1.73(3H,s), 2.44(3H,s), 3.07(2H,d,J=7.0Hz), 3.62(2H,br.s), 5.07(1H,m), 6.50(1H,d, J=8.4Hz), 6.60(1H,d,J=2.8Hz), 6.64(1H,dd, J=8.4, 2.8Hz), 7.29(2H,d,J=8.6Hz), 7.69(2H,d,J=8.4Hz)

Reference example 71

3-Iodo-2,2-dimethyl-1,2,3,4-tetrahydroquinolin-6-yl 4-methylbenzenesulfonate

To a dichloromethane (3.2mL) solution of 4-amino-3-(3-methylbut-2-en-1-yl)phenyl 4-methylbenzenesulfonate (53.3mg, 0.161 mmol), sodium carbonate (43mg, 0.40 mmol) and iodine (82mg, 0.32 mmol) were added and stirred under a nitrogen atmosphere at 20-25°C for 45 minutes. After the reaction, a 5% aqueous sodium thiosulfate solution

was poured into the reaction mixture and followed by extraction with ethyl acetate. The organic layer was washed with saturated aqueous sodium chloride and dried over anhydrous sodium sulfate. The solvent was distilled off under reduced pressure to give 3-iodo-2,2-dimethyl-1,2,3,4-tetrahydroquinolin-6-yl 4-methylbenzenesulfonate (70mg, 95%).
$^1$H-NMR ($CDCl_3$) δ :1.33(3H,s), 1.34(3H,s), 2.45(3H,s), 3.30(1H,dd,J=17.4, 8.1Hz), 3.45(1H,dd,J=17.4, 5.3Hz), 3.76 (1H,br.s), 4.32 (1H,dd,J=8.1, 5.3Hz), 6.33 (1H,dd,J=7.2, 1.8Hz), 6.58-6.62(2H, m), 7.28-7.31(2H,m), 7.67.7.71(2H,m)

Reference example 72

4-Chloro-6-{[(4-methylphenyl)sulfbnyl]methyl}-2-(trifluoromethyl)quionoline

A mixture of 6-bromomethyl-4-chloro-2-trifluoromethylquinoline (162mg, 0.50 mmol), sodium p-toluenesulfinate (134mg, 0.75 mmol) and tetrabutylammonium iodide was stirred in tetrahydrofuran (2mL) at 50°C for 2 hours. The reaction liquid was poured into a 3% aqueous sodium thiosulfate solution and extracted with ethyl acetate. The organic layer was dried over anhydrous magnesium sulfate, and the solvent was distilled off under reduced pressure. The obtained residue was washed with a mixed solvent of hexane-ethyl acetate (1:1) to give 4-chloro-6-{[(4-methylphenyl) sulfonyl] methyl}-2-(trifluoromethyl)quionoline (144mg, 72%).
$^1$H-NMR ($CDCl_3$) δ :2.43(3H,s), 4.54(2H,s), 7.26(2H,d,J=8.1Hz), 7.56(2H, d,J=8.3Hz), 7.63(1H,dd,J=8.1, 1.8Hz), 7.83 (1H,s), 7.93(2H,d,J=1.8Hz), 8.16 (2H,d,J=8.6Hz)

Reference example 73

1-Methyl-4-[(4-nitrobenzyl)sulfonyl]benzene

1-Methyl-4-[(4-nitrobenzyl)sulfonyl]benzene was produced by the same method as Reference example 72 from 4-nitrobenzyl bromide.
$^1$H-NMR ($CDCl_3$) δ :2.45(3H,s), 4.38(2H,s), 7.27-7.31(4H,m), 7.54(2H,d, J=8.3Hz), 8.14(2H,d,J=8.8Hz)

Reference example 74

(4-{[(4-Methylphenyl)sulfonyl]methyl}phenyl)amine

(4-{[(4-Methylphenyl)sulfonyl]methyl}phenyl)amine was produced by the same method as Reference example 1 from 1-methyl-4-[(4-nitrobenzyl)sulfonyl]benzene.
$^1$H-NMR ($CDCl_3$) δ :2.42(3H,s), 3.72(2H,br.s), 4.18(2H,s), 6.56(2H,d,J=8.4Hz), 6.86(2H,d,J=8.4Hz), 7.24(2H,d, J=8.4Hz), 7.52(2H,d,J=8.3Hz)

Reference example 75

2-Bromo-1-(bromomethyl)-4-nitrobenzene

To a mixture of 2-bromo-6-nitrobenzene (1.08g, 5.00 mmol) and N-bromosuccinimide (908mg, 5.10 mmol), α, α'-azobisisobutyronitrile (AIBN) (28mg) was added in carbon tetrachloride (15mL) at 70°C and stirred at 70°C for 3 hours. Further, AIBN (82mg) was added portionwise with stirring 70°C for 4 hours. After a dilution with chloroform, precipitate was filtered off and the solvent was distilled off under reduced pressure. The obtained residue was purified by silica gel column chromatography (hexane/ethyl acetate = 1/1) to give 2-bromo-1-(bromomethyl)-4-nitrobenzene (493mg, 33%).
$^1$H-NMR ($CDCl_3$) δ: 4.62(2H,s), 7.65(1H,d,J=8.4Hz), 8.17(1H,dd,J=8.4, 2.2Hz), 8.46(1H,d,J=2.2Hz)

Reference example 76

2-Bromo-1-{[(4-methylphenyl)sulfonyl]methyl}-4-nitrobenzene

2-Bromo-1-{[(4-methylphenyl)sulfonyl]methyl}-4-nitrobenzene was produced by the same method as Reference example 72 from 2-bromo-1-(bromomethyl)-4-nitrobenzene.
$^1$H-NMR ($CDCl_3$) δ :2.45(3H,s), 4.64(2H,s), 7.29(2H,d,J=7.9Hz), 7.56(2H,d, J=8.4Hz), 7.69(1H,d,J=8.6Hz), 8.18(1H,dd, J=8.6, 2.4Hz), 8.35(1H,d,J=2.4Hz)

Reference example 77

(3-Bromo-4-{[(4-methylphenyl)sulfonyl]methyl}phenyl)amine

(3-Bromo-4-{[(4-methylphenyl)sulfonyl]methyl}phenyl)amine was produced by the same method as Reference example 15 from 2-bromo-1-{[(4-methylphenyl)sulfonyl]methyl}-4-nitrobenzene.
LC-MS (M+1): 340.1

Reference example 78

(4-{[(4-Methylphenyl)sulfonyl]methyl}-3-vinylphenyl)amine

To a toluene (6.0mL) solution of (3-bromo-4-{[(4-methylphenyl) sulfonyl]methyl}phenyl)amine (442mg, 1.30 mmol) and tributylvinyltin (454mg, 1.43 mmol), triphenylphosphine (51mg, 0.20 mmol) and tetrakis (triphenylphosphine) palladium (75mg, 0.065 mmol) were added and refluxed under heating under a nitrogen atmosphere for 9 hours. After cooling, the residue concentrated under reduced pressure was purified by silica gel column chromatography (hexane/ ethyl acetate = 5/1 - 2/1) to give (4-{[(4-methylphenyl)sulfonyl]methyl}-3-vinylphenyl)amine (193mg, 52%).
$^1$H-NMR ($CDCl_3$) δ :2.40(3H,s), 3.80(2H,br.s), 4.29(2H,s), 5.08(1H,dd,J=11.0, 1.3Hz), 5.36(1H,dd,J=17.2, 1.3Hz), 6.50 (1H,dd,J=8.3, 2.5Hz), 6.60(1H,dd, J=17.2, 11.0Hz), 6.70(1H,d,J=2.5Hz), 6.85(1H,d,J=8.3Hz), 7.22(2H,d,J=8.1Hz), 7.51 (2H,d,J=8.3Hz)

Example 1

4,4-Dimethyl-2-oxo-1,4-dihydro-2H-3,1-benzoxazin-6-yl 4-methylbenzenesulfonate

To a tetrahydrofuran (4.7mL) solution of the compound (90mg, 0.47 mmol) prepared in Reference example 4, triethylamine (194 μL, 1.40 mmol) and p-toluenesulfonyl chloride (98mg, 0.51 mmol) were added and stirred at 20-25°C for 3.5 hours. After the reaction, water was added thereto, which was followed by extraction with ethyl acetate. The organic layer was washed with aqueous ammonium chloride and saturated aqueous sodium chloride subsequently, and dried over anhydrous magnesium sulfate. After filtration, the solvent was distilled off under reduced pressure. The obtained residue was recrystallized from a mixture of chloroform and hexane to give 4,4-dimethyl-2-oxo-1,4-dihydro-2H-3,1-benzoxazin-6-yl 4-methylbenzenesulfonate (153mg, 95%).
$^1$H-NMR ($CDCl_3$) δ :1.60(6H,s), 2.46(3H,s), 6.72(1H,d,J=2.5Hz), 6.74(1H, d,J=8.7Hz), 6.82(1H,dd,J=8.7, 2.5Hz), 7.33 (2H,d, J=8.5Hz), 7.69(2H,d,J=8.5Hz), 8.91(1H,s)

Example 2

4,4-Dimethyl-2-thioxo-1,4-dihydro-2H-3,1-benzoxazin-6-yl 4-methylbenzenesulfonate

[Chemical Formula 20]

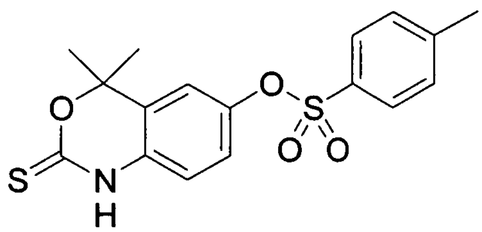

A mixture of the compound (70mg, 0.20 mmol) prepared in Example 1 and Lawesson's reagent (97mg, 0.24 mmol) was refluxed in toluene (1.0mL) under heating for 3 hours. The solvent was distilled off under reduced pressure and the obtained residue was purified by thin layer chromatography (hexane/ethyl acetate = 1/1) to give 4,4-dimethyl-2-thioxo-1,4-dihydro-2H-3,1-benzoxazin-6-yl 4-methylbenzenesulfonate (34mg, 47%).
1H-NMR ($CDCl_3$) δ :1.64(6H,s), 2.46(3H,s), 6.73-6.77(2H,m), 6.89(1H,dd,J=8.7, 2.5Hz), 7.34(2H,d,J=8.1Hz), 7.69(2H,d, J=8.4Hz), 9.32 (1H,s)

Example 3

2-Oxo-1,4-dihydro-2H-3,1-benzoxazin-6-yl 4-methylbenzenesulfonate

It was produced by the same method as Reference examples 3-4 and Example 1 from the compound prepared in Reference example 5.
$^1$H-NMR ($CDCl_3$) δ :2.47(3H,s), 5.26(2H,s), 6.72(1H,d,J=8.6Hz), 6.77(1H, dd,J=8.6, 2.0Hz), 6.90(1H,d,J=2.0Hz), 7.33 (2H,d, J=8.3Hz), 7.70(2H,d,J=8.3Hz), 8.51(1H,s)

Example 4

4,4-Dimethyl-2-oxo-1,4-dihydro-2H-3,1-benzoxazin-6-yl benzenesulfonate

It was produced by the same method as Example 1 from the compound prepared in Reference example 4.
$^1$H-NMR ($CDCl_3$) δ :1.59(6H,s), 6.68-6.86(3H,m), 7.52-7.57(2H,m), 7.67-7.72 (1H,m), 7.81-7.83(2H,m), 9.11(1H,s)

Example 5

4,4-Dimethyl-2-oxo-1,4-dihydro-2H-3,1-benzoxazin-6-yl 3-methylbenzenesulfonate

It was produced by the same method as Example 1 from the compound prepared in Reference example 4.
$^1$H-NMR ($CDCl_3$) δ :1.60-1.61(6H,m), 2.43(3H,s), 6.70-6.88(3H,m), 7.37-7.65 (4H,m), 9.00(1H,s)

Example 6

4,4-Dimethyl-2-oxo-1,4-dihydro-2H-3,1-benzoxazin-6-yl 2-methylbenzenesulfonate

It was produced by the same method as Example 1 from the compound prepared in Reference example 4.
$^1$H-NMR ($CDCl_3$) δ :1.58(6H,s), 2.76(3H,s), 6.68-6.87(3H,m), 7.26-7.34(1H,m), 7.41-7.43(1H,m), 7.53-7.58(1H,m), 7.76-7.80(1H,m), 8.99(1H,s)

Example 7

4,4-Dimethyl-2-oxo-1,4-dihydro-2H-3,1-benzoxazin-6-yl 2-fluorobenzenesulfonate

It was produced by the same method as Example 1 from the compound prepared in Reference example 4.
$^1$H-NMR ($CDCl_3$) δ :1.63(6H,s), 6.75-6.78(1H,m), 6.89-6.90(1H,m), 6.96-7.00(1H,m), 7.26-7.33(2H,m), 7.67-7.82(2H, m), 8.88(1H,s)

Example 8

4,4-Dimethyl-2-oxo-1,4-dihydro-2H-3,1-benzoxazin-6-yl 4-fluorobenzenesulfonate

It was produced by the same method as Example 1 from the compound prepared in Reference example 4.
$^1$H-NMR ($CDCl_3$) δ :1.63(6H,s), 6.75-6.85(3H,m), 7.19-7.26(3H,m), 7.83-7.87 (2H,m), 8.91(1H,s)

Example 9

2-Oxo-1,4-dihydro-2H-3,1-benzoxazin-6-yl 4-cyanobenzenesulfonate

It was produced by the same method as Example 1 from the compound prepared in Reference example 4.
$^1$H-NMR ($CDCl_3$) δ :1.65(6H,s), 6.79-6.84(3H,m), 7.85-7.87(2H,m), 7.96-7.99 (2H,m), 9.14(1H,s)

Example 10

2-Oxo-1,4-dihydro-2H-3,1-benzoxazin-6-yl 2-cyanobenzenesulfonate

It was produced by the same method as Example 1 from the compound prepared in Reference example 4. $^1$H-NMR ($CDCl_3$) δ :1.67(6H,s), 6.79-6.81(1H,m), 6.99-7.06(2H,m), 7.77-7.86 (2H,m), 7.93-7.97(1H,m), 8.09-8.14(1H, m), 8.89(1H,s)

Example 11

4,4-Dimethyl-2-oxo-1,4-dihydro-2H-3,1-benzoxazin-6-yl thiophene-2-sulfonate

It was produced by the same method as Example 1 from the compound prepared in Reference example 4. $^1$H-NMR ($CDCl_3$) δ :1.63(6H,s), 6.77-6.80(2H,m), 6.89-6.92(1H,m), 7.11-7.14(1H,m), 7.59-7.60(1H,m), 7.73-7.76(1H, m), 9.13(1H,s)

Example 12

4,4-Dimethyl-2-thioxo-1,4-dihydro-2H-3,1-benzoxazin-6-yl thiophene-2-sulfonate

[Chemical Formula 21]

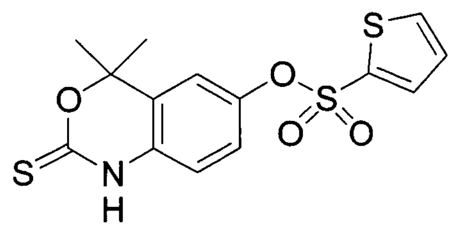

It was produced by the same method as Example 2 from the compound prepared in Example 11.
$^1$H-NMR ($CDCl_3$) δ :1.64(6H,s), 6.75-6.79(2H,m), 6.93-6.97(1H,m), 7.10-7.13 (1H,m), 7.57-7.59(1H,m), 7.73-7.75(1H, m), 9.28(1H, br.s)

Example 13

4,4-Dimethyl-2-oxo-1,4-dihydro-2H-3,1-benzoxazin-6-yl naphthalene-2-sulfonate

It was produced by the same method as Example 1 from the compound prepared in Reference example 4. $^1$H-NMR ($CDCl_3$) δ :1.46(6H,s), 6.63-6.72(2H,m), 6.84-6.88(1H,m), 7.62-7.74 (2H,m), 7.82-8.03(5H,m), 8.33(1H,m), 8.69(1H,s)

Example 14

4,4-Dimethyl-2-thioxo-1,4-dihydro-2H-3,1-benzoxazin-6-yl naphthalene-2-sulfonate

[Chemical Formula 22]

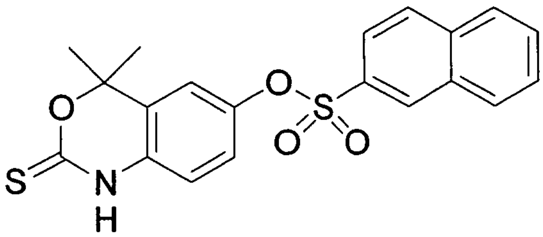

It was produced by the same method as Example 2 from the compound prepared in Example 13.
$^1$H-NMR ($CDCl_3$) δ : 1.49(6H,s), 6.65-6.72(2H,m), 6.88-6.92(1H,m), 7.61-7.73 (2H,m), 7.79-7.83(1H,m), 7.90-8.01(3H,m), 8.31(1H,m), 9.26(1H,br.s)

Example 15

4,4-Dimethyl-6-{[(4-methylphenyl)sulfonyl]methyl}-1,4-dihydro-2H-3,1-benzoxazin-2-one

To an N,N-dimethylformamide (1.5mL) solution of the compound (39.0mg, 0.188 mmol) prepared in Reference example 9 and triphenylphosphine (72.8mg, 0.278 mmol), N-bromosuccinimide (NBS) (49.4mg, 0.278 mmol) was added under ice-cooling and stirred at 20-25°C for 1.5 hours. After that, sodium p-toluenesulfinate (65.9mg, 0.370 mmol) and sodium iodide (2.8mg, 0.019 mmol) were added and stirred at 70°C for 2.5 hours. The reaction liquid was poured into a 10% aqueous sodium thiosulfate solution and extracted with a mixture of ethyl acetate-toluene (1:1) (20mL). The organic layer was dried over anhydrous magnesium sulfate and the solvent was distilled off under reduced pressure. The obtained residue was purified by thin layer chromatography (hexane/ethyl acetate = 1/2) to give 4,4-dimethyl-6-{[(4-methylphenyl)sulfonyl]methyl}-1,4-dihydro-2H-3,1-benzoxazin-2-one (17.1mg, 26%).
$^1$H-NMR ($CDCl_3$) δ :1.59(6H,s), 2.43(3H,s), 4.24(2H,s), 6.72-6.78(2H,m), 7.01(1H,d,J=8.3Hz), 7.27(2H,d,J=8.0Hz), 7.51(1H,d, J=8.2Hz)

Example 16

4,4-Dimethyl-6-{[(4-methylphenyl)sulfonyl]methyl}-1,4-dihydro-2H-3,1-benzoxazin-2-thione

[Chemical Formula 23]

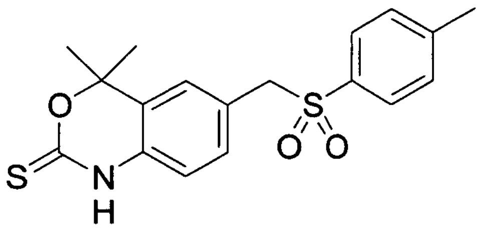

It was produced by the same method as Example 2 from the compound prepared in Example 15.
$^1$H-NMR ($CDCl_3$) δ :1.62(6H,s), 2.43(3H,s), 4.25(2H,s), 6.71-6.76(2H,m), 7.09(1H,dd,J=8.2, 1.9Hz), 7.28(2H,d,J=8.5Hz), 7.54(2H,d, J=8.2Hz), 8.98(1H,s)

Example 17

4,4-Dimethyl-6-[(phenylsulfonyl)methyl]-1,4-dihydro-2H-3,1-benzoxazin-2-one

It was produced by the same method as Example 15 from the compound prepared in Reference example 9. $^{1}$H-NMR ($CDCl_3$) δ :1.58(6H,s), 4.14(2H,s), 6.72-6.76(2H,m), 7.02-7.05(1H,m), 7.46-7.51(2H,m), 7.61-7-66(3H,m)

Example 18

4,4-Dimethyl-6-[(phenylsulfonyl)methyl]-1,4-dihydro-2H-3,1-benzoxazin-2-thione

[Chemical Formula 24]

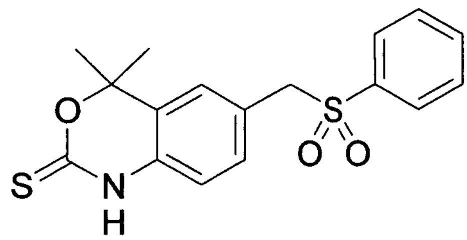

It was produced by the same method as Example 2 from the compound prepared in Example 17.
$^{1}$H-NMR ($CDCl_3$) δ :1.60(6H,s), 4.28(2H,s), 6.72-6.76(2H,m), 7.08-7.12(1H,m), 7.47-7.52(2H,m), 7.62-7.67(3H,m), 8.99 (1H,s)

Example 19

4,4-Dimethyl-5-{[(4-methylphenyl)sulfonyl]methyl}-1,4-dihydro-2H-3,1-benzoxazin-2-one

To a mixture of the compound (300mg, 1.57 mmol) prepared in Reference example 13 and N-bromosuccinimide (279mg, 1.57 mmol), α, α'-azobisisobutyronitrile (AIBN) (28mg) was added in carbon tetrachloride (6mL) at 70°C and stirred at 70°C for 22 hours. After filtering off the precipitate, the solvent was distilled off under reduced pressure. To the obtained residue, sodium p-toluenesulfinate (90mg, 0.51 mmol), tetrabutylammonium iodide (5.5mg, 0.015 mmol) and tetrahydrofuran (3mL) were added and stirred at 50°C for 3 hours. The reaction liquid was poured into a 3% aqueous sodium thiosulfate solution and extracted with ethyl acetate. After drying over anhydrous magnesium sulfate, the solvent was distilled off under reduced pressure. A part (12mg) of the residue was purified by thin layer chromatography (hexane/ethyl acetate = 2/3) to give 4,4-dimethyl-5-{[(4-methylphenyl)sulfonyl]methyl}-1,4-dihydro-2H-3,1-benzoxazin-2-one (4.9mg).
$^{1}$H-NMR ($CDCl_3$) δ :1.91(6H,s), 2.47(3H,s), 4.53(2H,s), 6.85(1H,d,J=7.9Hz), 7.22(1H,t,J=7.9Hz), 7.36(1H,d,J=7.9Hz), 7.59(1H,d,J=8.4Hz), 9.19(1H,s)

Example 20

4,4-Dimethyl-5-{[(4-methylphenyl)sulfonyl]methyl}-1,4-dihydro-2H-3,1-benzoxazin-2-thione

It was produced by the same method as Example 2 from the compound prepared in Example 19.
$^{1}$H-NMR ($CDCl_3$) δ :1.91(6H,s), 2.47(3H,s), 4.53(2H,s), 6.85(1H,d,J=7.9Hz), 7.22(1H,t,J=7.9Hz), 7.36(1H,d,J=7.9Hz), 7.59(1H,d,J=8.4Hz), 9.19(1H,s)

Example 21

N-(4,4-Dimethyl-2-oxo-1,4-dihydro-2H-3,1-benzoxazin-6-yl)-4-methylbenzenesulfonamide

To a tetrahydrofuran (1.3mL) solution of the compound (50mg, 0.26 mmol) prepared in Reference example

18, triethylamine (55 μL, 0.39 mmol) and p-toluenesulfonyl chloride (52mg, 0.28 mmol) were added under cooling at 0°C, stirred at 20-25°C for 13 hours and further refluxed under heating for 1.5 hours. Water was poured into the reaction liquid, which was followed by extraction with ethyl acetate. The separated organic layer was washed with water and saturated aqueous sodium chloride, subsequently, and dried over anhydrous sodium sulfate. The solvent was distilled off under reduced pressure and the obtained residue was purified by silica gel column chromatography (hexane/ethyl acetate = 1/1) to give N-(4,4-dimethyl-2-oxo-1,4-dihydro-2H-3,1-benzoxazin-6-yl)-4-methylbenzenesulfonamide (83mg, 91%).
$^1$H-NMR ($CDCl_3$) δ :1.63(6H,s), 2.39(3H,s), 6.70(1H,d,J=8.3Hz), 6.87(1H,dd, J=8.3, 2.3Hz), 6.93(1H,d,J=2.3Hz), 7.23 (2H,d,J=8.3Hz), 7.60(2H,d,J=8.3Hz), 8.89(1H,br.s)

Example 22

N-(4,4-Dimethyl-2-thioxo-1,4-dihydro-2H-3,1-benzoxazin-6-yl)-4-methylbenzenesulfonamide

[Chemical Formula 25]

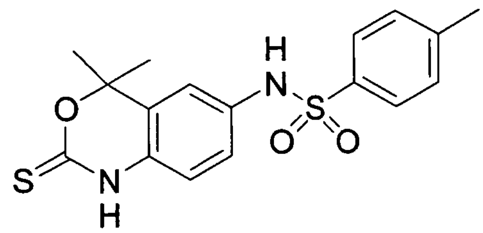

It was produced by the same method as Example 2 from the compound prepared in Example 21.
$^1$H-NMR ($CDCl_3$) δ :1.67(6H,s), 2.39(3H,s), 6.77(1H,d,J=8.4Hz), 6.91(1H,dd, J=8.4, 2.2Hz), 6.95(1H,d,J=2.2Hz), 7.25 (2H,d,J=8.2Hz), 7.36(1H,br.s), 7.63(2H, d,J=8.2Hz), 9.93(1H,br.s)

Example 23

N-(4,4-Dimethyl-2-oxo-1,4-dihydro-2H-3,1-benzoxazin-6-yl)thiophene-2-sulfonamide

[Chemical Formula 26]

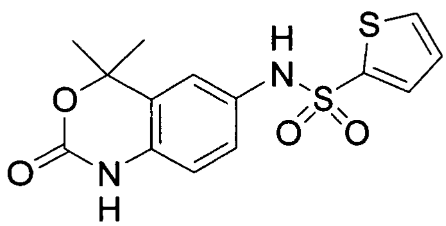

It was produced by the same method as Example 21 from the compound prepared in Reference example 18.
$^1$H-NMR ($CDCl_3$) δ :1.66(6H,s), 6.74(1H,d,J=8.4Hz), 6.94(1H,dd,J=8.4, 2.4Hz), 6.99(1H,d,J=2.4Hz), 7.03(1H,dd,J=5.0, 3.9Hz), 7.05(1H,br.s), 7.44(1H,dd,J=3.9, 1.3Hz), 7.56(1H,dd,J=5.0, 1.3Hz), 8.68(1H,br.s)

Example 24

N-(4,4-Dimethyl-2-thioxo-1,4-dihydro-2H-3,1-benzoxazin-6-yl)thiophene-2-sulfonamide

[Chemical Formula 27]

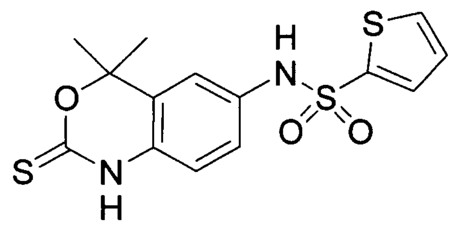

It was produced by the same method as Example 2 from the compound prepared in Example 23.
$^1$H-NMR ($CDCl_3$) δ :1.70(6H,s), 6.75(1H,d,J=2.3Hz), 6.81(1H,br.s), 6.96-7.00 (2H,m), 7.04(1H,dd,J=5.1, 3.8Hz), 7.46 (1H,dd,J=3.8, 1.3Hz), 7.58(1H,dd,J=5.1, 1.3Hz), 9.24(1H,br.s)

Example 25

N-(4,4-Dimethyl-2-oxo-1,4-dihydro-2H-3,1-benzoxazin-6-yl)-N-(2-thienylsulfonyl)acetamide

To a tetrahydrofuran (5.0mL) solution of the compound (100mg, 0.29 mmol) prepared in Example 23, triethylamine (124 μL, 0.89 mmol) and acetyl chloride (32 μL, 0.44 mmol) were added and stirred at 20-25°C for 16 hours. The reaction liquid was poured into water and extracted with ethyl acetate. The organic layer was washed with water and saturated aqueous sodium chloride subsequently, and dried over anhydrous sodium sulfate. The solvent was distilled off under reduced pressure and the obtained residue was purified by washing with diethyl ether to give N-(4,4-Dimethyl-2-oxo-1,4-dihydro-2H-3,1-benzoxazin-6-yl)-N-(2-thienylsulfonyl)acetamide (51.3mg, 46%).
$^1$H-NMR (DMSO-$d_6$) δ :1.59(6H,s), 1.86(3H,s), 6.98(1H,d,J=8.4Hz), 7.22-7.27 (2H,m), 7.84(1H,dd,J=1.3, 3.8Hz), 8.15 (1H,dd,J=1.5, 5.0Hz), 10.49(1H,br.s)

Example 26

N-(4,4-Dimethyl-2-thioxo-1,4-dihydro-2H-3,1-benzoxazin-6-yl)-N-(2-thienylsulfonyl)acetamide

[Chemical Formula 28]

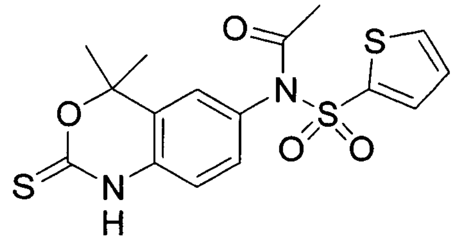

It was produced by the same method as Example 25 from the compound prepared in Example 24.

$^1$H-NMR (DMSO-$d_6$) δ :1.63(6H,s), 1.86(3H,s), 7.11-7.15(1H,m), 7.27(3H,dd, J=4.0, 4.9Hz), 7.60(1H,dd,J=1.3, 3.8Hz), 8.15(1H,dd,J=1.3, 4.9Hz), 12.37 (1H,br.s)

Example 27

N-(4,4-Dimethyl-2-oxo-1,4-dihydro-2H-3,1-benzoxazin-6-yl)thiophene-3-sulfonamide

It was produced by the same method as Example 21 from the compound prepared in Reference example 18.
$^1$H-NMR (DMSO-$d_6$) δ :1.48(6H,s), 6.75(1H,d,J=8.4Hz), 6.86-6.87(1H,m), 6.93(1H,dd,J=8.4, 2.2Hz), 7.18-7.20(1H,m), 7.68-7.71(1H,m), 8.05-8.07(1H,m), 9.94(1H,br.s), 10.15(1H,br.s)

Example 28

N-(4,4-Dimethyl-2-thioxo-1,4-dihydro-2H-3,1-benzoxazin-6-yl)thiophene-3-sulfonamide

[Chemical Formula 29]

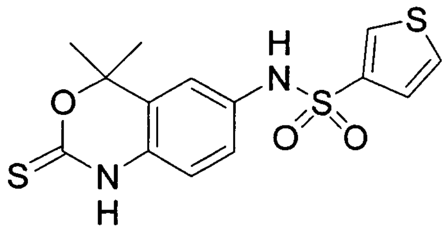

It was produced by the same method as Example 2 from the compound prepared in Example 27.
$^1$H-NMR (DMSO-$d_6$) δ :1.52(6H,s), 6.89-7.01(3H,m), 7.20(1H,dd,J=1.3, 5.1Hz), 7.70(1H,dd,J=3.1, 5.1Hz), 8.11(1H,dd, J=1.3, 3.0Hz), 10.15(1H,br.s), 12.12 (1H,br.s)

Example 29

N-(4,4-Dimethyl-2-thioxo-1,4-dihydro-2H-3,1-benzoxazin-6-yl)-2-fluorobenzenesulfonamide

[Chemical Formula 30]

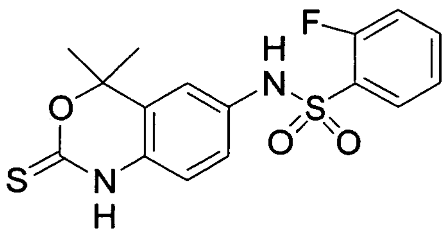

It was produced by the same method as Example 21 and then Example 2 from the compound prepared in Reference example 18.
$^1$H-NMR (DMSO-$d_6$) δ :1.49(6H,s), 6.89-6.92(2H,m), 6.98(1H,dd,J=2.0, 8.4Hz), 7.91(1H,dd-like,J=8.4, 21.6Hz), 10.40 (1H,br.s), 12.13(1H,s)

Example 30

N-(4,4-Dimethyl-2-thioxo-1,4-dihydro-2H-3,1-benzoxazin-6-yl)-4-(trifluoromethyl)benzenesulfonamide

[Chemical Formula 31]

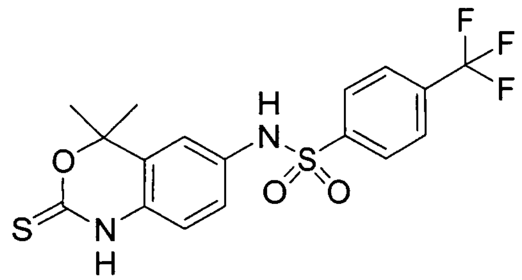

It was produced by the same method as Example 21 and then Example 2 from the compound prepared in Reference example 18.
$^1$H-NMR (DMSO-$d_6$) δ :1.49(6H,s), 6.87-7.00(3H,m), 7.31-7.43(2H,m), 7.64-7.71(1H,m), 7.75-7.81(1H,m), 10.55(1H, br.s), 12.10 (1H,s)

Example 31

N-(4,4-Dimethyl-2-oxo-1,4-dihydro-2H-pyrido[3,2-d] [1,3]oxazin-6-yl)-4-methylbenzenesulfonamide

To a dimethyl sulfoxide (1.5mL) suspension of p-toluenesulfonamide (256mg, 1.50 mmol), copper (I) iodide (286mg, 1.50 mmol) and cesium carbonate (244mg, 0.75 mmol), a dimethyl sulfoxide (1.5mL) solution of the compound (77mg, 0.30 mmol) prepared in Reference example 24 was added dropwise over 100 minutes under heating at 150°C, and further stirred with keeping at the same temperature for 40 minutes. After cooling, the reaction liquid was diluted with water and ethyl acetate, and the filtrate filtered with Celite was extracted with ethyl acetate and chloroform. The solvent was distilled off under reduced pressure and the obtained residue was purified by silica gel column chromatography (chloroform/ethyl acetate = 10/1) to give N-(4,4-dimethyl-2-oxo-1,4-dihydro-2H-pyrido[3,2-d][1,3]oxazin-6-yl)-4-methylbenzenesulfonamide (45.1mg, 43%).
$^1$H-NMR ($CDCl_3$) δ :1.56(6H,s), 2.39(3H,s), 7.10(2H,t-like,J=8.7Hz), 7.25(2H, d,J=8.2Hz), 7.76(2H,d,J=8.2Hz), 9.22(1H, br.s)

Example 32

N-(4,4-Dimethyl-2-thioxo-1,4-dihydro-2H-pyrido[3,2-d] [1,3]oxazin-6-yl)-4-methylbenzenesulfonamide

It was produced by the same method as Example 2 from the compound prepared in Example 31.
$^1$H-NMR ($CDCl_3$) δ :1.62(6H,s), 2.40(3H,s), 7.10-7.29(4H,m), 7.37(1H,br.s), 7.75-7.78(2H,m), 9.59(1H,br.s)

Example 33

N-(4,4-Dimethyl-2-thioxo-1,4-dihydro-2H-pyrido[3,4-d][1,3]oxazin-6-yl)-4-methylbenzenesulfonamide

It was produced by the same methods as Example 31 and then Example 2 from the compound prepared in Reference example 28.
$^1$H-NMR ($CDCl_3$) δ :1.73(6H,s), 2.39(3H,s), 7.23-7.30(4H,m), 7.67-7.70(2H,m), 7.98(H,br.s), 9.42(1H,br.s)

Example 34

N-(4,4-Dimethyl-2-thioxo-1,4-dihydro-2H-pyrido[3,4-d][1,3]oxazin-6-yl) thiophene-2-sulfonamide

It was produced by the same methods as Example 31 and then Example 2 from the compound prepared in Reference example 28.
$^1$H-NMR (DMSO-$d_6$) δ :1.59(6H,s), 7.00(1H,s), 7.10-7.13(1H,m), 7.67-7.71 (1H,m), 7.88-7.90(2H,m), 11.34(1H,br.s) 12.33(1H, br.s)

Example 35

4,4-Dimethyl-6-{[(4-methylphenyl)sulfonyl]methyl}-1,4-dihydro-2H-pyrido [2,3-d][1,3]oxazin-2-one

To a dichloromethane (5mL) suspension of the compound (83.1mg, 399 μ mol) prepared in Reference example 34, thionyl chloride (50 μL, 690 μ mol) was added dropwise at 0°C and stirred at 20-25°C for 4 hours. The reaction solution was concentrated under reduced pressure and further co-distilled with toluene. After concentration, tetrahydrofuran (5mL) was added thereto, and further tetrabutylammonium iodide (7.5mg, 23 μmol) and sodium p-toluenesulfinate (95.8mg, 538 μmol) were added and stirred at 50°C for 7 hours. The reaction solution was poured into aqueous sodium bicarbonate and extracted with ethyl acetate. The separated organic layer was washed with saturated aqueous sodium chloride and dried over anhydrous sodium sulfate. The obtained crude product obtained by concentrating under reduced pressure was purified by silica gel column chromatography (hexane/ethyl acetate = 1/1 - 1/5) to give 4,4-dimethyl-6-{[(4-methylphenyl)sulfonyl]methyl}-1,4-dihydro-2H-pyrido[2,3-d][1,3]oxazin-2-one (139mg, 99%).
$^1$H-NMR ($CDCl_3$) δ :1.67(6H,s), 2.45(3H,s), 4.25(2H,s), 7.28(1H,d,J=2.0Hz), 7.32(2H,d,J=8.1Hz), 7.89(1H,d,J=2.0Hz), 8.51(1H,br.s)

Example 36

4,4-Dimethyl-6-{[(4-methylphenyl)sulfonyl]methyl}-1,4-dihydro-2H-pyrido[2,3-d][1,3]oxazin-2-thione

[Chemical Formula 32]

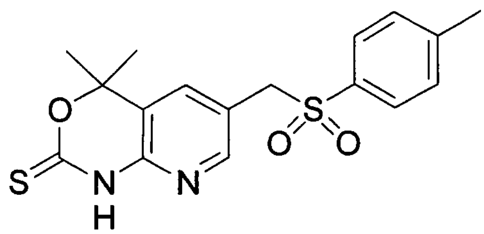

It was produced by the same method as Example 2 from the compound prepared in Example 35.
$^1$H-NMR ($CDCl_3$) δ :1.72(6H,s), 2.45(3H,s), 4.26(2H,s), 7.32(3H,m), 7.57(2H,d, J=8.3Hz), 7.99(1H,d,J=2.0Hz), 10.00 (1H,br.s)

Example 37

4,4-Dimethyl-6-[(2-thienylsulfonyl)methyl]-1,4-dihydro-2H-pyrido[2,3-d][1,3] oxazin-2-thione

[Chemical Formula 33]

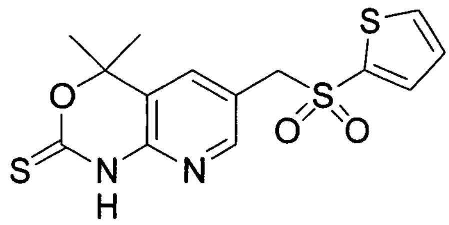

It was produced by the same methods as Example 35 and then Example 2 from the compound prepared in Reference example 34.
$^1$H-NMR ($CDCl_3$) δ :1.75(6H,s), 4.40(2H,s), 7.15(1H,t-like,J=4.1Hz), 7.44(1H,d, J=1.7Hz), 7.50(1H,d,J=3.7Hz), 7.74(1H, d,J=5.0Hz), 8.15(1H,d,J=1.7Hz), 10.70(1H,br.s)

Example 38

4,4-Dimethyl-6-[(pyridine-3-ylsulfonyl)methyl]-1,4-dihydro-2H-pyrido[2,3-d] [1,3]oxazin-2-thione

[Chemical Formula 34]

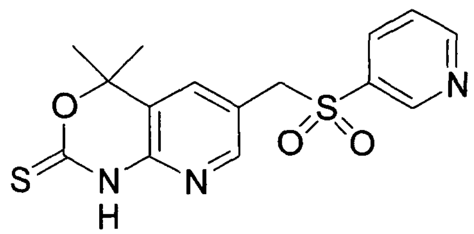

It was produced by the same methods as Example 35 and then Example 2 from the compound prepared in Reference example 34.
$^1$H-NMR ($CDCl_3$) δ :1.76(6H,s), 4.35(2H,s), 7.47(1H,d,J=2.0Hz), 7.52(1H,m), 8.01(1H,m), 8.14(1H,d,J=2.0Hz), 8.90(2H, m), 10.82(1H,br.s)

Example 39

N-(4,4-Dimethyl-2-oxo-1,4-dihydro-2H-pyrido[2,3-d][1,3]oxazin-6-yl)-N-(2-thienylsulfonyl)thiophene-2-sulfonamide

To a tetrahydrofuran (5mL) solution of the compound (150mg, 0.78 mmol) prepared in Reference example 36, triethylamine (163 μL, 1.16 mmol), 2-thiophenesulfonyl chloride (143mg, 0.78 mmol) and 4-dimethylaminopyridine (DMAP) (9.5mg, 0.078 mmol) were added and stirred at 50°C for 9 hours. The reaction liquid was poured into water and extracted with ethyl acetate. The organic layer was washed with water and saturated aqueous sodium chloride subsequently, and then dried over anhydrous magnesium sulfate. The solvent was distilled off under reduced pressure and the residue was purified by washing with diethyl ether to give N-(4,4-dimethyl-2-oxo-1,4-dihydro-2H-pyrido[2,3-d] [1,3] oxazin-6-yl)-N-(2-thienylsulfonyl)thiophene-2-sulfonamide (185mg, 49%).
$^1$H-NMR (DMSO-$d_6$) δ :1.54(6H,s), 7.26(1H,d,J=2.4Hz), 7.31(2H,dd,J=3.8, 5.0 Hz), 7.78(2H,dd,J=1.5, 3.8Hz), 7.86(1H, d,J=2.4Hz), 8.25(2H,dd,J=1.5, 5.0Hz)

Example 40

N-(4,4-Dimethyl-2-oxo-1,4-dihydro-2H-pyrido[2,3-d][1,3]oxazin-6-yl)thiophene-2-sulfonamide

To a tetrahydrofuran (5mL) solution of the compound (180mg, 0.78 mmol) prepared in Example 39, 1.0M tetrabutylammonium fluoride (TBAF)-tetrahydrofuran solution (1.4mL) was added and stirred at 20-25°C for 15 hours. The reaction liquid was poured into water and extracted with ethyl acetate. The organic layer was washed with water and saturated aqueous sodium chloride subsequently, and then dried over anhydrous magnesium sulfate. The solvent was distilled off under reduced pressure and the residue was purified by silica gel chromatography (ethyl acetate/hexane = 6/4) to give N-(4,4-dimethyl-2-oxo-1,4-dihydro-2H-pyrido[2,3-d][1,3]oxazin-6-yl)thiophene-2-sulfonamide (78mg, 30%).
$^1$H-NMR (DMSO-$d_6$) δ :1.53(6H,s), 7.12-7.15(1H,m), 7.33-7.34(1H,m), 7.47-7.49(1H,m), 7.81-7.83(1H,m) 7.91-7.93(1H, m)

Example 41

N-(4,4-Dimethyl-2-thioxo-1,4-dihydro-2H-pyrido[2,3-d][1,3]oxazin-6-yl) thiophene-2-sulfonamide

[Chemical Formula 35]

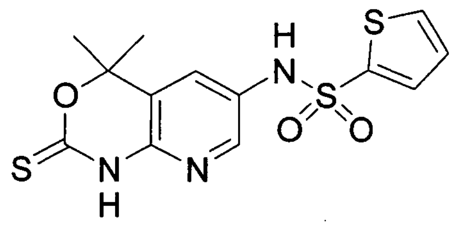

It was produced by the same method as Example 2 from the compound prepared in Example 40.
$^1$H-NMR (DMSO-$d_6$) δ :1.58(6H,s), 7.14(1H,dd,J=3.8, 5.0Hz), 7.42(1H,d, J=2.4Hz), 7.52(1H,dd,J=3.8, 1.4Hz), 7.91(1H, d,J=2.4Hz), 7.93(1H,dd,J=1.4, 5.0Hz), 10.52(1H,s), 12.63(1H,s)

Example 42

N-(4,4-Dimethyl-2-thioxo-1,4-dihydro-2H-pyrido[2,3-d][1,3]oxazin-6-yl)-4-methylbenzenesulfonamide

It was produced by the same methods as Examples 39-40 and then Example 2 from the compound prepared in Reference example 36.
$^1$H-NMR (DMSO-$d_6$) δ :1.56(6H,s), 2.33(3H,s), 7.36(3H,m), 7.58(2H,m), 7.83(1H,d,J=2.4), 10.30(1H,br.s), 12.58(1H, br.s)

Example 43

N-(2-Oxo-4-phenyl-1,4-dihydro-2H-3,1-benzoxazin-6-yl)thiophene-2-sulfonamide

The compound (150mg, 0.625 mmol) prepared in Reference example 39 was dissolved in acetonitrile (12.5mL) under heating. After it was allowed to be cooled, 2,4,6-collidine (123 μL, 0.94 mmol) and 2-thiophenesulfonyl chloride (120mg, 0.66 mmol) were added and stirred at 20-25°C for one hour and 50°C for 5 hours. The solvent was distilled off under reduced pressure. Water was added to the obtained residue, which was followed by extraction with ethyl acetate. The organic layer was washed with diluted hydrochloric acid and saturated aqueous sodium chloride subsequently, and then dried over anhydrous magnesium sulfate. The solvent was distilled off under reduced pressure and the obtained residue was purified by silica gel column chromatography (hexane/ethyl acetate = 7/3) to give N-(2-oxo-4-phenyl-1,4-dihydro-2H-3,1-benzoxazin-6-yl)thiophene-2-sulfonamide.
$^1$H-NMR (DMSO-$d_6$) δ :6.47(1H,s), 6.64-6.65(1H,d,J=2.2Hz), 6.80(1H,d, J=8.6Hz), 7.00-7.09(2H,m), 7.16-7.20(2H,m),

7.32-7.34(1H,m), 7.38-7.45(3H,m), 7.86-7.89(1H,m), 10.15(1H,br.s), 10.30(1H,s)

Example 44

N-(4-Phenyl-2-thioxo-1,4-dihydro-2H-3,1-benzoxazin-6-yl)thiophene-2-sulfonamide

[Chemical Formula 36]

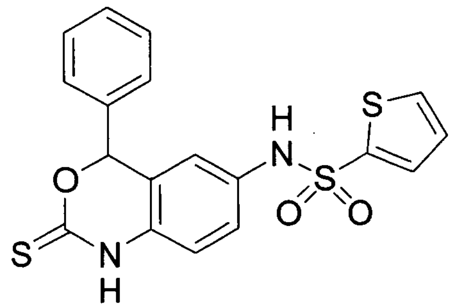

A mixture of the compound (50mg, 0.13 mmol) prepared in Example 43 and Lawesson's reagent (63mg, 0.16 mmol) was stirred in a mixed solvent of toluene (2.0mL) and tetrahydrofuran (2.0mL) at 50°C for 5 hours and further 80°C for 5 hours under heating. The solvent was distilled off under reduced pressure and the obtained residue was purified by thin layer chromatography (hexane/ethyl acetate = 1/1) to give N-(4-phenyl-2-thioxo-1,4-dihydro-2H-3,1-benzoxazin-6-yl)thiophene-2-sulfonamide (28mg, 54%).
$^1$H-NMR (DMSO-$d_6$) δ :6.60(1H,s), 6.70(1H,d,J=2.0Hz), 6.99(1H,d,J=8.6Hz), 7.09(2H,m), 7.19-7.22(1H,m), 7.36(1H, dd,J=3.8, 1.3Hz), 7.42-7.46(3H,m), 7.89 (1H,dd,J=5.0, 1.3Hz), 10.33(1H,br.s), 12.27(1H,s)

Example 45

N-(2-Oxo-4-vinyl-1,4-dihydro-2H-3,1-benzoxazin-6-yl)thiophene-2-sulfonamide

It was produced by the same method as Example 43 from the compound prepared in Reference example 43.
$^1$H-NMR (DMSO-$d_6$) δ :5.28(1H,d,J=17.0Hz), 5.40(1H,d,J=10.3Hz), 5.75(1H, d,J=6.2Hz), 5.93-6.04(1H,m), 6.77(1H,d, J=8.4Hz), 6.96-7.03(3H,m), 7.45(1H, dd,J=1.3, 3.8Hz), 7.55(1H,dd,J=1.3, 5.1Hz), 8.77(1H,br.s)

Example 46

N-(2-Thioxo-4-vinyl-1,4-dihydro-2H-3,1-benzoxazin-6-yl)thiophene-2-sulfonamide

[Chemical Formula 37]

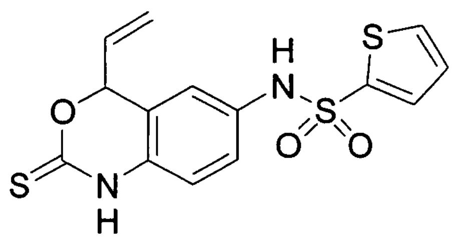

It was produced by the same method as Example 2 from the compound prepared in Example 45.
$^1$H-NMR (DMSO-$d_6$) δ :5.23(1H,d,J=16.8Hz), 5.38(1H,d,J=10.1Hz), 5.86-6.01(2H,m), 5.93-6.04(1H,m), 6.86-7.11(4H, m), 7.47-7.49(1H,m), 7.88-7.90(1H,m), 10.40(1H,br.s), 12.19(1H,s)

Example 47

N-(4-Ethynyl-4-methyl-2-oxo-1,4-dihydro-2H-3,1-benzoxazin-6-yl)thiophene-2-sulfonamide

It was produced by the same method as Example 43 from the compound prepared in Reference example 47.
$^1$H-NMR (DMSO-$d_6$) δ :1.76(3H,s), 3.96(1H,s), 6.83(1H,d,J=8.3Hz), 7.02-7.12(3H,m), 7.46-7.47(1H,m), 7.88-7.90(1H, m), 10.28(1H,br.s), 10.49(1H,s)

Example 48

N-(4-Ethynyl-4-methyl-2-thioxo-1,4-dihydro-2H-3,1-benzoxazin-6-yl) thiophene-2-sulfonamide

[Chemical Formula 38]

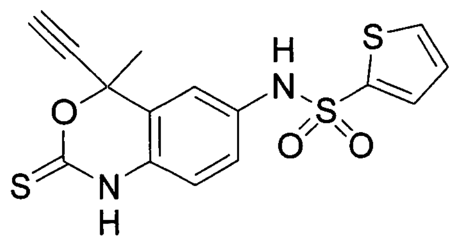

A mixture of the compound (100mg, 0.287 mmol) prepared in Example 47 and Lawesson's reagent (139mg, 0.344 mmol) was refluxed in tetrahydrofuran (2.0mL) at 80°C for 14 hours under heating. The solvent was distilled off under reduced pressure and the obtained residue was purified by thin layer chromatography (hexane/ethyl acetate = 1/1) to give N-(4-ethynyl-4-methyl-2-thioxo-1,4-dihydro-2H-3,1-benzoxazin-6-yl)thiophene-2-sulfonamide (28mg, 26%).
$^1$H-NMR (DMSO-$d_6$) δ :1.81(3H,s), 4.07(1H,s), 6.97-7.00(1H,m), 7.09-7.13(3H, m), 7.49-7.51(1H,m), 7.90(1H,dd,J=5.0, 1.3Hz), 10.46(1H,br.s), 12.46(1H,br.s)

Example 49

N-(4-Methyl-2-oxo-4-vinyl-1,4-dihydro-2H-3,1-benzoxazin-6-yl)thiophene-2-sulfonamide

[Chemical Formula 39]

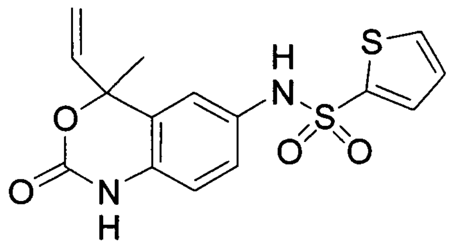

It was produced by the same method as Example 43 from the compound prepared in Reference example 48.
$^1$H-NMR (DMSO-$d_6$) δ :1.59(3H,s), 4.86(1H,d,J=17.0Hz), 5.16(1H,d,J=10.6Hz), 5.92(1H,dd,J=17.0, 10.7Hz), 6.78(1H, d,J=8.4Hz), 6.89(1H,d,J=2.4Hz), 6.99(1H,dd,J=8.4, 2.4Hz), 7.10(1H,dd,J=5.0, 3.9Hz), 7.44(1H,dd,J=3.9, 1.3Hz), 7.88 (1H,dd,J=5.0, 1.3Hz), 10.18(1H,br.s), 10.26(1H,s)

Example 50

N-(4-Methyl-2-thioxo-4-vinyl-1,4-dihydro-2H-3,1-benzoxazin-6-yl)thiophene-2-sulfonamide

[Chemical Formula 40]

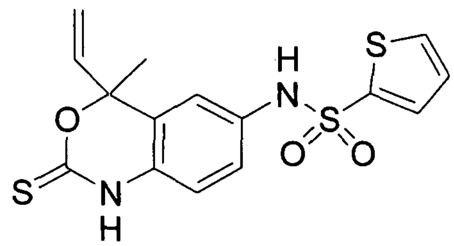

It was produced by the same method as Example 48 from the compound prepared in Example 49.
$^1$H-NMR (DMSO-$d_6$) δ :1.66(3H,s), 4.90(1H,d,J=17.2Hz), 5.22(1H,d,J=10.4Hz), 5.91(1H,dd,J=17.0, 10.4Hz), 6.92-7.12 (4H,m), 7.46-7.48(1H,m), 7.88-7.89 (1H,m), 10.37(1H,br.s), 12.22(1H,br.s)

Example 51

2, 2,4.Trimethyl-1,2-dihydroquinolin-6-yl 4-methylbenzenesulfonate

[Chemical Formula 41]

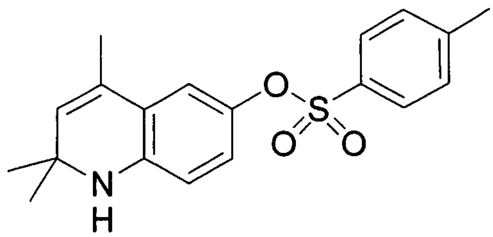

To an acetone (6.0mL) solution of the compound (263mg, 1.00 mmol) prepared in Reference example 50, scandium trifluoromethanesulfonate (49mg, 0.10 mmol) was added, and stirred in a sealed tube at 120°C for 29 hours under heating. The reaction liquid was concentrated under reduced pressure and the obtained crude product was purified by silica gel column chromatography (hexane/ethyl acetate = 10/1 - 5/1) to give 2,2,4-trimethyl-1,2-dihydroquinolin-6-yl 4-methylbenzenesulfonate (113mg, 33%).
$^1$H-NMR ($CDCl_3$) δ :1.25(6H,s), 1.79(3H,d,J=1.3Hz), 2.44(3H,s), 3.69(1H,br.s), 5.30(1H,s), 6.24-6.27(1H,m), 6.53-6.56 (2H,m), 7.30(2H,d,J=8.3Hz), 7.71(2H, d,J=8.3Hz)

Example 52

2,2,4-Trimethyl-1,2-dihydroquinolin-6-yl naphthalene-2-sulfonate

[Chemical Formula 42]

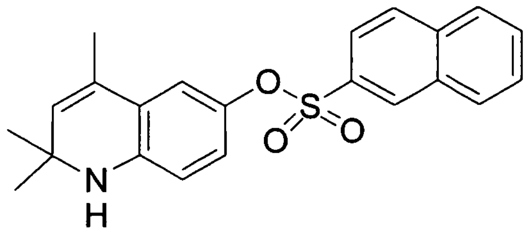

It was produced by the same methods as Reference examples 49-50 and then Example 51.
$^1$H-NMR ($CDCl_3$) δ :1.25(6H,s), 1.67(3H,s), 5.26-5.27(1H,m), 6.19-6.22(1H,m), 6.51-6.59(2H,m), 7.58-7.71(2H,m), 7.83-8.04 (4H,m), 8.37(1H,m)

Example 53

7-Chloro-2,2,4-trimethyl-1,2-dihydroquinolin-6-yl 4-methylbenzenesulfonate

It was produced by the same methods as Reference examples 49-50 and then Example 51 from 2-chloro-4-nitrophenol.
$^1$H-NMR ($CDCl_3$) δ :1.26(6H,s), 1.85(3H,m), 2.46(3H,s), 3.74(1H,br.s), 5.32(1H,s), 6.32(1H,s), 6.83(1H,s), 7.26-7.32 (2H,m), 7.76-7.81(2H,m)

Example 54

7-Fluoro-2,2,4-trimethyl-1,2-dihydroquinolin-6-yl 4-methylbenzenesulfonate

[Chemical Formula 43]

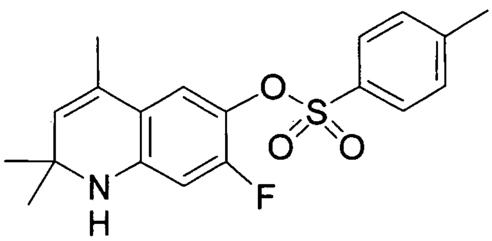

It was produced by the same methods as Reference examples 49-50 and then Example 51 from 2-fluoro-4-nitrophenol.
$^1$H-NMR ($CDCl_3$) δ :1.23-1.26(6H,m), 1.56(3H,m), 1.83(3H,m), 2.45(3H,s), 3.76(1H,br.s), 5.27(1H,s), 6.02-6.06(1H,m), 6.73-6.78(1H,m), 7.29-7.32(2H,m), 7.75-7.78(2H,m)

Example 55

2,2,4,7-Tetramethyl-1,2-dihydroquinolin-6-yl 4-methylbenzenesulfonate

It was produced by the same methods as Reference examples 49-50 and then Example 51 from 2-methyl-4-

nitrophenol.
$^1$H-NMR ($CDCl_3$) δ :1.24(6H,s), 1.74(3H,m), 1.94(3H,s), 2.45(3H,s), 3.30(1H,br.s), 5.26(1H,s), 6.16(1H,s), 6.49(1H,s), 6.49(1H,s), 7.29-7.32(2H,m), 7.72-7.75(2H,m)

Example 56

8-Bromo-2,2,4-trimethyl-1,2-dihydroquinolin-6-yl 4-methylbenzenesulfonate

A mixture of the compound (103mg, 0.30 mmol) prepared in Example 51 and N-bromosuccinimide (64mg, 0.36 mmol) was stirred in tetrahydrofuran (1.0mL) at 20-25°C for 10 hours. The solvent was distilled off under reduced pressure and the obtained residue was purified by thin layer chromatography (hexane/ethyl acetate = 2/1) to give 8-bromo-2,2,4-trimethyl-1,2-dihydroquinolin-6-yl 4-methylbenzenesulfonate (61mg, 48%).
$^1$H-NMR ($CDCl_3$) δ :1.16-1.30(6H,s), 1.46(3H,s), 2.44(3H,s), 3.96(1H,s), 5.05(1H,s), 6.43-6.48(1H,m), 6.70-6.77(1H,s), 7.32(2H,d,J=8.6Hz), 7.71(2H,d,J=8.2Hz)

Example 57

2,2,4-Trimethyl-6-{[(4-methylphenyl)sulfonyl]methyl}-1,2-dihydroquinoline

[Chemical Formula 44]

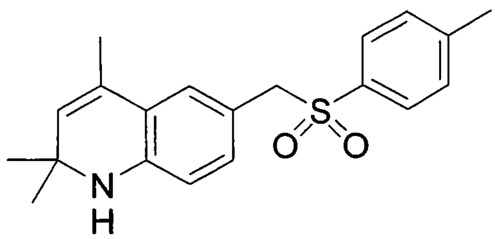

To a dichloromethane (5.9mL) solution of the compound (262mg, 0.593 mmol) prepared in Reference example 55, trifluoroacetic acid (1.2mL) was added dropwise at 20-25°C, and the mixture was stirred at the same temperature for 2 hours. The reaction liquid was diluted with ethyl acetate and poured into saturated aqueous sodium bicarbonate. The mixture was made to alkali by a small amount of aqueous ammonia and extracted with ethyl acetate. The organic layer was washed with saturated aqueous sodium chloride and dried over anhydrous magnesium sulfate. The crude product concentrated under reduced pressure was purified by silica gel column chromatography (hexane/ethyl acetate = 5/1) to give 2,2,4-trimethyl-6-{[(4-methylphenyl)sulfonyl]methyl}-1,2-dihydroquinoline (180mg, 89%).
$^1$H-NMR ($CDCl_3$) δ :1.26(6H,s), 1.79(3H,d,J=1.3Hz), 2.41(3H,s), 3.75(1H,br.s), 4.15(2H,s), 5.28(1H,d,J=1.3Hz), 6.32 (1H,d,J=8.1Hz), 6.59 (1H,d,J=1.8Hz), 6.71(1H,dd,J=8.1, 1.8Hz), 7.24(2H,d,J=8.4Hz), 7.54(2H,d,J=8.4Hz)

Example 58

2,2,4-Trimethyl-6-{[(4-methylphenyl)sulfonyl]ethyl}-1,2-dihydroquinoline

It was produced by the same method as Example 57 from the compound prepared in Reference example 56.
$^1$H-NMR ($CDCl_3$) δ :1.26(6H,s), 1.69(3H,d,J=7.2Hz), 1.79(3H,s), 2.39(3H,s), 3.76 (1H,br.s), 4.09(1H,q,J=7.2Hz), 5.27 (1H,s), 6.32(1H,d,J=8.3Hz), 6.62(1H,d, J=2.0Hz), 6.78(1H,dd,J=8.3, 2.0Hz), 7.19(2H,d,J=8.3Hz), 7.45(2H, d,J=8.3Hz)

Example 59

2,2,4-Trimethyl-6-{1-[(4-methylphenyl)sulfonyl]-2-phenylethy}-1,2-dihydroquinoline

It was produced by the same methods as Example 58 and then Example 57 from the compound prepared in Reference example 55.
$^1$H-NMR ($CDCl_3$) δ :1.24(6H,s), 1.76(3H,d,J=1.3Hz), 2.38(3H,s), 3.32(1H, dd,J=13.9, 11.6Hz), 3.73(1H,dd,J=13.9, 2.9Hz), 4.12(1H,dd,J=11.6, 2.9Hz), 5.25 (1H,d,J=1.1Hz), 6.27(1H,d,J=8.1Hz), 6.56(1H,d,J=1.8Hz), 6.76(1H,dd,J=8.1, 1.8Hz), 7.00-7.14(5H,m), 7.18(2H,d,J=8.3Hz), 7.47(2H,d,J=8.3Hz)

Example 60

4-Methyl-N-(2,2,4-trimethyl-1,2-dihydroquinolin-6-yl)benzenesulfonamide

[Chemical Formula 45]

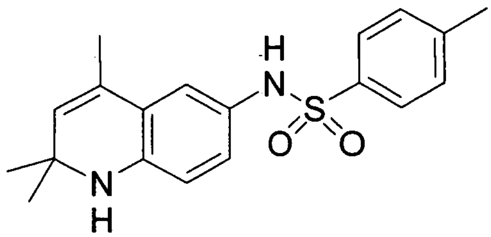

It was produced by the same method as Example 51 from N-(4'-aminophenyl)-4-methylbenzenesulfonamide.
$^1$H-NMR ($CDCl_3$) δ :1.25(6H,s), 1.83(3H,d,J=1.3Hz), 2.39(3H,s), 3.70(1H,br.s), 5.30(1H,dd,J=1.5Hz), 6.02(1H,s), 6.28 (1H,d,J=8.3Hz), 6.62(1H,dd,J=8.3, 2.4Hz), 6.66(1H,d,J=2.4Hz), 7.22(2H,d,J=8.4Hz), 7.58(2H,d,J=8.4Hz)

Example 61

N-(2,2,4-Trimethyl-1,2-dihydroquinolin-6-yl)naphthalene-2-sulfonamide

[Chemical Formula 46]

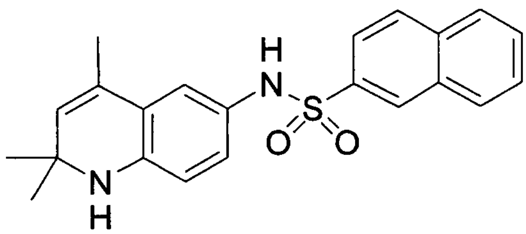

To a tetrahydrofuran (1mL) solution of the compound (50mg, 0.26 mmol) prepared in Reference example 58, 2-naphthalenesulfonyl chloride (66.2mg, 0.29 mmol) and triethylamine (80.6mg, 0.80 mmol) were added and stirred at 20-25°C for 3 hours. The reaction liquid was poured into water and extracted with ethyl acetate. The organic layer was washed with water and saturated aqueous sodium chloride subsequently, and dried over anhydrous magnesium sulfate. The solvent was distilled off under reduced pressure and the obtained residue was purified by thin layer chromatography (hexane/ethyl acetate = 10/1) to give N-(2,2,4-trimethyl-1,2-dihydroquinolin-6-yl) naphthalene-2-sulfonamide (71mg, 70%).

$^{1}H$-NMR ($CDCl_3$) δ :1.22(6H,s), 1.58(3H,m), 1.73(3H,m), 3.67(1H,br.s), 5.26(1H,s), 6.19-6.24(2H,m), 6.58-6.62(1H,m), 6.68-6.69(1H,m), 7.54-7.73 (3H,m), 7.86-7.99(3H,m), 8.26(1H,m)

Example 62

N-(2,2,4-Trimethyl-1,2-dihydroquinolin-6-yl)thiophene-2-sulfonamide

[Chemical Formula 47]

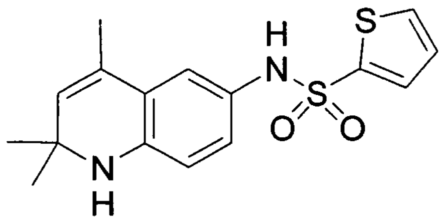

It was produced by the same method as Example 61 from the compound prepared in Reference example 58.
$^{1}H$-NMR ($CDCl_3$) δ :1.26(6H,s), 1.59(3H,s), 1.89(3H,d,J=1.26), 3.73(1H,s), 5.31(1H,s), 6.20(1H,br.s), 6.30-6.32(1H,m), 6.68-6.72 (2H,m), 6.99-7.02(1H,m), 7.39-7.41(1H,m), 7.53-7.55(1H,m)

Example 63

N-(2,2,4-Trimethyl-1,2-dihydroquinolin-6-yl)benzenesulfonamide

[Chemical Formula 48]

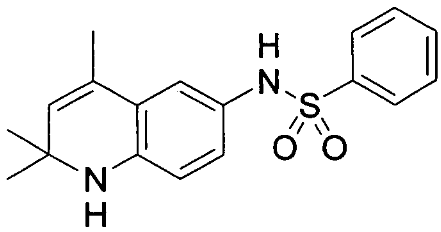

It was produced by the same method as Example 61 from the compound prepared in Reference example 58.
$^{1}H$-NMR ($CDCl_3$) δ:1.25(6H,s), 1.81(3H,s), 3.71(1H,s), 5.30 (1H,s), 6.16(1H,s), 6.26-6.30(1H,m), 6.60-6.66(2H,m), 7.39-7.46(2H,m), 7.50-7.56(1H,m), 7.68-7.73 (2H,m)

Example 64

4-Chloro-N-(2,2,4-trimethyl-1,2-dihydroquinolin-6-yl)benzenesulfonamide

It was produced by the same method as Example 61 from the compound prepared in Reference example 58.
$^{1}H$-NMR ($CDCl_3$) δ :1.26(6H,s), 1.84(3H,s), 3.73(1H,s), 5.32 (1H,s), 6.20(1H,s), 6.28(1H,d,J=8.3Hz), 6.60(1H,dd,J=2.4, 3.8Hz), 6.68(1H,d,J=2.4Hz), 7.40(2H,d, J=6.7Hz), 7.63(2H,d,J=8.4Hz)

Example 65

4-Methoxy-N-(2,2,4-trimethyl-1,2-dihydroquinolin-6-yl)benzenesulfonamide

It was produced by the same method as Example 61 from the compound prepared in Reference example 58. $^1H$-NMR ($CDCl_3$) $\delta$ :1.25(6H,s), 1.84(3H,s), 3.71(1H,s), 3.84(1H,s), 5.31(1H,s), 6.08(1H,s), 6.28(1H,d,J=8.2Hz), 6.62 (1H,dd,J=2.4, 3.8Hz), 6.68(1H,d,J=2.4Hz), 6.89(2H,d,J=6.7Hz), 7.63(2H,d, J=8.4Hz)

Example 66

5-Chloro-N-(2,2,4-trimethyl-1,2-dihydroquinolin-6-yl)thiophene-2-sulfonamide

[Chemical Formula 49]

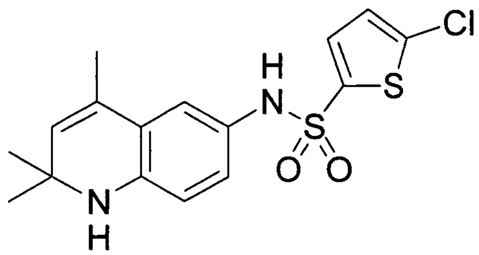

It was produced by the same method as Example 61 from the compound prepared in Reference example 58. $^1H$-NMR ($CDCl_3$) $\delta$ :1.26(6H,s), 1.89(3H,s), 3.76(1H,s), 5.33(1H,s), 6.20(1H,s), 6.33(1H,d,J=8.2Hz), 6.70(1H,dd,J=2.4, 3.8Hz), 6.76(1H,d,J=2.4Hz), 6.84(2H, d,J=4.OHz), 7.18(2H,d,J=4.0Hz)

Example 67

2-Fluoro-N-(2,2,4-trimethyl-1,2-dihydroquinolin-6-yl)benzenesulfonamide

[Chemical Formula 50]

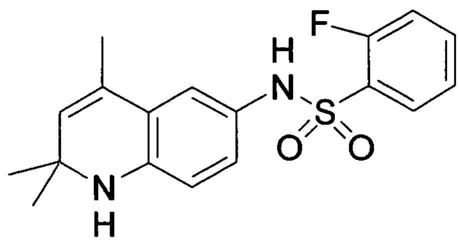

It was produced by the same method as Example 61 from the compound prepared in Reference example 58. $^1H$-NMR ($CDCl_3$) $\delta$ :1.22(6H,s), 1.83(3H,s), 3.49(1H,s), 5.28(1H,s), 6.23-6.26(1H,d,J=8.22Hz), 6.44(14H,br.s), 6.69-6.74 (2H,m), 7.14-7.24(2H,m), 7.49-7.59(1H,m), 7.69-7.74(1H,m)

Example 68

3-Fluoro-N-(2,2,4-trimethyl-1,2-dihydroquinolin-6-yl)benzenesulfonamide

[Chemical Formula 51]

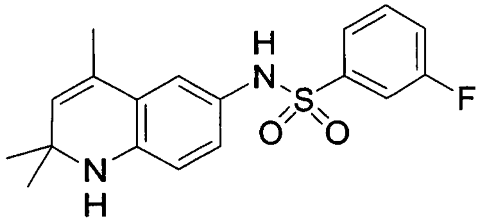

It was produced by the same method as Example 61 from the compound prepared in Reference example 58.
$^1$H-NMR ($CDCl_3$) δ :1.23(6H,s), 1.81(3H,m), 3.71(1H,br.s), 5.28(1H,s), 6.20(1H,br.s), 6.25-6.28(1H,d,J=8.22Hz), 6.59-6.65(2H,m), 7.18-7.24(1H,m), 7.36-7.48(3H,m)

Example 69

4-Fluoro-N-(2,2,4-trimethyl-1,2-dihydroquinolin-6-yl)benzenesulfonamide

[Chemical Formula 52]

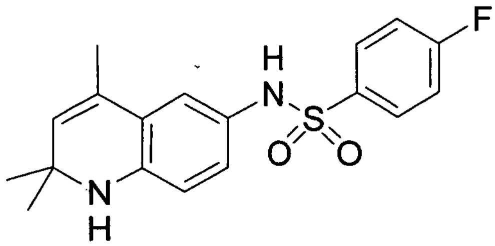

It was produced by the same method as Example 61 from the compound prepared in Reference example 58.
$^1$H-NMR ($CDCl_3$) δ :1.25(6H,s), 1.83(3H,m), 3.72(1H,br.s), 5.31(1H,s), 6.24-6.29(2H,m), 6.59-6.67(2H,m), 7.06-7.13 (2H,m), 7.60- 7.73(2H,m)

Example 70

4-(Trifluoromethyl)-N-(2,2,4-trimethyl-1,2-dihydroquinolin-6-yl) benzenesulfonamide

[Chemical Formula 53]

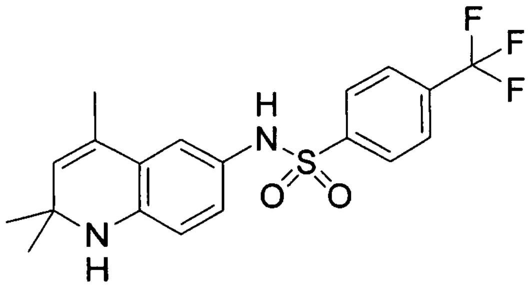

It was produced by the same method as Example 61 from the compound prepared in Reference example 58.
$^1$H-NMR ($CDCl_3$) δ :1.25(6H,s), 1.80(3H,m), 3.74(1H,br.s), 5.31(1H,s), 6.27-6.29(1H,d,J=8.0Hz), 6.60-6.65(2H,m), 7.68-7.71(2H,m), 7.81-7.84(2H,m)

Example 71

4-Nitro-N-(2,2,4-trimethyl-1,2-dihydroquinolin-6-yl)benzenesulfonamide

It was produced by the same method as Example 61 from the compound prepared in Reference example 58. $^1$H-NMR ($CDCl_3$) δ :1.26(6H,s), 1.84(3H,s), 3.78(1H,br.s), 5.32(1H,s), 6.26-6.33 (2H,m), 6.55-6.59(1H,m), 6.71-6.72 (1H,m), 7.86-7.89(2H,m), 8.26-8.29(2H,m)

Example 72

4-Amino-N-(2,2,4-trimethyl-1,2-dihydroquinolin-6-yl)benzenesulfonamide

It was produced by the same method as Reference example 15 from the compound prepared in Example 71. $^1$H-NMR ($CDCl_3$) δ :1.24(6H,s), 1.84(3H,s), 4.05-4.15(1H,br.s), 5.30(1H,s), 6.04(1H,s), 6.26-6.29(1H,m), 6.56-6.69(4H, m), 7.44-7.47(2H,m)

Example 73

N-(2,2,4-Trimethyl-1,2-dihydroquinolin-6-yl)pyridine-3-sulfonamide

[Chemical Formula 54]

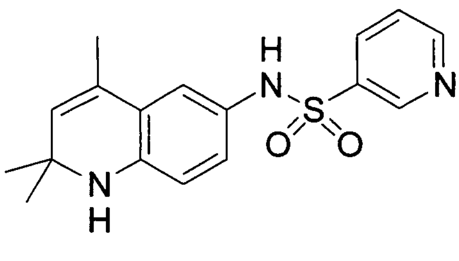

It was produced by the same method as Example 61 from the compound prepared in Reference example 58.
$^1$H-NMR ($CDCl_3$) δ :1.26(6H,s), 1.83(3H,s), 3.74(3H,s), 5.31(1H,s), 6.23(1H,s), 6.28(1H,d,J=8.4Hz), 6.62(1H,dd,J=8.4, 2.4Hz), 6.68(1H,d,J=2.4Hz), 7.34-7.40 (1H,m), 7.90-7.96(2H,m), 8.74-8.78(1H, m), 8.93(2H,d,J=4.6Hz)

Example 74

N-(2,2,4-Trimethyl-1,2-dihydroquinolin-6-yl)thiophene-3-sulfonamide

[Chemical Formula 55]

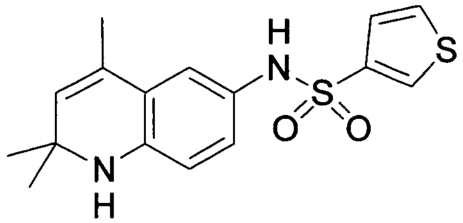

It was produced by the same method as Example 61 from the compound prepared in Reference example 58.
$^1$H-NMR ($CDCl_3$) δ :1.25(6H,s), 1.85(3H,m), 3.73(1H,br.s), 5.31(1H,s), 6.17(1H, br.s), 6.29-6.32(1H,m), 6.66-6.68(2H, m), 7.21-7.22(1H,m), 7.33-7.36(1H,m), 7.75-7.76(1H,m)

Example 75

N-(2,2,4-Trimethyl-1,2-dihydroquinolin-6-yl)-1-benzothiophene-2-sulfonamide

[Chemical Formula 56]

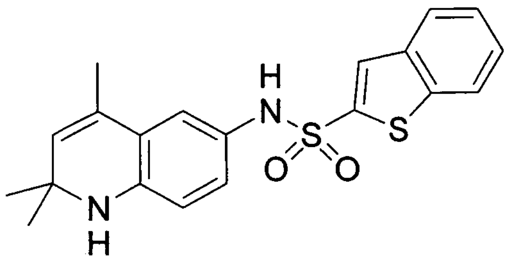

It was produced by the same method as Example 61 from the compound prepared in Reference example 58.
$^1$H-NMR ($CDCl_3$) δ :1.25(6H,s), 1.77(3H,m), 3.72(1H,br.s), 5.28(1H,s), 6.25-6.29(2H,m), 6.70-6.72(1H,m), 6.77(1H,br.s), 7.39-7.49(2H,m), 7.63(1H,br.s), 7.78-7.85(2H,m)

Example 76

4-(1-Hydroxyethyl)-N-(2,2,4-trimethyl-1,2-dihydroquinolin-6-yl) benzenesulfonamide

It was produced by the same method as Example 61 and then Reference example 9 from the compound prepared in Reference example 58.
LC-MS (M+1): 373.1

Example 77

3-Methyl-N-(2,2,4-trimethyl-1,2-dihydroquinolin-6-yl)benzenesulfonamide

It was produced by the same method as Example 61 from the compound prepared in Reference example 58.
$^1$H-NMR ($CDCl_3$) δ :1.25(6H,s), 1.82(3H,m), 2.36(3H,s), 3.70(1H,br.s), 5.30(1H,s), 6.05(1H,br.s), 6.26-6.30(1H,m),

6.61-6.64(2H,m), 7.30-7.34(2H,m), 7.44-7.52(2H,m)

Example 78

2-Methyl-N-(2,2,4-trimethyl-1,2-dihydroquinolin-6-yl)benzenesulfonamide

[Chemical Formula 57]

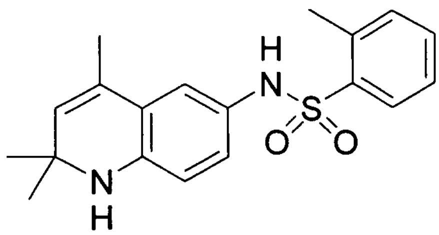

It was produced by the same method as Example 61 from the compound prepared in Reference example 58.
$^1$H-NMR ($CDCl_3$) δ :1.22(6H,s), 2.61(3H,m), 3.86-3.75(1H,s), 5.27(1H,s), 6.08-6.28(2H,m), 6.57-6.67(2H,m), 7.19-7.32 (2H,m), 7.83-7.85(1H,m)

Example 79

N-(7-Chloro-2,2,4-trimethyl-1,2-dihydroquinolin-6-yl)-4-methylbenzenesulfonamide

It was produced by the same method as Reference example 57 from the compound prepared in Reference example 60.
$^1$H-NMR ($CDCl_3$) δ :1.26(6H,s), 1.97(3H,m), 2.38(3H,s), 3.70(1H,br.s), 5.35(1H,s), 6.26(1H,s), 6.42(1H,s), 7.18-7.20 (2H,m), 7.26-7.27(1H,m), 7.55-7.58(2H,m)

Example 80

4-Methyl-N-(2,2,4,7-tetramethyl-1,2-dihydroquinolin-6-yl)benzenesulfonamide

[Chemical Formula 58]

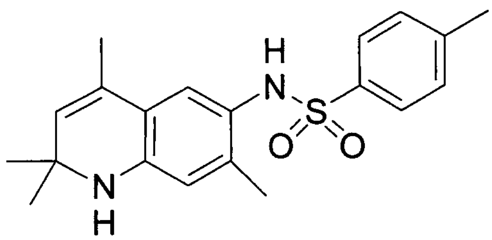

It was produced by the same method as Reference example 57 from the compound prepared in Reference example 62.
$^1$H-NMR ($CDCl_3$) δ :1.24(6H,s), 1.76-1.77(3H,m), 1.89(3H,s), 2.40(3H,s), 3.65(1H,br.s), 5.24(1H,s), 5.90(1H,s), 6.17 (1H,s), 6.63(1H,s), 7.21-7.24(2H,m), 7.57-7.60(2H,m)

Example 81

N,4-Dimethyl-N-(2,2,4-trimethyl-1,2-dihydroquinolin-6-yl) benzenesulfonamide

[Chemical Formula 59]

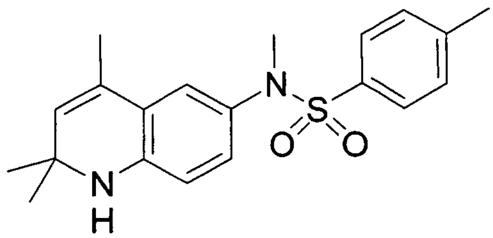

A mixture of the compound (52mg, 0.15 mmol) prepared in Example 60, iodomethane (33 μL, 0.53 mmol) and potassium carbonate (50mg, 0.37 mmol) was stirred in acetone (2.5mL) at 20-25°C for 3.5 hours. After filtrating off the precipitate, the solvent was distilled off under reduced pressure and the obtained residue was purified by silica gel column chromatography (hexane/ethyl acetate = 10/1 - 8/1) to give N,4-dimethyl-N-(2,2,4-trimethyl-1,2-dihydroquinolin-6-yl)benzenesulfonamide (33mg, 62%).
$^1$H-NMR ($CDCl_3$) δ :1.25(6H,s), 1.80(3H,d,J=1.3Hz), 2.40(3H,s), 3.08(3H,s), 3.74(1H,br.s), 5.27(1H,d,J=1.3Hz), 6.27 (1H,d,J=8.4Hz), 6.59(1H,dd,J=8.4, 2.4Hz), 6.65(1H,d,J=2.4Hz), 7.23(2H, d,J=8.3Hz), 7.48(2H,d,J=8.3Hz)

Example 82

N-Benzyl-4-methyl-N-(2,2,4-trimethyl-1,2-dihydroquinolin-6-yl) benzenesulfonamide

It was produced by the same method as Example 81 from the compound prepared in Example 60.
$^1$H-NMR ($CDCl_3$) δ :1.23(6H,s), 1.71(3H,d,J=1.1Hz), 2.44(3H,s), 3.70(1H,br.s), 4.63(2H,s), 5.24(1H,s), 6.19(1H,d, J=8.1Hz), 6.48-6.53(2H,m), 7.19-7.29(7H,m), 7.60(2H,d,J=8.3Hz)

Example 83

N-Allyl-4-methyl-N-(2,2,4-trimethyl-1,2-dihydroquinolin-6-yl) benzenesulfonamide

It was produced by the same method as Example 81 from the compound prepared in Example 60.
$^1$H-NMR ($CDCl_3$) δ :1.27(6H,s), 1.80(3H,s), 2.42(3H,s), 3.74(1H,s), 4.09(1H,d,J=6.4Hz), 5.00-5.11(2H,m), 5.28(1H,s), 5.68-5.81 (1H,m), 6.27(1H,d,J=7.9Hz), 6.56-6.61(2H,m), 7.25(2H,d, J=8.0Hz), 7.55(2H,d,J=8.2Hz)

Example 84

N-Isopropyl-4-methyl-N-(2,2,4-trimethyl-1,2-dihydroquinolin-6-yl) benzenesulfonamide

It was produced by the same method as Example 81 from the compound prepared in Example 60.
$^1$H-NMR ($CDCl_3$) δ :1.01(6H,d,J=6.6Hz), 1.29(6H,s), 1.82(3H,s), 2.41(3H,s), 3.77(1H,s), 4.51-4.62(1H,m), 5.28(1H,s), 5.68-5.81 (1H,m), 6.31(1H,d,J=8.6Hz), 6.57-6.63(2H,m), 7.24(2H,d, J=8.2Hz), 7.64(2H,d,J=8.3Hz)

Example 85

N-Methyl-N-(2,2,4-trimethyl-1,2-dihydroquinolin-6-yl)thiophene-2-sulfonamide

[Chemical Formula 60]

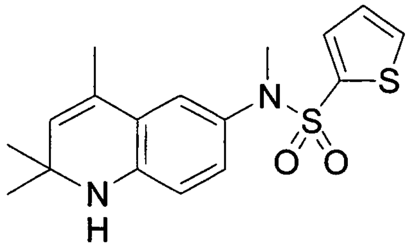

It was produced by the same method as Example 81 from the compound prepared in Example 62.
$^1$H-NMR ($CDCl_3$) δ :1.27(6H,s), 1.84(3H,s), 3.18(3H,s), 3.76(1H,s), 5.30(1H,s), 6.31(1H,d,J=8.2Hz), 6.65-6.73(2H,m), 7.06-7.10(1H,m), 7.35-7.39(1H,m), 7.55-7.59(1H,m)

Example 86

N-[(4-Methylphenyl)sulfonyl]-N-(2,2,4-trimethyl-1,2-dihydroquinolin-6-yl) acetamide

[Chemical Formula 61]

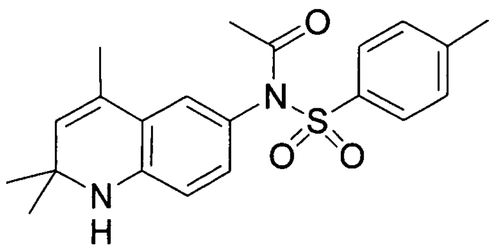

A mixture of the compound (34mg, 0.10 mmol) prepared in Example 60, acetyl chloride (7.0 μL, 0.10 mmol) and triethylamine (42 μL, 0.30 mmol) was stirred in tetrahydrofuran (0.5mL) at 20-25°C for 17 hours. Acetyl chloride (7.0 μL, 0.10 mmol) and triethylamine (28 μ L, 0.20 mmol) were further added and stirred at 50°C for 3 hours. After filtrating off the precipitate, the solvent was distilled off under reduced pressure and the obtained residue was purified by thin layer chromatography (chloroform/ethyl acetate = 10/1) to give N-[(4-methylphenyl)sulfonyl]-N-(2,2,4-trimethyl-1,2-dihydroquinolin-6-yl)acetamide (24.0mg, 62%).
$^1$H-NMR ($CDCl_3$) δ :1.32(6H,s), 1.89(3H,s), 1.93(3H,s), 2.45(3H,s), 3.94(1H,s), 5.35(1H,s), 6.42(1H,d,J=8.2Hz), 6.77-6.85(2H,m), 7.33(2H,d,J=8.0Hz), 7.93(2H,d,J=8.2Hz)

Example 87

N-(Phenylsulfonyl)-N-(2,2,4-trimethyl-1,2-dihydroquinolin-6-yl)acetamide

[Chemical Formula 62]

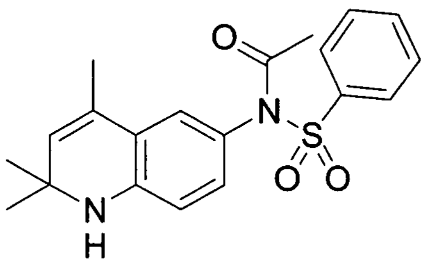

It was produced by the same method as Example 86 from the compound prepared in Example 63.
$^1$H-NMR ($CDCl_3$) δ :1.32(6H,s), 1.90(3H,s), 1.93(3H,s), 3.95(1H,s), 5.36(1H,s), 6.42(1H,d,J=8.3Hz), 6.81(1H,dd,J=8.3, 2.4Hz), 6.84(1H,d,J=2.4Hz), 7.51-7.67(3H,m), 8.06(2H,d,J=7.1Hz)

Example 88

N-[(4-Chlorophenyl)sulfonyl]-N-(2,2,4-trimethyl-1,2-dihydroquinolin-6-yl) acetamide

[Chemical Formula 63]

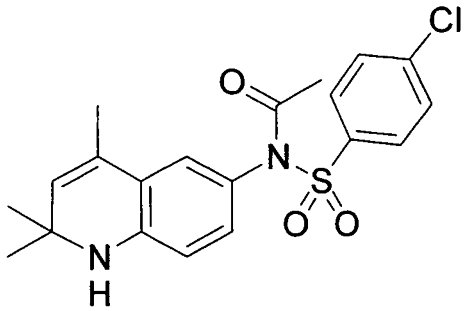

It was produced by the same method as Example 86 from the compound prepared in Example 64.
$^1$H-NMR ($CDCl_3$) δ : 1.33(6H,s), 1.90(3H,s), 1.94(3H,s), 3.96(1H,s), 5.36(1H,s), 6.42(1H,d,J=8.3Hz), 6.77(1H,dd,J=8.3, 2.4Hz), 6.83(1H,d,J=2.4Hz), 7.51(2H,d, J=8.5Hz), 7.99(2H,d,J=8.5Hz)

Example 89

N-[(5-Chloro-2-thienyl)sulfonyl]-N-(2,2,4-trimethyl-1,2-dihydroquinolin-6-yl) acetamide

[Chemical Formula 64]

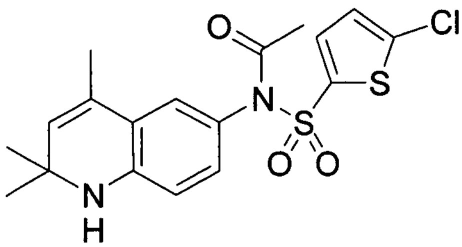

It was produced by the same method as Example 86 from the compound prepared in Example 66.
$^1$H-NMR ($CDCl_3$) δ : 1.32(6H,s), 1.94(3H,s), 1.96(3H,s), 3.96(1H,s), 5.36(1H,s), 6.41(1H,d,J=8.3Hz), 6.78(1H,dd,J=8.3, 2.3Hz), 6.84(1H,d,J=2.3Hz), 6.96(1H,d,J=4.1Hz), 7.66(1H,d,J=4.1Hz)

Example 90

Methyl N-[(4-methylphenyl)sulfonyl]-N-(2,2,4-trimethyl-1,2-dihydroquinolin-6-yl)glycinate

It was produced by the same method as Example 81 from the compound prepared in Example 60.
$^1$H-NMR ($CDCl_3$) δ :1.26(6H,s), 1.79(3H,s), 2.41(3H,s), 3.69(3H,s), 3.77(1H,s), 4.34(2H,s), 5.27(1H,s), 6.26(1H,d, J=9.3Hz), 6.73-6.78(2H,m), 7.24(2H,d, J=8.4Hz), 7.59(2H,d,J=8.2Hz)

Example 91

Methyl oxo[(2-thienylsulfonyl)-(2,2,4-trimethyl-1,2-dihydroquinolin-6-yl) amino] acetate

[Chemical Formula 65]

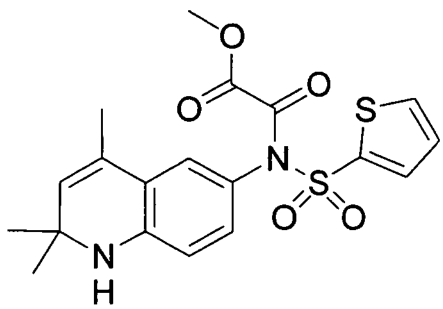

To a tetrahydrofuran (3.0mL) solution of the compound (315mg, 0.942 mmol) prepared in Example 62, sodium hydride (content: 60%) (54mg, 1.4 mmol) was added under ice-cooling and stirred for 10 minutes. Under ice-cooling, a tetrahydrofuran (2.0mL) solution of methyl chloroglyoxylate (104μ L, 1.13 mmol) was added dropwise to the reaction liquid and stirred at 20-25°C for 14 hours. Water was poured into the reaction liquid, which was followed by extraction with ethyl acetate. The organic layer was washed with water and saturated aqueous sodium chloride subsequently, and dried over anhydrous sodium sulfate. The solvent was distilled off under reduced pressure and the obtained residue was purified by silica gel column chromatography (ethyl acetate/hexane = 4/1) to give methyl oxo[(2-thienylsulfonyl)-(2,2,4-trimethyl-1,2-dihydroquinolin-6-yl)amino]acetate (317mg, 80%).
$^1$H-NMR ($CDCl_3$) δ :1.29(6H,s), 1.84(3H,d,J=1.3Hz), 3.76(3H,s), 3.98(1H,br.s), 5.30(1H,s), 6.34(1H,d,J=7.9Hz), 6.71-6.74(2H,m), 7.15(1H,dd,J=5.0, 3.9Hz), 7.75(1H,dd,J=5.0, 1.3Hz), 7.82(1H,dd, J=3.9, 1.3Hz)

Example 92

Methyl 2-{[[(4-methylphenyl)sulfonyl]-(2,2,4-trimethyl-1,2-dihydroquinolin-6-yl)amino]sulfonyl}benzoate

It was produced by the same method as Example 91 from the compound prepared in Example 60.
$^1$H-NMR ($CDCl_3$) δ :1.26(3H,s), 1.30(3H,s), 1.74(3H,d,J=1.5Hz), 2.45(3H,s), 3.66(3H,s), 3.89(1H,br.s), 5.27(1H,d, J=1.3Hz), 6.27(1H,d,J=8.4Hz), 6.62(1H, d,J=2.4Hz), 6.66(1H,dd,J=8.3, 2.4Hz), 7.26-7.31(2H,m), 7.51-7.55(1H,m), 7.61-7.69(2H,m), 7.76-7.80(2H,m), 8.34-8.40(1H,m)

Example 93

N,4-Dimethyl-N-(2,2,4,7-tetramethyl-1,2-dihydroquinolin-6-yl) benzenesulfonamide

It was produced by the same method as Reference example 57 from the compound prepared in Reference

example 64.
$^1$H-NMR ($CDCl_3$) δ :1.07(6H,m), 1.57(3H,s), 2.26(3H,s), 2.44(3H,s), 3.09(3H,s), 3.70(1H,br.s), 5.19(1H,s), 6.10(1H,s), 6.27(1H,s), 7.26-7.30(3H,m), 7.61-7.63(2H,m)

Example 94

4-Methyl-N-(6,6,8-trimethyl-5,6-dihydro-1,5-naphthyridin-2-yl) benzenesulfonamide

It was produced by the same method as Reference example 57 from the compound prepared in Reference example 66.
$^1$H-NMR ($CDCl_3$) δ :1.26(6H,s), 1.82(3H,d,J=1.3Hz), 2.37(3H,s), 5.49(1H,m), 6.65(1H,d,J=8.4Hz), 6.99(1H,d,J=8.4Hz), 7.20-7.23 (1H,m), 7.69-7.71(1H,m)

Example 95

2,2,4-Trimethyl-1,2,3,4-tetrahydroquinolin-6-yl 4-methylbenzenesulfonate

To a methanol (5.0mL) solution of the hydrochloride (68mg, 0.18 mmol) of the compound prepared in Example 51, 10% palladium-carbon (containing 50% water) (74mg) was added and stirred under a hydrogen atmosphere of 0.3MPa at 20-25°C for one hour. After the reaction, the catalyst was filtered off and the filtrate was concentrated under reduced pressure. The residue was subjected to extraction-separation with chloroform-aqueous sodium bicarbonate. The organic layer was dried over anhydrous sodium sulfate. The solvent was distilled off under reduced pressure and the obtained residue was purified by silica gel column chromatography (hexane/ethyl acetate = 10/1 - 5/1) to give 2,2,4-trimethyl-1,2,3,4-tetrahydroquinolin-6-yl 4-methylbenzenesulfonate (35mg, 56%).
$^1$H-NMR ($CDCl_3$) δ :1.12(3H,d,J=6.6Hz), 1.14(3H,s), 1.22(3H,s), 1.34(1H, t-like,J=13Hz), 1.69(1H,dd,J=13, 5.6Hz), 2.44 (3H,s), 2.79(1H,m), 3.61(1H,br.s), 6.26(1H,d,J=8.6Hz), 6.56(1H,dd,J=8.6, 2.7Hz), 6.66(1H,dd,J=2.7, 1.1Hz), 7.29(2H, d,J=8.7Hz), 7.70(2H,d, J=8.7Hz)

Example 96

2, 2,4-Trimethyl-6-{[(4-methylphenyl)sulfonyl]methyl}-1, 2, 3,4-tetrahydroquinoline

[Chemical Formula 66]

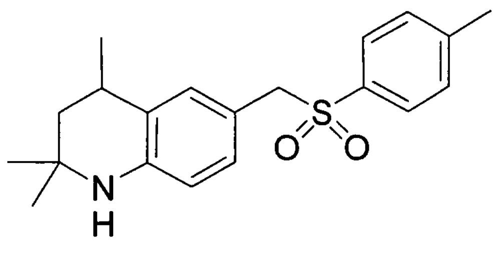

It was produced by the same method as Example 95 from the compound prepared in Example 57.
$^1$H-NMR ($CDCl_3$) δ :1.12(3H,d,J=6.8Hz), 1.15(3H,s), 1.23(3H,s), 1.38 (1H,d, J=12.7Hz), 1.70(1H,dd,J=12.7, 5.6Hz), 2.41(3H,s), 2.77(1H,hept,J=6.2Hz), 3.67 (1H,br.s), 4.13(1H,d,J=13.9Hz), 4.19(1H,d,J=13.9Hz), 6.33(1H,d,J=8.1Hz), 6.69 (1H,d,J=1.8Hz), 6.73(1H,dd,J=8.1, 1.8Hz), 7.23(2H,d,J=8.3Hz), 7.52(2H,d, J=8.3Hz)

Example 97

4-Methyl-N-(2,2,4-trimethy-1,2,3,4-tetrahydroquinolin-6-yl) benzenesulfonamide

[Chemical Formula 67]

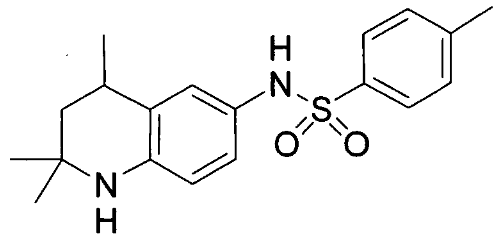

It was produced by the same method as Example 95 from the hydrochloride of the compound prepared in Example 60. $^1$H-NMR ($CDCl_3$) δ :1.14(3H,s), 1.16(3H,d,J=6.8Hz), 1.22(3H,s), 1.35 (1H,t-like,J=13Hz), 1.70(1H,dd,J=13, 5.6Hz), 2.39 (3H,s), 2.80(1H,m), 6.09(1H,br.s), 6.28(1H,d,J=8.5Hz), 6.61(1H,dd, J=8.5, 2.4Hz), 6.75(1H,d,J=2.4Hz), 7.21(2H,d, J=8.5Hz), 7.57(2H,d, J=8.4Hz)

Example 98

N-(2,2,4-Trimethy-1,2,3,4-tetrahydroquinolin-6-yl)thiophene-2-sulfonamide

[Chemical Formula 68]

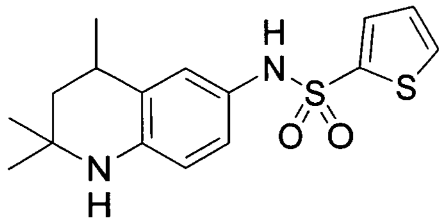

It was produced by the same method as Example 61 from the compound prepared in Reference example 67.
$^1$H-NMR ($CDCl_3$) δ :1.12-1.28(9H,m), 1.38(1H,t,J=12.6Hz), 1.72(1H,dd,J=12.8, 5.7Hz), 2.76-2.90(1H,m), 6.12(1H,s), 6.32(1H,d,J=8.4Hz), 6.68(1H,dd,J=8.4, 2.4Hz), 6.83(1H,s), 7.00(1H,t, J=3.8Hz), 7.36-7.40(1H,m), 7.50-7.55(1H,m)

Example 99

N-(2,2,4-Trimethy-1,2,3,4-tetrahydroquinolin-6-yl)naphthalene-2-sulfonamide

[Chemical Formula 69]

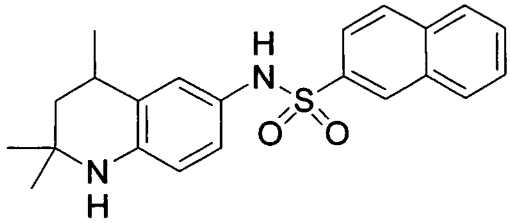

It was produced by the same method as Example 95 from the compound prepared in Example 61.
$^1$H-NMR ($CDCl_3$) δ :1.01-1.20(9H,m), 1.31(1H,t,J=12.4Hz), 1.65(1H,dd,J=12.8, 5.4Hz), 2.69-2.77(1H,m), 6.17(1H,br.s), 6.27(1H,d, J=8.1Hz), 6.62(1H,dd,J=8.4, 2.5Hz), 6.74(1H,s), 7.54-7.64(2H,m), 7.70(1H,dd,J=8.8, 1.5Hz), 7.85-7.90(3H, m), 8.24(1H,s)

Example 100

N,4-Dimethyl-N-(2,2,4-trimethy-1,2,3,4-tetrahydroquinolin-6-yl) benzenesulfonamide

[Chemical Formula 70]

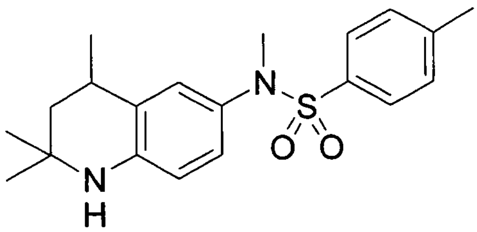

It was produced by the same method as Example 81 from the compound prepared in Example 97.
$^1$H-NMR ($CDCl_3$) δ :1.11-1.30(9H,m), 1.39(1H,t,J=12.5Hz), 1.71(1H,dd,J=5.7, 13.0Hz), 2.42(3H,s), 2.75-2.88(1H,m), 3.10(1H,s), 6.30(1H,d,J=8.6Hz), 6.55-6.63(1H,m), 6.79(1H,s), 7.24(2H,d,J=8.6Hz), 7.50(2H,d,J=8.3Hz)

Example 101

N-[(4-Methylphenyl)sulfonyl]-N-(2,2,4-trimethy-1,2,3,4-tetrahydroquinolin-6-yl)acetamide

[Chemical Formula 71]

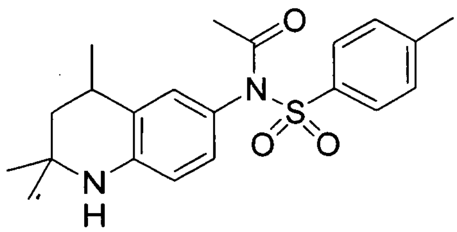

It was produced by the same method as Reference example 67 from the compound prepared in Example 86.
$^1$H-NMR ($CDCl_3$) δ :1.20-1.32(9H,m), 1.44(1H,t,J=12.6Hz), 1.76(1H,dd,J=12.7, 5.2Hz), 1.87(3H,s), 2.44(3H,s), 3.92(1H, s), 6.43(1H,t,J=8.4Hz), 6.80(1H,dd, J=8.4, 1.9Hz), 6.97(1H,s), 7.32(2H,d,J=8.1Hz), 7.93(2H,d,J=8.4Hz)

Example 102

2-Methyl-1,2,3,4-tetrahydroquinolin-6-yl 4-methylbenzenesulfonate

To a methanol (3.0mL) solution of the compound (24.5mg, 0.0782 mmol) prepared in Reference example 68

and nickel (II) chloride hexahydrate (28mg, 0.12 mmol), sodium borohydride (44mg, 1.2 mmol) was added under ice-cooling and stirred at 0°C for 10 minutes. An aqueous ammonium chloride solution was added to the reaction liquid, which was followed by extraction with ethyl acetate. The organic layer was dried over anhydrous sodium sulfate. The solvent was distilled off under reduced pressure and the obtained residue was purified by silica gel column chromatography (hexane/ethyl acetate = 10/1 - 5/1) to give 2-methyl-1,2,3,4-tetrahydroquinolin-6-yl 4-methylbenzenesulfonate (12.8mg, 52%).
1H-NMR ($CDCl_3$) δ :1.19(3H,d,J=6.2Hz), 1.45-1.56(1H,m), 1.85-1.93(1H,m), 2.44(3H,s), 2.57-2.80(2H,m), 3.30-3.41 (1H,m), 3.71(1H,br.s), 6.26(1H, d,J=8.6Hz), 6.45(1H,dd,J=8.6, 2.7Hz), 6.64(1H,d,J=2.7Hz), 7.28-7.31(2H,m), 7.69-7.72 (2H,m)

Example 103

2,2-Dimethyl-1,2,3,4-tetrahydroquinolin-6-yl 4-methylbenzenesulfonate

A mixture of the compound (37.7mg, 0.0824 mmol) prepared in Reference example 71, tri-n-butyltin hydride (54 μL, 0.20 mmol) and α, α'-azobisisobutyronitrile (AIBN) (4mg) was refluxed in toluene (1.6mL) under heating for 3 hours. The solvent was distilled off under reduced pressure and the obtained residue was purified by column chromatography (hexane/ethyl acetate = 15/1) and further recrystallized from toluene-hexane to give 2,2-dimethyl-1,2,3,4-tetrahydroquinolin-6-yl 4-methylbenzenesulfonate (13.5mg, 49%).
$^1$H-NMR ($CDCl_3$) δ :1.18(6H,s), 1.64(2H,t,J=6.8Hz), 2.44(3H,s), 2.67 (2H,t,J=6.8Hz), 3.62(1H,br.s), 6.24(1H,d,J=8.7Hz), 6.46(1H,dd, J=8.7, 2.8Hz), 6.73(1H,d,J=2.8Hz), 7.29(2H,d,J=8.5Hz), 7.51(2H,d,J=8.5Hz)

Example 104

6-{[(4-Methylphenyl)sulfonyl]methyl}-2-(trifluoromethyl)-1,2,3,4-tetrahydroquinoline

[Chemical Formula 72]

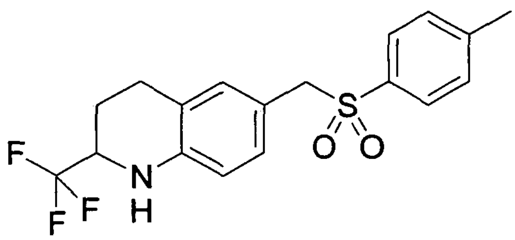

It was produced by the same method as Example 102 from the compound prepared in Reference example 72.
$^1$H-NMR ($CDCl_3$) δ :2.04-2.10(2H,m), 2.43(3H,s), 2.68-2.75(2H,m), 3.80-3.91(1H,m), 4.14(2H,s), 7.93(1H,d,J=8.2Hz), 6.66-6.72(1H,m), 6.76(1H,s), 7.26(2H,d,J=8.4Hz), 7.55(2H,d,J=8.4Hz)

Example 105

4-[(Methylsulfonyl)amino]phenyl 4-methylbenzenesulfonate

[Chemical Formula 73]

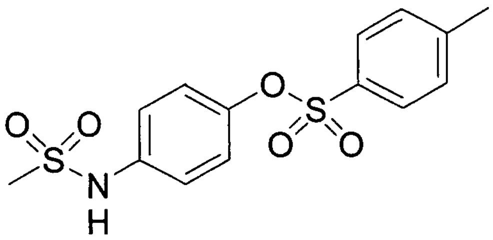

To a dichloromethane (5.0mL) solution of the compound (205mg, 0.778 mmol) prepared in Reference example 50 and triethylamine (162 μL, 1.17 mmol), methanesulfonyl chloride (73 μL, 0.93 mmol) was added and stirred at 20-25°C for 2 hours. Further, methanesulfonyl chloride (46 μL, 0.59 mmol) was added thereto and stirred for 2 hours. Water was poured into the reaction liquid, which was followed by extraction with ethyl acetate. The organic layer was dried over anhydrous sodium sulfate. The solvent was distilled off under reduced pressure and the obtained residue was purified by silica gel column chromatography (toluene/ethyl acetate = 6/1) to give 4-[(methylsulfonyl)amino]phenyl 4-methylbenzenesulfonate (106mg, 40%).
$^1$H-NMR ($CDCl_3$) δ :2.47(3H,s), 3.01(3H,s), 6.44(1H,br.s), 6.99(1H,dt,J=9.0, 2.2Hz), 7.14(1H,dt,J=9 2.2Hz), 7.32(2H,d, J=8.4Hz), 7.72(2H,d,J=8.4Hz)

Example 106

4-[(Butylsulfonyl)amino]phenyl 4-methylbenzenesulfonate

[Chemical Formula 74]

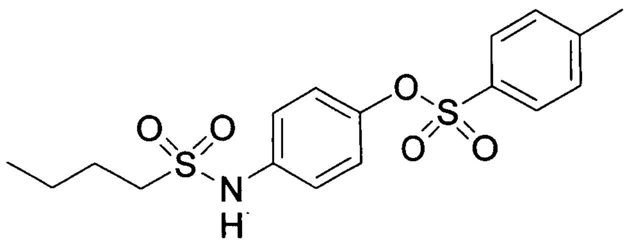

It was produced by the same method as Example 105 from the compound prepared in Reference example 50.
LC-MS (M+1): 384.1

Example 107

4-[(Isopropylsulfonyl)amino]phenyl 4-methylbenzenesulfonate

It was produced by the same method as Example 105 from the compound prepared in Reference example 50.
LC-MS (M+1): 370.1

Example 108

3-Methyl-4-[(methylsulfonyl)amino]phenyl 4-methylbenzenesulfonate

It was produced by the same methods as Reference examples 49-50 and then Example 105 from 3-methyl-4-nitrophenol.
LC-MS (M+1): 356.1

Example 109

2-Methyl-4-[(methylsulfonyl)amino]phenyl 4-methylbenzenesulfonate

[Chemical Formula 75]

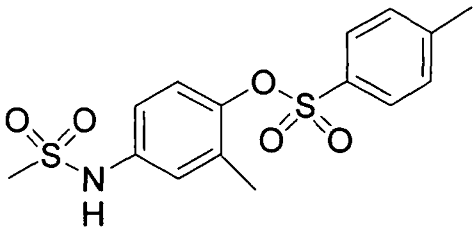

It was produced by the same methods as Reference examples 49-50 and then Example 105 from 2-methyl-4-nitrophenol.
$^1$H-NMR ($CDCl_3$) δ :2.07(3H,s), 2.47(3H,s), 3.01(3H,s), 6.95-7.04(3H,m), 7.33-7.36(2H,m), 7.74-7.77(2H,m)

Example 110

2-Fluoro-4-[(methylsulfonyl)amino]phenyl 4-methylbenzenesulfonate

[Chemical Formula 76]

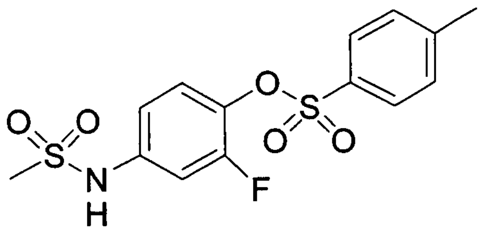

It was produced by the same methods as Reference examples 49-50 and then Example 105 from 2-fluoro-4-nitrophenol.
$^1$H-NMR ($CDCl_3$) δ :2.47(3H,s), 3.09(3H,s), 6.54-6.56(1H,br.s), 6.85-6.90(1H,m), 7.01-7.08(1H,m), 7.18-7.26(2H,m), 7.33-7.36(2H,m), 7.76-7.78(2H,m)

Example 111

2-Chloro-4-[(methylsulfonyl)amino]phenyl 4-methylbenzenesulfonate

It was produced by the same methods as Reference examples 49-50 and then Example 105 from 2-chloro-4-nitrophenol.
$^1$H-NMR ($CDCl_3$) δ :2.47(3H,s), 3.05(3H,s), 7.06-7.10(1H,m), 7.26-7.36(4H,m), 7.78-7.80(2H,m)

Example 112

N-(4-{[(4-Methylphenyl)sulfonyl]methyl}phenyl)methanesulfonamide

[Chemical Formula 77]

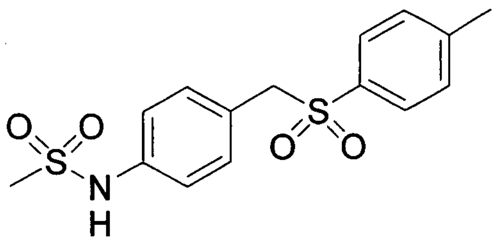

To a dichloromethane (3mL) solution of the compound (50mg, 0.19 mmol) prepared in Reference example 74, triethylamine (97mg, 0.96 mmol) and methanesulfonyl chloride (66mg, 0.57 mmol) were added and stirred at 20-25°C for 6 hours. Further, triethylamine (97mg, 0.96 mmol), methanesulfonyl chloride (66mg, 0.57 mmol) and 4-dimethylaminopyridine (DMAP) (2mg) were added thereto and stirred for 10 hours. The reaction liquid was poured into aqueous sodium bicarbonate, and then extracted with ethyl acetate. The organic layer was washed with water and saturated aqueous sodium chloride subsequently, and dried over anhydrous magnesium sulfate. The solvent was distilled off under reduced pressure. To a tetrahydrofuran (3mL) solution of the obtained residue (crude dimesyl compound, 73mg), 1.0M tetrabutylammonium fluoride (TBAF)-tetrahydrofuran solution (0.38mL) was added and stirred at 20-25°C for 2 hours. The reaction liquid was poured into water and extracted with ethyl acetate. The organic layer was washed with water and saturated aqueous sodium chloride subsequently, and dried over anhydrous magnesium sulfate. The solvent was distilled off under reduced pressure and the obtained residue was purified by silica gel column chromatography (ethyl acetate/hexane = 1/1) to give N-(4-{[(4-methylphenyl)sulfonyl]methyl}phenyl)methanesulfonamide (47mg, 72%).
$^1$H-NMR ($CDCl_3$) δ :2.38(3H,s), 2.97(3H,s), 4.55(2H,s), 7.09(4H,m), 7.36-7.39(2H,m), 7.56-7.59(2H,m), 9.80(1H,br.s)

Example 113

N-(2-Methyl-4-{[(4-Methylphenyl)sulfonyl]methyl}phenyl) methane sulfonamide

It was produced by the same methods as Reference examples 73 and 15 and then Example 112 from 3-methyl-4-nitrobenzyl bromide.
$^1$H-NMR ($CDCl_3$) δ :2.26(3H,s), 2.44(3H,s), 3.03(3H,s), 4.22(2H,m), 6.23(1H,br.s), 6.89-6.92(1H,m), 7.09(1H,m), 7.26-7.29(2H,m), 7.35-7.38(1H,m), 7.56-7.61(1H,m)

Example 114

N-(4-{[(4-Methylphenyl)sulfonyl]methyl}-3-vinylphenyl)methanesulfonamide

It was produced by the same method as Example 112 from the compound prepared in Reference example 78.
$^1$H-NMR ($CDCl_3$) δ :2.41(3H,s), 3.00(3H,s), 4.34(2H,s), 5.22(1H,dd,J=0.9, 10.8Hz), 5.47(1H,dd,J=0.9, 10.8Hz), 6.69 (1H,dd,J=10.8, 17.2Hz), 6.93(1H,br.s), 7.06-7.08(2H,m), 7.24-7.27(4H,m), 7.52-7.57(2H,m)

Example 115

4-Methyl-N-(4-{[(4-methylsulfonyl)amino]phenyl} benzenesulfonamide

It was produced by the same method as Example 105 from N-(4'-aminophenyl)-4-methylbenzenesulfonamide.
$^1$H-NMR (DMSO-$d_6$) δ :2.32(3H,s), 2.89(3H,s),6.99-7.06(4H,m), 7.32(2H,d, J=8.2Hz), 7.60(2H,d,J=8.2Hz), 9.56(1H, br.s), 10.08(1H,br.s)

Example 116

N,4-Dimethyl-N-{2-methyl-4-[(methylsulfonyl)amino]phenyl} benzenesulfonamide

It was produced by the same method as Example 112 from the compound prepared in Reference example 64.
$^1$H-NMR ($CDCl_3$) δ :2.38(3H,s), 2.46(2H,s), 3.03(3H,s), 3.09(3H,s), 6.56-6.59(1H,d,J=8.6Hz), 6.95-7.11(2H,m),

7.30-7.33(2H,m), 7.58-7.61(2H,m)

Test Example 1: Aldosterone Receptor Binding Assay

Flank incision (both sides) was subjected to a rat to adrenalectomize. After suturing the rat's incised parts, the rat was again returned to a breeding cage and bred for a duration of 3 to 5 days to collect a kidney from the rat; the collected kidney was homogenized with 0.1 M Tris-HCl, 0.25M sucrose, 0.1M $Na_2MoO_4$, 2mM DTT buffer solution (pH 7.4), the resultant solution was subjected to centrifugal separation at 105,000G for 30 minutes, and the resultant supernatant was being a soluble fraction of the kidney.
In the presence of 2μM of RU-486 (glucocorticoid receptor antagonist), 0.5 to 1.5mg of the soluble fraction of the rat's kidney, [$^3$H] aldosterone (2nM) as a marker ligand and a substance to be tested were reacted in the total capacity of 100μL at 4°C for about 18 hours. Thereafter, the reactant solution was added with 50μL of 5% dextran coated charcoal and mixed, and then centrifugated at 3,000G for 5 minutes. Subsequently, 100 μL of the supernatant was collected and mixed with 3.5mL of liquid scintillator ACS-II (Amersham) to measure a radioactivity with a liquid scintillation counter (manufactured by Packard, Tri Carb 2700TR). A non-specific binding was determined by adding 2μM of aldosterone to the reactant solution. As the result of the test, binding inhibiting rates (%) of tested substance having a concentration range of 0.1nM to 10000nM to the marker ligand were determined according the following equation.
$$\text{Binding inhibiting rate (\%)} = 100-[(B\text{-}N)/(B_0\text{-}N)]\times 100$$

B: Binding amount of the marker ligand in the co-presence of the tested substance and marker ligand,
$B_0$: Binding amount of the marker ligand in the absence of the tested substance, and
N: Binding amount of the marker ligand in the presence of excess aldosterone (2μM).

$IC_{50}$ value (μM) being equivalent to the tested substance concentration of 50% inhibition was calculated from a concentration-reaction curve.
The compounds obtained in Examples were subjected to the test shown in Test Example 1.
The results of the aldosterone receptor binding assay are shown in Table 1.
[Table 1]

| Compound (Example No.) | MR $IC_{50}$ (μM) | Compound (Example No.) | MR $IC_{50}$ (μM) |
|---|---|---|---|
| 2 | 0.32 | 66 | 1.3 |
| 12 | 0.083 | 67 | 0.6 |
| 14 | 0.47 | 68 | 1.5 |
| 16 | 0.007 | 69 | 1.2 |
| 18 | 0.039 | 70 | 0.3 |
| 22 | 0.21 | 73 | 0.21 |
| 23 | 2.4 | 74 | 0.12 |
| 24 | 0.013 | 75 | 0.39 |
| 26 | 0.015 | 78 | 0.9 |
| 28 | 0.013 | 80 | 1.3 |
| 29 | 1.6 | 81 | 0.5 |
| 30 | 0.16 | 85 | 0.15 |
| 36 | 1.3 | 86 | 0.27 |
| 37 | 0.96 | 87 | 0.5 |
| 38 | 6.6 | 88 | 0.3 |

(continued)

| Compound (Example No.) | MR $IC_{50}$ (μM) | Compound (Example No.) | MR $IC_{50}$ (μM) |
|---|---|---|---|
| 41 | 5.5 | 89 | 0.3 |
| 44 | 0.86 | 91 | 0.1 |
| 46 | 2.9 | 96 | 0.5 |
| 48 | 0.32 | 97 | 2.2 |
| 49 | 2.7 | 98 | 0.094 |
| 50 | 0.011 | 99 | 3.9 |
| 51 | 0.3 | 100 | 2.1 |
| 52 | 0.73 | 101 | 0.19 |
| 54 | 0.2 | 104 | 1.8 |
| 57 | 0.029 | 105 | 6.6 |
| 60 | 0.3 | 106 | 8 |
| 61 | 0.52 | 109 | 16 |
| 62 | 0.13 | 110 | 9.4 |
| 63 | 0.36 | 112 | 5.4 |

Test Example 2: Antagonistic Action (in vivo Antagonist Activity) against Decrease of Na/K Ratio in Urine Induced with Aldosterone by Using Rat of Which Adrenal Glands Were Extirpated

After anesthetizing a rat by administering 50mg/kg of sodium pentobarbital in its abdominal cavity and disinfecting its skin with the Isodine diluted solution, the rat was incised. After adrenalectomy and confirming the hemostasis, the muscular layer and skin were sutured in this order and then the sutured part was disinfected with the Isodine diluted solution. After breeding 3 days or more, a rat showing good progress was subjected to the following test.
It was fasted from the day preceding an examination, put into a metabolic cage at the day of the examination, and then subjected to a urine collection for 4 hours. When a substance to be tested was administered orally, it was administered in an amount of 5ml/kg with 0.5% methylcellulose as a solvent. The aldosterone was dissolved with 1% ethanol/physiological saline and administered subcutaneously. The substance to be tested was administered 30 minutes before administering aldosterone. In order to stabilize and sufficiently secure the amount of the urine, physiological saline was administered orally in an amount of 20ml/kg at the time of administering aldosterone. The colleted urine, after measuring its volume, was subjected to a measurement of electrolyte (sodium: Na and potassium: K) concentration in the urine with a biochemistry autoanalyzer (manufactured by Beckman, Synchron CX3 system Delta).

The compound obtained in Example 24 and eplerenone being an aldosterone receptor antagonist were subjected to the test shown in Test Example 2. The result of each group was summarized and the average value and standard error value of the Na/K ratio in the urine are shown in Fig. 1. The compound of Example 24 dose-dependently inhibited the decrease of the urine Na/K ratio which was induced with aldosterone.

Industrial Applicability

Invention Compound is useful for an agent treating and preventing cardiovascular diseases including hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism and inflammations.

## Claims

1. An agent for preventing or treating hypertesion, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations comprising a compound represented by the formula (1):

[Chemical Formula 1]

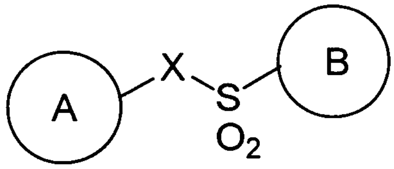
(1)

[wherein A represents any of groups represented by the following formula (A-1), (A-2), (A-3), (A-4) or (A-5);

[Chemical Formula 2]

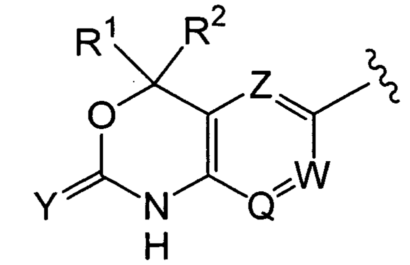
(A-1)

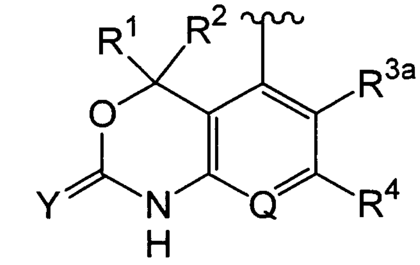
(A-2)

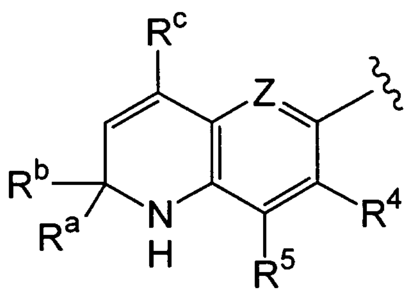
(A-3)

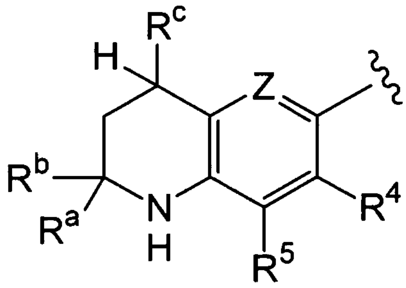
(A-4)

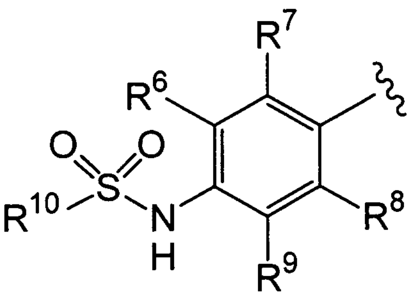
(A-5)

(wherein $R^1$ and $R^2$ each independently represent a hydrogen atom, optionally substituted alkyl group, optionally substituted alkenyl group, optionally substituted alkynyl group, optionally substituted cycloalkyl group, optionally substituted aryl group or optionally substituted heteroaryl group, or $R^1$ and $R^2$ represent an optionally substituted cycloalkane ring or optionally substituted saturated heterocyclic ring by being combined together with each other's

adjacent carbon atom;

Z represents a nitrogen atom or $CR^3$, W represents a nitrogen atom or $CR^4$, and Q represents a nitrogen atom or $CR^5$;
R3, $R^{3a}$, $R^4$, $R^5$, $R^6$, $R^7$, $R^8$ and $R^9$ each independently represent a hydrogen atom, halogen atom, optionally substituted alkyl group, optionally substituted cycloalkyl group, optionally substituted alkenyl group, optionally substituted alkynyl group, optionally substituted aryl group, optionally substituted heteroaryl group, cyano group, nitro group, hydroxyl group, optionally substituted amino group, optionally substituted alkoxy group, optionally substituted alkanoyl group, optionally substituted alkoxycarbonyl group, carboxy group, optionally substituted carbamoyl group, optionally substituted alkylthio group, optionally substituted alkylsulfinyl group, optionally substituted sulfamoyl group or optionally substituted alkylsulfonyl group;
$R^{10}$ represents an optionally substituted alkyl group;
$R^a$, $R^b$ and $R^c$, each independently represent a hydrogen atom or optionally substituted alkyl group; and
Y represents an oxygen atom or sulfur atom);
X represents an oxygen atom, $NR^{11}$ or $CR^{12}R^{13}$
(wherein $R^{11}$ represents a hydrogen atom, optionally substituted alkyl group, optionally substituted alkanoyl group, optionally substituted aroyl group, optionally substituted alkoxycarbonyl group, optionally substituted alkylsulfonyl group, optionally substituted arylsulfonyl group, optionally substituted heteroarylsulfonyl group or the group represented by the formula -C(O)-C(O)-$OR^{11a}$ (wherein $R^{11a}$ represents a hydrogen atom or optionally substituted alkyl group); and
$R^{12}$ and $R^{13}$ each independently represent a hydrogen atom, optionally substituted alkyl group or optionally substituted cycloalkyl group, or $R^{12}$ and $R^{13}$ represent an optionally substituted cycloalkane ring by being combined together with each other's adjacent carbon atom); and
B represents an optionally substituted aryl group or optionally substituted heteroaryl group],
a prodrug thereof or a pharmaceutically acceptable salt thereof.

**2.** A compound represented by the formula (1a);

[Chemical Formula 3]

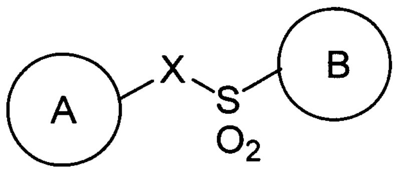

(1a)

[wherein A represents any of groups represented by the following formula (A-1), (A-2), (A-3), (A-4) or (A-5);

[Chemical Formula 4]

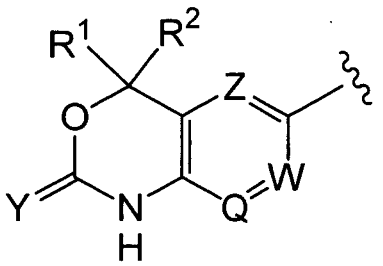

(A-1)

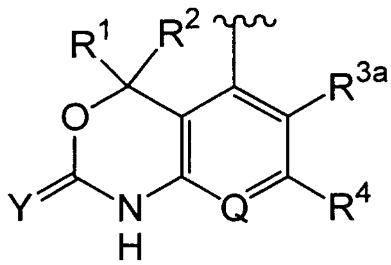

(A-2)

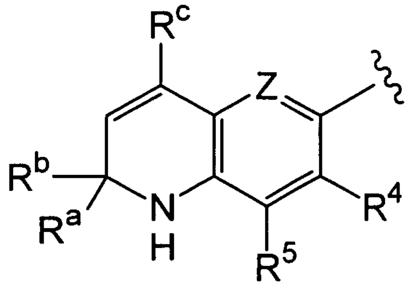

(A-3)

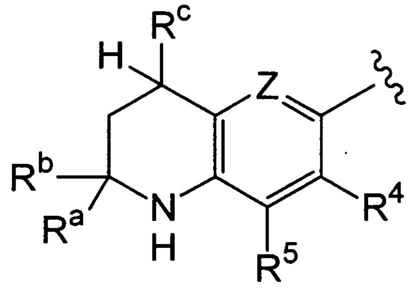

(A-4)

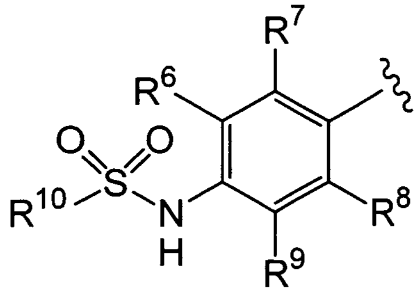

(A-5)

(wherein $R^1$ and $R^2$ each independently represent a hydrogen atom, optionally substituted alkyl group, optionally substituted alkenyl group, optionally substituted alkynyl group, optionally substituted cycloalkyl group, optionally substituted aryl group or optionally substituted heteroaryl group, or $R^1$ and $R^2$ represent an optionally substituted cycloalkane ring or optionally substituted saturated heterocyclic ring by being combined together with each other's adjacent carbon atom;

Z represents a nitrogen atom or $CR^3$, W represents a nitrogen atom or $CR^4$, and Q represents a nitrogen atom or $CR^5$;

R3, $R^{3a}$, $R^4$, $R^5$, $R^6$, $R^7$, $R^8$ and $R^9$ each independently represent a hydrogen atom, halogen atom, optionally substituted alkyl group, optionally substituted cycloalkyl group, optionally substituted alkenyl group, optionally substituted alkynyl group, optionally substituted aryl group, optionally substituted heteroaryl group, cyano group, nitro group, hydroxyl group, optionally substituted amino group, optionally substituted alkoxy group, optionally substituted alkanoyl group, optionally substituted alkoxycarbonyl group, carboxy group, optionally substituted carbamoyl group, optionally substituted alkylthio group, optionally substituted alkylsulfinyl group, optionally substituted sulfamoyl group or optionally substituted alkylsulfonyl group;

$R^{10}$ represents an optionally substituted alkyl group;

$R^a$, $R^b$ and $R^c$ each independently represent a hydrogen atom or optionally substituted alkyl group; and

Y represents an oxygen atom or sulfur atom);

X represents an oxygen atom, $NR^{11}$ or $CR^{12}R^{13}$

(wherein $R^{11}$ represents a hydrogen atom, optionally substituted alkyl group, optionally substituted alkanoyl group, optionally substituted aroyl group, optionally substituted alkoxycarbonyl group, optionally substituted alkylsulfonyl group, optionally substituted arylsulfonyl group, optionally substituted heteroarylsulfonyl group or the group represented by the formula -C(O)-C(O)-$OR^{11a}$ (wherein $R^{11a}$ represents a hydrogen atom or optionally substituted alkyl group); and
$R^{12}$ and $R^{13}$ each independently represent a hydrogen atom, optionally substituted alkyl group or optionally substituted cycloalkyl group, or $R^{12}$ and $R^{13}$ represent an optionally substituted cycloalkane ring by being combined together with each other's adjacent carbon atom); and
B represents an optionally substituted aryl group or optionally substituted heteroaryl group;

wherein, when the A is the compound represented by the formula (A-1), Z is $CR^3$, W is $CR^4$ and Q is $CR^5$, $R^1$ represents an optionally substituted alkenyl group, optionally substituted alkynyl group, optionally substituted cycloalkyl group, optionally substituted aryl group or optionally substituted heteroaryl group],
a prodrug thereof or a pharmaceutically acceptable salt thereof.

**3.** The compound, the prodrug thereof or the pharmaceutically acceptable salt thereof described in claim 2, wherein X is an oxygen atom, $NR^{11}$ or $CR^{12}R^{13}$ (wherein $R^{11}$ represents a hydrogen atom, optionally substituted alkyl group or optionally substituted alkanoyl group; and $R^{12}$ and $R^{13}$ each independently represent a hydrogen atom or optionally substituted alkyl group).

**4.** The compound, the prodrug thereof or the pharmaceutically acceptable salt thereof described in claim 3, wherein B is an optionally substituted phenyl, optionally substituted naphthyl, optionally substituted pyridyl, optionally substituted furanyl, optionally substituted pyrrolyl or optionally substituted thienyl.

**5.** The compound, the prodrug thereof or the pharmaceutically acceptable salt thereof described in claim 4, wherein A is the group represented by the formula (A-6);

[Chemical Formula 5]

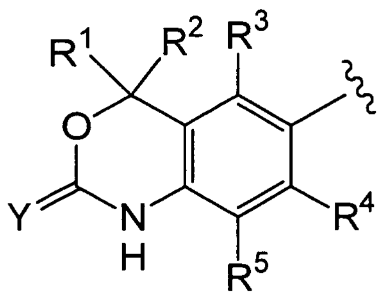

(A-6)

wherein $R^1$ is an optionally substituted alkenyl group, optionally substituted alkynyl group, optionally substituted cycloalkyl group, optionally substituted aryl group or optionally substituted heteroaryl group.

**6.** The compound, the prodrug thereof or the pharmaceutically acceptable salt thereof described in claim 5, wherein $R^3$, $R^4$ and $R^5$ are each independently a hydrogen atom, halogen atom, optionally substituted alkyl group, hydroxyl group or optionally substituted alkoxy group.

**7.** The compound, the prodrug thereof or the pharmaceutically acceptable salt thereof described in claim 4, wherein A is the group represented by the formula (A-7);

[Chemical Formula 6]

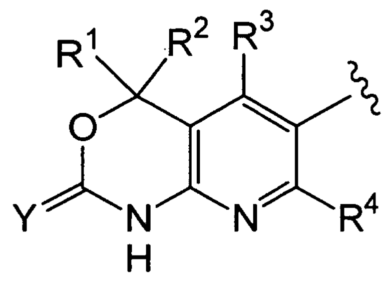

(A-7)

wherein $R^1$ and $R^2$ are each independently a hydrogen atom or optionally substituted alkyl group.

8. The compound, the prodrug thereof or the pharmaceutically acceptable salt thereof described in claim 7, wherein $R^3$ and $R^4$ are each independently a hydrogen atom, halogen atom, optionally substituted alkyl group, hydroxyl group or optionally substituted alkoxy group.

9. The compound, the prodrug thereof or the pharmaceutically acceptable salt thereof described in claim 4, wherein the A is the group represented by the formula (A-3) or (A-4), and $R^4$ and $R^5$ are each independently a hydrogen atom, halogen atom, optionally substituted alkyl group, hydroxyl group or optionally substituted alkoxy group.

10. The compound, the prodrug thereof or the pharmaceutically acceptable salt thereof described in claim 2, wherein the A is the group represented by the formula (A-5).

11. A pharmaceutical composition comprising the compound, the prodrug thereof or the pharmaceutically acceptable salt thereof described in any of claims 2 to 10, as its active ingredient.

12. An agent for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations, comprising the compound, the prodrug thereof or the pharmaceutically acceptable salt thereof described in any of claims 2 to 10, as its active ingredient.

13. A method for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations, which comprises administering the compound, the prodrug thereof or the pharmaceutically acceptable salt thereof described in any of claims 2 to 10 in an effective dose to a patient requiring treatment.

14. A use of the compound, the prodrug thereof or the pharmaceutically acceptable salt thereof described in any of claims 2 to 10 for manufacturing an agent for preventing or treating hypertension, cerebral stroke, arrhythmia, cardiac failure, cardiac hypertrophy, arteriosclerosis, blood vessel restenosis, kidney fibrosis, myocardial infarction, diabetes complications, renal diseases, edema, primary aldosteronism or inflammations.

Fig. 1

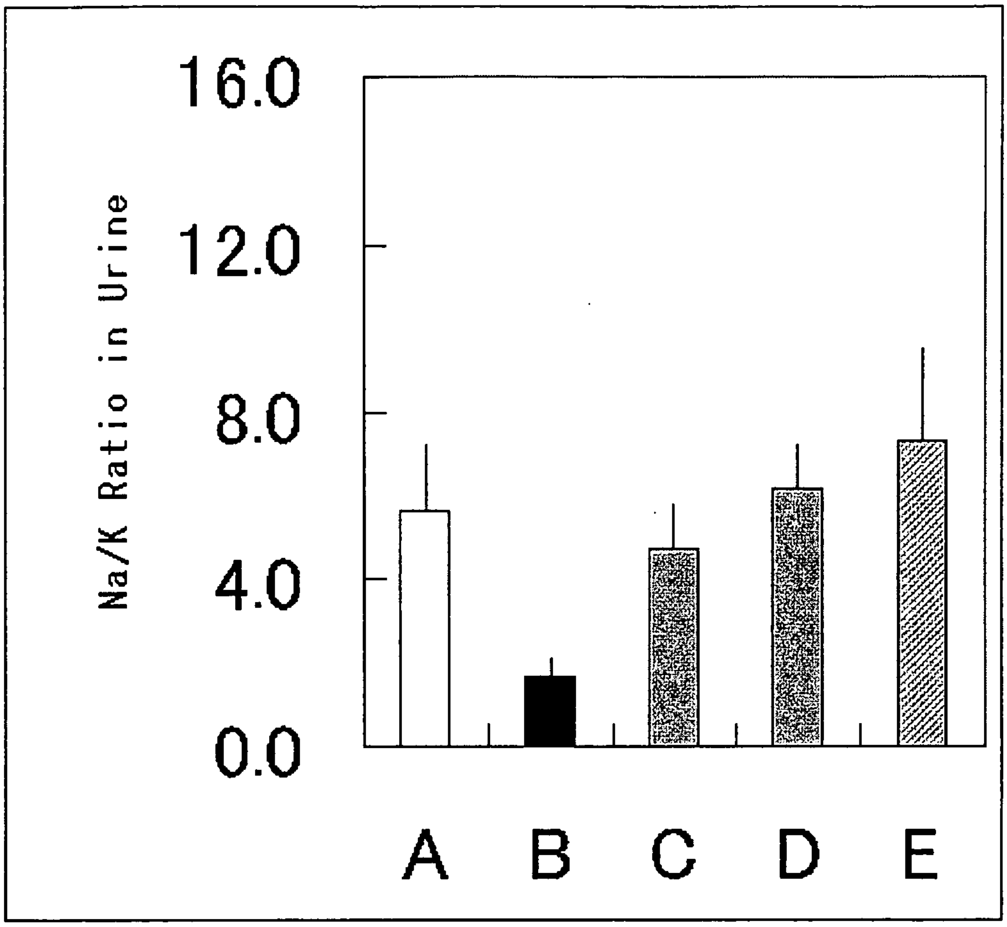

## INTERNATIONAL SEARCH REPORT

International application No.
PCT/JP2006/300509

A. CLASSIFICATION OF SUBJECT MATTER
***A61K31/18***(2006.01), ***A61K31/255***(2006.01), ***A61K31/4375***(2006.01), ***A61K31/47***(2006.01), ***A61K31/4709***(2006.01), ***A61K31/536***(2006.01), ***A61K31/5365***(2006.01), ***A61P7/10***(2006.01), ***A61P9/00***(2006.01), ***A61P9/04***(2006.01),
According to International Patent Classification (IPC) or to both national classification and IPC

B. FIELDS SEARCHED
Minimum documentation searched (classification system followed by classification symbols)
A61K31/4375, A61K31/47, A61K31/4709, A61K31/536, A61K31/5365, A61P7/10, *A61P9/00*(2006.01), A61P9/04, A61P9/06, A61P9/10, C07D265/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X<br>P,A | US 2005/0085470 A1 (ZHANG P),<br>21 April, 2005 (21.04.05),<br>Full text; Claims<br>(Family: none) | 2-6,11<br>1,12,14 |
| A | JP 2002-543193 A (WYETH),<br>17 December, 2002 (17.12.02),<br>Full text<br>& WO 2000/66571 A1 | 1-6,11,12,14 |
| A | JP 2003-508387 A (LIGAND PHARM INC.),<br>04 March, 2003 (04.03.03),<br>Full text<br>& WO 2001/16108 A2 | 1-6,11,12,14 |

☒ Further documents are listed in the continuation of Box C.
☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 April, 2006 (17.04.06) | Date of mailing of the international search report<br>25 April, 2006 (25.04.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/300509

C (Continuation). DOCUMENTS CONSIDERED TO BE RELEVANT

| Category* | Citation of document, with indication, where appropriate, of the relevant passages | Relevant to claim No. |
|---|---|---|
| A | JP 57-48975 A (BOEHRINGER INGELHEIM), 20 March, 1982 (20.03.82), Full text & US 4341778 A | 1-6,11,12,14 |

Form PCT/ISA/210 (continuation of second sheet) (April 2005)

**INTERNATIONAL SEARCH REPORT**

International application No.
PCT/JP2006/300509

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 13
because they relate to subject matter not required to be searched by this Authority, namely:
Claim 13 pertains to methods for treatment of the human body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Part of claims 1-4, 11, 12, and 14 and claims 5-6

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/300509

Continuation of Box No.III of continuation of first sheet(2)

With respect to the subject matters of claims 1-12 and 14, there is no basic skeleton common among a group of substituents represented by the formulae (A-1) and (A-2), a group of substituents represented by (A-3) and (A-4), and a group of substituents represented by (A-5). However, the group $-XSO_2B$ itself, which is common among the compounds of the invention, is known as a structure in compounds, such as a benzenesulfonamide group. To have this group cannot hence be regarded as a special technical feature of the invention. Furthermore, compounds which are usable in the same medical applications as the compounds of the invention and are compounds comprising benzoxazine and an aryl group (JP 57-48975 A) or compounds having a quinoline structure (JP 10-510840) are known. It is not understood that there is a basic structure common between benzoxazine and a fused ring comprising a nitrogenous heteroaryl and oxazine, which equally have an oxazine structure, and between the case of a quinoline structure and other cases. In view of this, there is no "special technical feature" among the subject matters concerning the compounds of the invention (and a use thereof), which have at least the five substituents as group (A).

Therefore, these five subject matters are not considered to be so linked as to form a single general inventive concept.

The invention involves at least the following five subject matters.

1) A compound which has substituents (A-1) and (A-2) and has a benzoxazine structure, and a use thereof

Part of claims 1-4, claims 5 and 6, and part of claims 11, 12, and 14

2) A compound having substituents (A-1) and (A-2) and a use thereof which are not within 1)

Part of claims 1-4, claims 7 and 8, and part of claims 11, 12, and 14

3) A compound which has substituents (A-3) and (A-4) and has a quinoline structure and a use thereof

Part of claims 1-4 and part of claims 9, 11, 12, and 14

4) A compound having substituents (A-3) and (A-4) and a use thereof which are not within 3)

Part of claims 1-4 and part of claims 9, 11, 12, and 14

5) A compound having a substituent (A-5) and a use thereof

Part of claims 1-4, claim 10, and part of claims 11, 12, and 14

Form PCT/ISA/210 (extra sheet) (April 2005)

**INTERNATIONAL SEARCH REPORT**

International application No.
PCT/JP2006/300509

Continuation of A. CLASSIFICATION OF SUBJECT MATTER
(International Patent Classification (IPC))

***A61P9/06***(2006.01), ***A61P9/10***(2006.01), ***A61P9/12***(2006.01), ***A61P13/12***(2006.01), ***A61P29/00***(2006.01), ***C07C311/08***(2006.01), ***C07C311/21***(2006.01), ***C07C317/32***(2006.01), ***C07D215/12***(2006.01), ***C07D215/20***(2006.01), ***C07D215/38***(2006.01), ***C07D265/18***(2006.01), ***C07D409/12***(2006.01), ***C07D413/12***(2006.01), ***C07D471/04***(2006.01), ***C07D498/04***(2006.01)

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004067529 A
- WO 9814436 A
- WO 0116108 A
- WO 9619458 A
- WO 0066570 A
- WO 0066592 A
- WO 03004028 A


### Non-patent literature cited in the description


- **T. W. GREENE.** Protective Groups in Organic Synthesis. John Wiley & Sons Inc, 1981
- *Tetrahedron Lett.,* 2002, vol. 43, 3907-3910
- **R.C. LAROCK.** *Comprehensive Organic Transformations,* 1989